# EUROPEAN PATENT SPECIFICATION

(11) **EP 4 182 322 B1**
(45) Date of publication and mention of the grant of the patent: **24.04.2024**
(21) Application number: 21746405.6
(22) Date of filing: 14.07.2021
(51) Int. Cl.: C07D 498/04, A61K 31/5383, A61P 11/00

(54) **PYRIDO OXAZINE DERIVATIVES AS ALK5 INHIBITORS**
PYRIDO-OXAZIN-DERIVATE ALS ALK5-INHIBITOREN
DÉRIVÉS DE PYRIDO-OXAZINE EN TANT QU'INHIBITEURS D'ALK5

(30) Priority: 15.07.2020 EP 20185897
(43) Date of publication of application: 24.05.2023
(73) Proprietor: Chiesi Farmaceutici S.p.A., 43122 Parma (IT)
(72) Inventor: PALA, Daniele, 43122 Parma (IT); PIZZIRANI, Daniela, 43122 Parma (IT); BRUNO, Paolo, 43122 Parma (IT); RONCHI, Paolo, 43122 Parma (IT); BIAGETTI, Matteo, 43122 Parma (IT); GUARIENTO, Sara, 43122 Parma (IT); FIORELLI, Claudio, 43122 Parma (IT)
(74) Representative: Chiesi Farmaceutici S.p.A.
(86) International application number: PCT/EP2021/069656
(87) International publication number: WO 2022/013311

(56) References cited:
- WO-A1-03/097639
- WO-A1-2009/133070

## Description

### FIELD OF THE INVENTION

The present invention generally relates to compounds inhibiting the transforming growth factor β (TGF β) type I receptor (ALK5) (hereinafter ALK5 inhibitors), methods of preparing such compounds, pharmaceutical compositions containing them and therapeutic use thereof.

The compounds of the invention may be useful for instance in the treatment of many diseases, disorders, or conditions associated with ALK5 signaling pathway.

### BACKGROUND OF THE INVENTION

The Transforming Growth Factor β (TGF β) is a protein belonging to the TGF β superfamily.

It is involved in several processes, both cellular, such as proliferation, migration and differentiation, and biological, including wound healing, immunesuppression, cancerogenesis and extracellular matrix production.

The TGF β superfamily also includes, among others, other members known as activins (Acts) (see e.g. Hinck AP, FEBS Letters 586 (2012); 1860-1870).

The binding of the peptide initiates the TGF β signalling cascade through the formation of a heterotetrameric complex composed of two different serine/threonine kinases receptors: type 1 (TGFβR1/ALK5) and type 2 (TGFβR2).

TGFβR1/ALK5 is recruited and activated through the phosphorylation of its intracellular domain by TGFβR2, leading in turn to the phosphorylation of the receptor-activated (R)-Smad family, resulting in the activation of target gene transcription (see e.g. Sheppard D., Proc Am Thorac Soc. (2006);(3):413-417).

Similarly to the TGF β signaling,the type I receptor for activin, ALK4, leads to the activation of target gene transcription (see e.g. Heldin CH et al., Cold Spring Harb Perspect Biol. (2016) Aug 1;8(8)).

Several studies have linked an excessive and/or dysregulated TGFβ activity with many diseases including cancer and fibrosis (see e.g. Syed V, J Cell Biochem. (2016) Jun;117(6):1279-87; Jakowlew SB. Cancer Metastasis Rev. (2006) Sep;25(3):435-57). Among fibrotic disorders, a crucial role of TGFβ has been shown in organs such as lung, heart, liver, and kidney (see e.g. Alhamad EH, J Thorac Dis. (2015);7(3):386-93). In particular, TGFβ expression is increased in fibrotic lung diseases, such as idiopathic pulmonary fibrosis (IPF), and in chronic inflammatory conditions, such as chronic obstructive pulmonary disease and asthma (see e.g. Thomas BJ et al., Am J Respir Cell Mol Biol. (2016);(55):759-766).

In lung, TGFβ is expressed in several cell types, like epithelial cells, endothelial cells, connective tissue cells, macrophages and fibroblasts.

These cell populations may produce excess of TGFβ in IPF human lung tissue. Moreover, high levels of TGFβ have been detected in lung tissue and BAL of IPF patients (see e.g. Bergeron A et al., Eur Respir J (2003);22:69-76).

TGFβ gene expression and TGFβ protein production have been observed to increase in a variety of animal models of pulmonary fibrosis caused by bleomycin, silica, asbestos, and radiation (see e.g. Wei F et al., Int Immunopharmacol. (2017) Jul;48:67-75; Choe JY et al., Inflamm Res. (2010) Mar;59(3):177-88; Wang X et al., Respir Res (2009);10, 36) and it has also been reported how the TGFβ expression is sufficient to induce progressive fibrosis in rodents (see e.g. Sime PJ et al., J Clin Invest (1997);100:768-776; Kim KK et al.).

Contrarily, TGFβ signaling inhibition obtained by employing knockout (KO) animals can inhibit fibrosis development through TGFβ-linked mechanisms (see e.g. Bonniaud P et al., Am J Respir Crit Care Med (2005);171:889-898; 34).

Similar results have been achieved with inhibition of TGFβR1 in mouse bleomycin disease model (see e.g. Wei Y et al., J Clin Invest. (2017);127(10):3675-3688).

Activin signaling dysregulation, similarly to TGFβ, is associated to fibroblasts proliferation, myofibroblasts differentiation and accumulation of extracellular matrix (ECM) (see e.g. Yamashita et al., J. Am. Soc. Nephrol. (2004) 15, 91-101). Moreover, overexpression of activin has been linked to pathological conditions and fibrosis development in different organs, such as liver (see e.g. Patella et al., Am. J. Physiol. Gastrointest. Liver Physiol. (2006) 290, G137-G144), kidney (see e.g. Agapova et al., Kidney Int. (2016) 89, 1231-1243), heart (see e.g. Yndestad et al., Circulation (2004) 109,1379-1385), and lung (see e.g. de Kretser et al., Crit.Care (2013) 17:R263).

Taken together these data suggest the importance of targeting ALK5 and/or ALK4, preferably ALK5, to treat pharmacologically the aforementioned diseases, linked to dysregulated TGF signaling pathway.

The TGFβ signaling is strongly involved in the cardiovascular homeostasis (see e.g. van Meeteren LA et al., Springer (2013)). Several studies in humans and mice have shown the main role of TGFβ in angiogenesis and vascular morphogenesis. Moreover, TGFβ plays a key role in the development and functionality of cardiac valves. It is therefore clear the importance of a selective regulation of TGFβ pathway to target the pathological effects avoiding the suppression of the signaling needed for a correct homeostasis.

The answer to this crucial point could be addressed by using the inhalation route to deliver an antiTGFβ drug.

The inhalatory route would allow the treatment of the affected lung compartment bypassing the issue of the heart exposure.

Various compounds have been described in the literature as ALK5 and/or ALK4 inhibitors.

WO2008/006583, WO2009/087212, WO2009/087224, WO2009/087225, WO2009/133070, WO2009/013335 and WO2009/050183 (Novartis) disclose respectively pyrimidine, pyridine, imidazo pyridine, pyrrolo pyrimidine and pyrrolo pyridine, imidazo pyridazine, imidazo pyridine derivatives useful for the treatment of ALK4- or ALK5-mediated diseases such as inflammatory or obstructive airways diseases, pulmonary hypertension and pulmonary fibrosis.

WO00/61576 and US2003/0149277 (Smithkline Beecham Corp) disclose triarylimidazole derivatives as ALK5 inhibitors useful for the treatment of, among others, renal disease, wound healing, kidney disease, congestive heart failure, ulcers, impaired neurological function and any disease wherein fibrosis is a major component.

WO01/62756 (Smithkline Beecham P.L.C.) discloses pyridinylimidazole derivatives as ALK5 inhibitors useful for the treatment of, among others, renal disease, wound healing, kidney disease, congestive heart failure, ulcers, impaired neurological function and any disease wherein fibrosis is a major component.

WO03/087304 (Biogen Inc.) discloses tri-substituted heteroaryls as ALK5 and/or ALK4 inhibitors useful for the treatment of, among others, idiopathic pulmonary fibrosis, diabetic nephropathy, hepatic fibrosis, pulmonary fibrosis, acute lung injury, post-infarction cardiac fibrosis, fibrotic cancers and fibroma.

WO2013/009140 (SK Chemicals Co) discloses 2-pyridyl substituted imidazole derivatives as ALK5 and/or ALK4 receptor inhibitors useful for the treatment of, among others, renal, liver or pulmonary fibrosis.

Pyrido oxazine derivatives have been disclosed in the literature, but not as ALK5 inhibitors.

WO2013/093849 (Novartis) discloses, among other compounds, pyrido oxazine derivatives as inhibitors of Pi3K, wherein the pyridine condensed to the oxazine ring is linked to a 5-membered heterocyclic ring through an O or NH bridge. These compounds are disclosed as useful for the treatment of, among others, disorders or diseases selected from rheumatoid arthritis, systemic lupus erythematosus, multiple sclerosis, allergy and malaria.

Of note, inhibition of ALK5 receptor may be useful for the treatment of fibrosis and disease, disorder and conditions that result from fibrosis.

Several efforts have been done in the past years to develop novel ALK5 receptor inhibitors useful for the treatment of several disease and some of those compounds have shown efficacy also in humans.

However, there remains a potential for developing inhibitors of ALK5 receptor characterized by good potency, useful for the treatment of diseases or conditions associated with a dysregulation of ALK5 signaling pathway, in particular fibrosis.

In particular, there remains a potential for developing inhibitors of ALK5 receptor useful for the treatment of diseases or conditions associated with a dysregulation of ALK5 signaling pathway in the respiratory field, in particular idiopathic pulmonary fibrosis (IPF), to be administered by the inhalation route and characterized by a good inhalatory profile, that corresponds to a good activity in the lung, a good lung retention and to a low metabolic stability in order to minimize the systemic exposure and correlated safety issues.

In this direction, we have surprisingly found a new series of compounds of general formula (I) that solves the problem of providing potent inhibitors of ALK5 receptor for administration by inhalation, that shows, at the same time, a good inhalatory profile, low metabolic stability, low systemic exposure, improved safety and tolerability, and a good selectivity across the kinome.

### SUMMARY OF THE INVENTION

In a first aspect the present invention relates to compounds of formula (I) 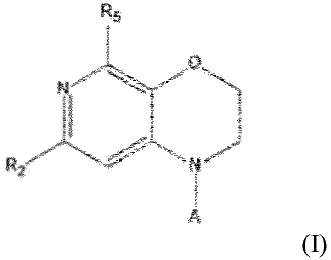 wherein
**A** is selected from the groups consisting of **A1** and **A2** 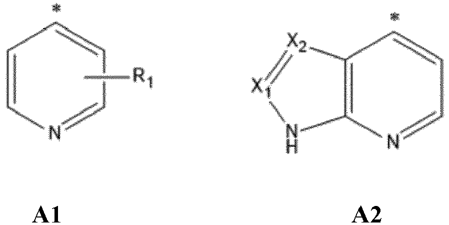
**R₁** is H or is selected from the group consisting of -NR₃C(O)R₄ and -NR₃R_{3'};
**X₁** and **X₂** are independently N or CH;
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄** is selected from the group consisting of -(C₁-C₆)alkyl, -NR_{A}R_{B}, -(C₁-C₆)alkylene-NR_{A}R_{B}, -(C₁-C₆)alkylene-O-(C₁₋C₆)alkyl, -NH(C₁-C₆)alkylene-heterocycloalkyl and -(C₁-C₆)alkylene-heterocycloalkyl, wherein said heterocycloalkyl is optionally substituted by one or more groups selected from oxo, alkoxy, -(C₁₋C₆)alkylene-OH, -C(O)O-(C₁-C₆)alkyl, -(C₁₋C₆)alkylene-C(O)O-(C₁-C₆)alkyl, -(C₁₋C₆)alkylene-C(O)NH-(C₁₋C₆)alkyl, -(C₁₋C₆)alkylene-C(O)NH-(C₁₋C₆)alkyl-OH, -(C₁₋C₆)alkylene-NHSO₂-(C₁₋C₆)alkyl, -(C₁₋C₆)alkylene-CONH₂, -(C₁₋C₆)alkylene-NR_{A}R_{B}, and -(C₁-C₆)alkyl;
**R_{A}** is H or is selected from the group consisting of -(C₁-C₆)alkyl, heterocycloalkyl and -(C₁-C₆)hydroxyalkyl;
**R_{B}** is selected from the group consisting of -(C₁-C₆)alkyl, -(C₁-C₆)alkylene-C(O)O-(C₁-C₆)alkyl, heterocycloalkyl, -(C₁-C₆)alkylene-heterocycloalkyl, -SO₂-(C₁-C₆)alkyl, -(C₁-C₆)alkylene-NH-SO₂-(C₁-C₆)alkyl, -(C₁-C₆)alkylene-SO₂-(C₁₋C₆)alkyl and -(C₁-C₆)hydroxyalkyl, wherein said heterocycloalkyl of the -(C₁-C₆)alkylene-heterocycloalkyl may be optionally substituted by one or more -(C₁-C₆)alkyl;
**R₅** is H or selected from the group consisting of -NH₂, -C(O)OH, -C(O)O-(C₁₋C₆)alkyl, -(C₁-C₆)haloalkyl and -OH;
and pharmaceutically acceptable salts thereof.

In a second aspect, the invention refers to a pharmaceutical composition comprising a compound of formula (I) and pharmaceutically acceptable salts thereof in a mixture with one or more pharmaceutically acceptable carrier or excipient.

In a third aspect, the invention refers to a compound of formula (I) and pharmaceutically acceptable salts or to a pharmaceutical composition comprising a compound of formula (I) and pharmaceutically acceptable salts thereof for use as a medicament.

In a further aspect, the invention refers to a compound of formula (I) and pharmaceutically acceptable salts thereof or to a pharmaceutical composition comprising a compound of formula (I) and pharmaceutically acceptable salts thereof for use in preventing and/or treating a disease, disorder or condition mediated by ALK5 receptor in a mammal.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment of fibrosis and/or diseases, disorders, or conditions that involve fibrosis.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment idiopathic pulmonary fibrosis (IPF).

### DETAILED DESCRIPTION OF THE INVENTION

### Definitions

Unless otherwise specified, the compound of formula (I) of the present invention is intended to include also stereoisomers, tautomers or pharmaceutically acceptable salts or solvates thereof.

The term "pharmaceutically acceptable salts", as used herein, refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts comprise ions of alkali or alkaline earth metals such as potassium, sodium, calcium or magnesium.

Those obtained by reacting the main compound, functioning as a base, with an inorganic or organic acid to form a salt comprise, for example, salts of hydrochloric acid, hydrobromic acid, sulfuric acid, phosphoric acid, methane sulfonic acid, camphor sulfonic acid, acetic acid, oxalic acid, maleic acid, fumaric acid, succinic acid and citric acid.

The term "solvate" means a physical association of a compound of this invention with one or more solvent molecules, whether organic or inorganic. This physical association includes hydrogen bonding. In certain instances, the solvate will be capable of isolation, for example, when one or more solvent molecules are incorporated in the crystal lattice of the crystalline solid. The solvate may comprise either a stoichiometric or nonstoichiometric amount of the solvent molecules.

The term "stereoisomer" refers to isomers of identical constitution that differ in the arrangement of their atoms in space. Enantiomers and diastereomers are examples of stereoisomers.

The term "racemate" or "racemic mixture" refers to a composition composed of equimolar quantities of two enantiomeric species, wherein the composition is devoid of optical activity.

The term "tautomer" refers to each of two or more isomers of a compound that exist together in equilibrium and are readily interchanged by migration of an atom or group within the molecule.

The term "halogen" or "halogen atoms" or "halo" as used herein includes fluorine, chlorine, bromine, and iodine atom.

The term "(Cₓ-C_{y})alkyl" wherein x and y are integers, refers to a straight or branched chain alkyl group having from x to y carbon atoms. Thus, when x is 1 and y is 6, for example, the term includes methyl, ethyl, n-propyl, isopropyl, n-butyl, isobutyl, sec-butyl, t-butyl, n-pentyl and n-hexyl.

The term "(Cₓ-C_{y})alkoxy" wherein x and y are integers, refers to a straight or branched hydrocarbon of the indicated number of carbons, linked to the rest of the molecule through an oxygen bridge.

The term "(Cₓ-C_{y})alkylene" wherein x and y are integers, refers to a Cₓ-C_{y}alkyl radical having in total two unsatisfied valencies, such as a divalent methylene radical.

The expressions "(Cₓ-C_{y})haloalkyl" wherein x and y are integers, refer to the above defined "Cₓ-C_{y}alkyl" groups wherein one or more hydrogen atoms are replaced by one or more halogen atoms, which can be the same or different.

Examples of said "(Cₓ-C_{y})haloalkyl" groups may thus include halogenated, poly-halogenated and fully halogenated alkyl groups wherein all hydrogen atoms are replaced by halogen atoms, e.g. trifluoromethyl.

The term "(Cₓ-C_{y})cycloalkyl" wherein x and y are integers, refers to saturated cyclic hydrocarbon groups containing the indicated number of ring carbon atoms. Examples include cyclopropyl, cyclobutyl, cyclopentyl, cyclohexyl, cycloheptyl.

The term "aryl" refers to mono cyclic carbon ring systems which have 6-membered ring wherein the ring is aromatic. Examples of suitable aryl monocyclic ring systems include, for instance, phenyl.

The term "heteroaryl" refers to a mono- or bi-cyclic aromatic group containing one or more heteroatoms selected from S, N and O, and includes groups having two such monocyclic rings, or one such monocyclic ring and one monocyclic aryl ring, which are fused through a common bond.

The term "(Cₓ-C_{y})heterocycloalkyl" wherein x and y are integers, refers to saturated or partially unsaturated monocyclic (Cₓ-C_{y})cycloalkyl groups in which at least one ring carbon atom is replaced by at least one heteroatom (e.g. N, S or O) or may bear an -oxo (=O) substituent group. Said heterocycloalkyl may be further optionally substituted on the available positions in the ring, namely on a carbon atom, or on a heteroatom available for substitution. Substitution may be on a carbon atom including spiro disubstitution, forming bicyclic system where two heterocyclic rings are connected through a single carbon atom, as well as on two adjacent carbon atoms forming an additional condensed 5 to 6 membered heterocycloalkyl ring. Moreover, said heterocycloalkyl may be a diazabicyclo ring.

The term "(Cₓ-C_{y})hydroxyalkyl" wherein x and y are integers, refers to the above defined "(C₁-C₆)alkyl" groups wherein one or more hydrogen atoms are replaced by one or more hydroxy (OH) group.

Throughout the specification the use of an asterisk "*" in the definition of a structural formula, indicates the point of attachment for the radical group to the rest of the molecule.

A dash ("-") that is not between two letters or symbols is meant to represent the point of attachment for a substituent.

The carbonyl group is herein preferably represented as -C(O)- as an alternative to the other common representations such as -CO-, -(CO)- or -C(=O)-.

In general, the bracketed group is a lateral group, not included into the chain, and brackets are used, when deemed useful, to help disambiguating linear chemical formulas; e.g. the sulfonyl group -SO₂- might be also represented as -S(O)₂- to disambiguate e.g. with respect to the sulfinic group -S(O)O-.

The present invention relates to novel compounds differing from the structures disclosed in the art at least for a common new core scaffold. In fact the invention relates to compounds that are 2,3-dihydro-1H-pyrido[3,4-b][1,4]oxazine derivatives, wherein the nitrogen of the oxazine is linked to a pyridine or a pyridine fused to a 5-membered heterocyclic ring, which are inhibitors of receptor ALK5, that have therapeutically desirable characteristics, particularly promising for some fibrosis, including idiopathic pulmonary fibrosis (IPF).

The compounds of the invention are active as inhibitors of ALK5 receptor, they are potent and show improved properties such as a good inhalatory profile, a low metabolic stability, a low systemic exposure, improved safety and tolerability, and a good selectivity across the kinome.

In this respect, the state of the art does not describe or suggest pyrido oxazine derivatives of general formula (I) of the present invention having inhibitory activity on receptor ALK5 which represents a solution to the aforementioned need.

WO2013/093849 (Novartis) discloses, among other compounds, pyrido oxazine derivatives. The compounds of formula (I) of the present invention differ from the Novartis' ones for the substituents on the pyridine condensed to the oxazine ring and, also, for the substituents on the pyridine linked to the oxazine ring.

Novartis discloses compounds as inhibitors of Pi3K. Novartis neither discloses compounds as ALK5 inhibitors, nor compounds for the treatment of fibrosis.

In more details, the present invention refers to a series of compounds represented by the general formula (I) as herein below described in details, which are endowed with an inhhibitory activity on receptor ALK5.

Advantageously, the inhibitory action on receptor ALK5 can be effective in the treatment of those diseases where these receptors play a relevant role in the pathogenesis such as fibrosis and disease, disorder and condition from fibrosis.

Differently from similar compounds of the prior art, the compounds of formula (I) of the present invention are able to act as inhibitors of ALK5 receptor, particularly appreciated by the skilled person when looking at a suitable and efficacious compounds useful for the treatment of fibrosis, in particular idiopathic pulmonary fibrosis.

As indicated in the experimental part, in particular in Table 4, the compounds of formula (I) of the present invention show a notable potency with respect to their inhibitory activity on receptor ALK5, below about 10 nM, confirming that they are able to inhibit ALK5 receptor involved in fibrosis and diseases that result from fibrosis.

In the experimental part, comparative examples, in particular in Table 5, it is shown that, conversely to the compounds C1 and C2 characterized by a pyrido oxazine ring wherein the oxazine is linked to a 5-membered heterocyclic ring, in the compounds of the present invention, characterized by the oxazine ring linked to a pyridine or pyridine fused to a 5-membered heterocyclic ring, the presence of such pyridine or pyridine fused to a 5-membered heterocyclic ring unexpectedly and remarkably determines a relevant increase in the inhibitory activity on the ALK5 receptor.

Advantageously, the compounds of the present invention are endowed by a very high potency, they could be administered in human at a lower dosage respect to the compounds of the prior art, thus reducing the adverse events that typically occur administering higher dosages of drug.

In addition to being notably potent with respect to their inhibitory activity on receptor ALK5, the compounds of the present invention are also characterized by a good inhalatory profile, that permits to act effectively in the lung compartment and have, at the same time, a low metabolic stability, that allows to minimize the drawbacks associated with the systemic exposure, such as safety and tolerability issues.

Therefore, the compounds of the present invention are particularly appreciated by the skilled person when looking at a suitable and efficacious compounds useful for the treatment of fibrosis, in particular idiopathic pulmonary fibrosis, administered by the inhalation route and characterized by a good inhalatory profile, that corresponds to a good activity on the lung, a good lung retention and to a low metabolic stability, that minimizes the systemic exposure and correlated safety issues.

Thus, in one aspect the present invention relates to a compound of general formula (I) 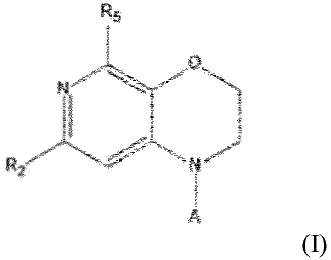 wherein
**A** is selected from the groups consisting of **A1** and **A2** 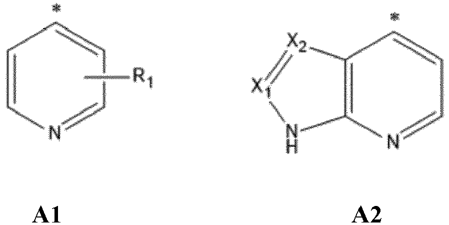
**R₁** is H or is selected from the group consisting of -NR₃C(O)R₄ and -NR₃R_{3'};
**X₁** and **X₂** are independently N or CH;
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄** is selected from the group consisting of -(C₁-C₆)alkyl, -NR_{A}R_{B}, -(C₁-C₆)alkylene-NR_{A}R_{B}, -(C₁-C₆)alkylene-O-(C₁₋C₆)alkyl, -NH(C₁-C₆)alkylene-heterocycloalkyl and -(C₁-C₆)alkylene-heterocycloalkyl, wherein said heterocycloalkyl is optionally substituted by one or more groups selected from oxo, alkoxy, -(C₁₋C₆)alkylene-OH, -C(O)O-(C₁-C₆)alkyl, -(C₁₋C₆)alkylene-C(O)O-(C₁-C₆)alkyl, -(C₁₋C₆)alkylene-C(O)NH-(C₁₋C₆)alkyl, -(C₁₋C₆)alkylene-C(O)NH-(C₁₋C₆)alkyl-OH, -(C₁₋C₆)alkylene-NHSO₂-(C₁₋C₆)alkyl, -(C₁₋C₆)alkylene-CONH₂, -(C₁₋C₆)alkylene-NR_{A}R_{B}, and -(C₁-C₆)alkyl;
**R_{A}** is H or is selected from the group consisting of -(C₁-C₆)alkyl, heterocycloalkyl and -(C₁-C₆)hydroxyalkyl;
**R_{B}** is selected from the group consisting of -(C₁-C₆)alkyl, -(C₁-C₆)alkylene-C(O)O-(C₁-C₆)alkyl, heterocycloalkyl, -(C₁-C₆)alkylene-heterocycloalkyl, -SO₂-(C₁-C₆)alkyl, -(C₁-C₆)alkylene-NH-SO₂-(C₁-C₆)alkyl, -(C₁-C₆)alkylene-SO₂-(C₁₋C₆)alkyl and -(C₁-C₆)hydroxyalkyl, wherein said heterocycloalkyl of the -(C₁-C₆)alkylene-heterocycloalkyl may be optionally substituted by one or more -(C₁-C₆)alkyl;
**R₅** is H or selected from the group consisting of -NH₂, -C(O)OH, -C(O)O-(C₁₋C₆)alkyl, -(C₁-C₆)haloalkyl and -OH;
   and pharmaceutically acceptable salts thereof.

In another aspect the present invention relates to a compound of general formula (I) as ALK5 inhibitors 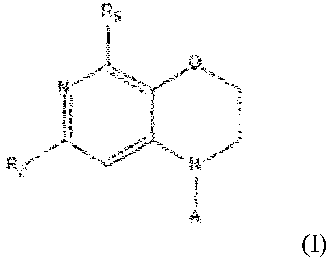 wherein
**A** is selected from the groups consisting of **A1** and **A2** 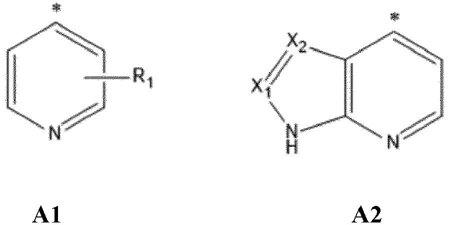
**R₁** is H or is selected from the group consisting of -NR₃C(O)R₄ and -NR₃R_{3'};
**X₁** and **X₂** are independently N or CH;
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄ is** selected from the group consisting of -(C₁-C₆)alkyl, -(C₁₋C₆)alkylene-NR_{A}R_{B}, and -(C₁-C₆)alkylene-heterocycloalkyl, wherein said heterocycloalkyl is optionally substituted by one or more groups selected from oxo and -(C₁-C₆)alkyl;
**R_{A}** is H or is selected from the group consisting of -(C₁-C₆)alkyl, heterocycloalkyl and -(C₁-C₆)hydroxyalkyl;
**R_{B}** is selected from the group consisting of -(C₁-C₆)alkyl, -(C₁₋C₆)alkylene-C(O)O-(C₁-C₆)alkyl, heterocycloalkyl, -SO₂-(C₁₋C₆)alkyl, -(C₁₋C₆)alkylene-SO₂-(C₁₋C₆)alkyl and -(C₁-C₆)hydroxyalkyl;
**R₅** is H or -NH₂;
   and pharmaceutically acceptable salts thereof.

In a preferred embodiment, **R₅** is H.

In a preferred embodiment **R₂** is phenyl substituted by one or two groups selected from fluorine, chlorine, methyl, cyclopropyl and isopropyl or **R₂** is pyridyl optionally substituted by one or more fluorine or chlorine.

In a particularly preferred embodiment the present invention refers to a compound of formula (I), wherein **A** is **A1** 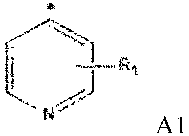 represented by the formula (Ia) 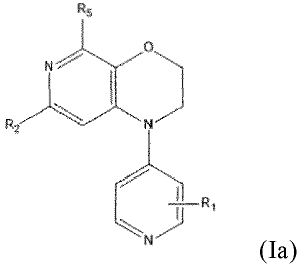
**R₁** is H or is selected from the group consisting of -NR₃C(O)R₄ and -NR₃R_{3'};
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄** is selected from the group consisting of -(C₁-C₆)alkyl, -NR_{A}R_{B}, -(C₁-C₆)alkylene-NR_{A}R_{B}, -(C₁-C₆)alkylene-O-(C₁₋C₆)alkyl, -NH(C₁-C₆)alkylene-heterocycloalkyl and -(C₁-C₆)alkylene-heterocycloalkyl, wherein said heterocycloalkyl is optionally substituted by one or more groups selected from oxo, alkoxy, -(C₁₋C₆)alkylene-OH, -C(O)O-(C₁-C₆)alkyl, -(C₁₋C₆)alkylene-C(O)O-(C₁-C₆)alkyl, -(C₁₋C₆)alkylene-C(O)NH-(C₁₋C₆)alkyl, -(C₁₋C₆)alkylene-C(O)NH-(C₁₋C₆)alkyl-OH, -(C₁₋C₆)alkylene-NHSO₂-(C₁₋C₆)alkyl, -(C₁₋C₆)alkylene-CONH₂, -(C₁₋C₆)alkylene-NR_{A}R_{B}, and -(C₁-C₆)alkyl;
**R_{A}** is H or is selected from the group consisting of -(C₁-C₆)alkyl, heterocycloalkyl and -(C₁-C₆)hydroxyalkyl;
**R_{B}** is selected from the group consisting of -(C₁-C₆)alkyl, -(C₁-C₆)alkylene-C(O)O-(C₁-C₆)alkyl, heterocycloalkyl, -(C₁-C₆)alkylene-heterocycloalkyl, -SO₂-(C₁-C₆)alkyl, -(C₁-C₆)alkylene-NH-SO₂-(C₁-C₆)alkyl, -(C₁-C₆)alkylene-SO₂-(C₁₋C₆)alkyl and -(C₁-C₆)hydroxyalkyl, wherein said heterocycloalkyl of the -(C₁-C₆)alkylene-heterocycloalkyl may be optionally substituted by one or more -(C₁-C₆)alkyl;
**R₅** is H or selected from the group consisting of -NH₂, -C(O)OH, -C(O)O-(C₁₋C₆)alkyl, -(C₁-C₆)haloalkyl and -OH;
and pharmaceutically acceptable salts thereof.

According to a preferred embodiment, the invention refers to at least one of the compounds of Formula (Ia) listed in the Table 1 below and pharmaceutically acceptable salts thereof.

**Table 1: List of preferred compounds of Formula (Ia)**

| **Example No.** | **Structure** | **Chemical Name** |
|---|---|---|
| 1 | 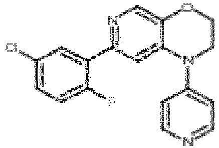 | 4-[7-(5-chloro-2-fluorophenyl)-1H,2H,3H-pyrido[3,4-b][1,4]oxazin-1-yl]pyridine |
| 2 | 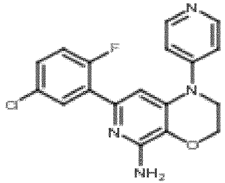 | 7-(5-chloro-2-fluorophenyl)-1-(pyridin-4-yl)-1H,2H,3H-pyrido[3,4-b][1,4]oxazin-5-amine |
| 3 | 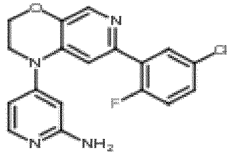 | 4-[7-(5-chloro-2-fluorophenyl)-1H,2H,3H-pyrido[3,4-b][1,4]oxazin-1-yl]pyridin-2-amine |
| 4 | 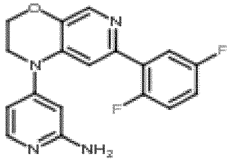 | 4-[7-(2,5-difluorophenyl)-1H,2H,3H-pyrido[3,4-b][1,4]oxazin-1-yl]pyridin-2-amine |
| 5 | 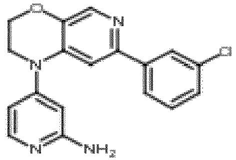 | 4-[7-(3-chlorophenyl)-1H,2H,3H-pyrido[3,4-b][1,4]oxazin-1-yl]pyridin-2-amine |
| 6 | 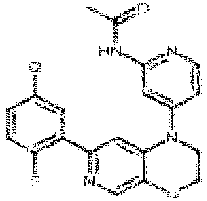 | N- { 4-[7-(5-chloro-2-fluorophenyl)-1H,2H,3H-pyrido[3,4-b][1,4]oxazin-1-yl]pyridin-2-yl} acetamide |
| 7 | 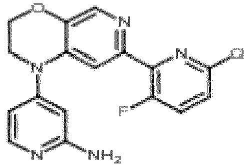 | [7-(6-chloro-3-fluoropyridin-2-yl)-1H,2H,3H-pyrido[3,4-b][1,4]oxazin-1-yl]pyridin-2-amine |
| 8 | 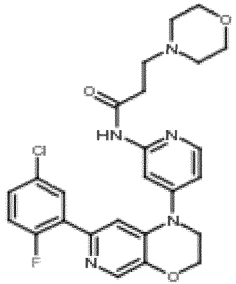 | N-{4-[7-(5-chloro-2-fluorophenyl)-1H,2H,3H-pyrido[3,4-b][1,4]oxazin-1-yl]pyridin-2-yl}-3-(morpholin-4-yl)propanamide |
| 9 | 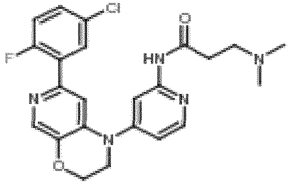 | N-{4-[7-(5-chloro-2-fluorophenyl)-1H,2H,3H-pyrido[3,4-b][1,4]oxazin-1-yl]pyridin-2-yl}-3-(dimethylamino)propanamide |
| 10 | 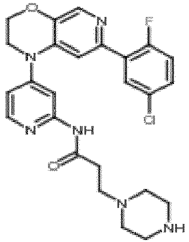 | N-{4-[7-(5-chloro-2-fluorophenyl)-1H,2H,3H-pyrido[3,4-b][1,4]oxazin-1-yl]pyridin-2-yl}-3-(piperazin-1-yl)propanamide |
| 11 | 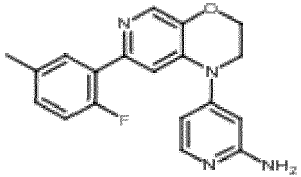 | 4-[7-(2-fluoro-5-methylphenyl)-1H,2H,3H-pyrido[3,4-b][1,4]oxazin-1-yl]pyridin-2-amine |
| 12 | 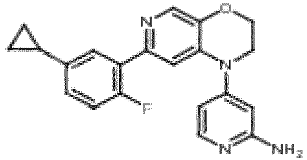 | 4-[7-(5-cyclopropyl-2-fluorophenyl)-1H,2H,3H-pyrido[3,4-b][1,4]oxazin-1-yl]pyridin-2-amine |
| 13 | 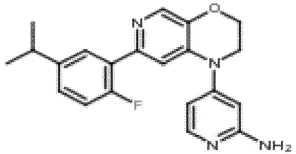 | 4-{7-[2-fluoro-5-(propan-2-yl)phenyl]-1H,2H,3H-pyrido[3,4-b][1,4]oxazin-1-yl}pyridin-2-amine |
| 14 | 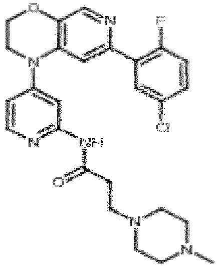 | N-{4-[7-(5-chloro-2-fluorophenyl)-1H,2H,3H-pyrido[3,4-b][1,4]oxazin-1-yl]pyridin-2-yl}-3-(4-methylpiperazin-1-yl)propanamide |
| 18 | 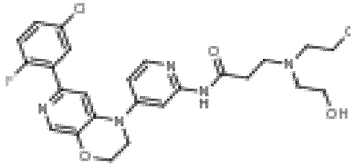 | 3-[bis(2-hydroxyethyl)amino]-N-{4-[7-(5-chloro-2-fluorophenyl)-1H,2H,3H-pyrido[3,4-b][1,4]oxazin-1-yl]pyridin-2-yl}propanamide |
| 19 | 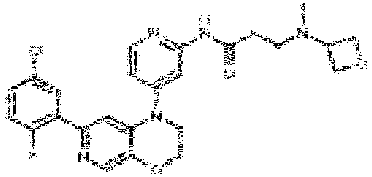 | N-{4-[7-(5-chloro-2-fluorophenyl)-1H,2H,3H-pyrido[3,4-b][1,4]oxazin-1-yl]pyridin-2-yl}-3-[methyl(oxetan-3- yl)amino]propanamide |
| 20 | 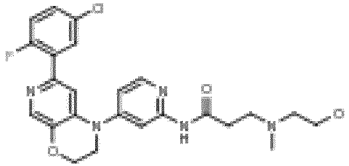 | N-{4-[7-(5-chloro-2-fluorophenyl)-1H,2H,3H-pyrido[3,4-b][1,4]oxazin-1-yl]pyridin-2-yl}-3-[(2-hydroxyethyl)(methyl)amino]propan amide |
| 21 | 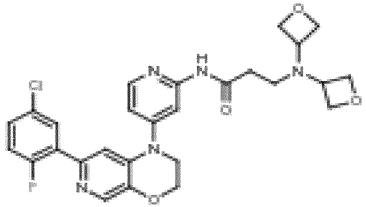 | 3-[bis(oxetan-3-yl)amino]-N-{4-[7-(5-chloro-2-fluorophenyl)-1H,2H,3H-pyrido[3,4-b][1,4]oxazin-1-yl]pyridin-2-yl}propanamide |
| 22 | 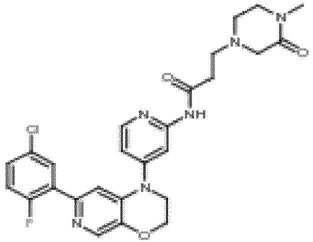 | N-{4-[7-(5-chloro-2-fluorophenyl)-1H,2H,3H-pyrido[3,4-b][1,4]oxazin-1-yl]pyridin-2-yl}-3-(4-methyl-3-oxopiperazin-1-yl)propanamide |
| 23 | 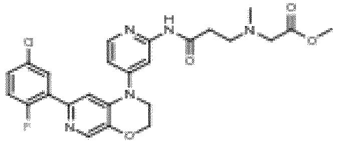 | methyl 2-{[2-({4-[7-(5-chloro-2-fluorophenyl)-1H,2H,3H-pyrido[3,4-b][1,4]oxazin-1-yl]pyridin-2-yl}carbamoyl)ethyl](methyl)amino} acetate |
| 24 | 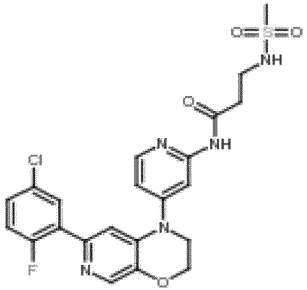 | N-{4-[7-(5-chloro-2-fluorophenyl)-1H,2H,3H-pyrido[3,4-b][1,4]oxazin-1-yl]pyridin-2-yl}-3-methanesulfonamidopropanamide |
| 25 | 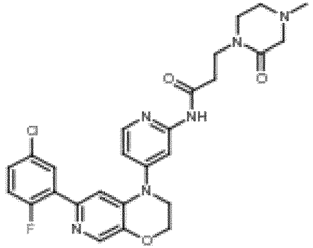 | N-{4-[7-(5-chloro-2-fluorophenyl)-1H,2H,3H-pyrido[3,4-b][1,4]oxazin-1-yl]pyridin-2-yl}-3-(4-methyl-2-oxopiperazin-1-yl)propanamide |
| 26 | 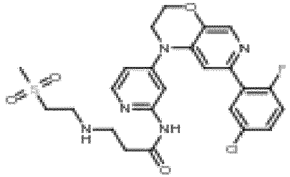 | N-{4-[7-(5-chloro-2-fluorophenyl)-1H,2H,3H-pyrido[3,4-b][1,4]oxazin-1-yl]pyridin-2-yl}-3-[(2-methanesulfonylethyl)amino]propan amide |
| 27 | 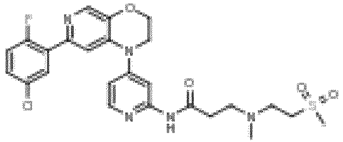 | N-{4-[7-(5-chloro-2-fluorophenyl)-1H,2H,3H-pyrido[3,4-b][1,4]oxazin-1-yl]pyridin-2-yl}-3-[(2-methanesulfonylethyl)(methyl)amin o]propanamide |
| 28 | 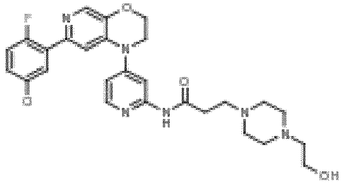 | N-{4-[7-(5-chloro-2-fluorophenyl)-1H,2H,3H-pyrido[3,4-b][1,4]oxazin-1-yl]pyridin-2-yl}-3-[4-(2-hydroxyethyl)piperazin-1-yl]propanamide |
| 29 | 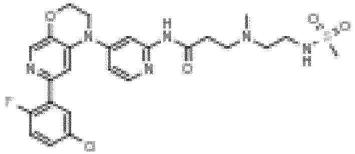 | N-{4-[7-(5-chloro-2-fluorophenyl)-1H,2H,3H-pyrido[3,4-b][1,4]oxazin-1-yl]pyridin-2-yl}-3-[(2-methanesulfonamidoethyl)(methyl)a mino]propanamide |
| 30 | 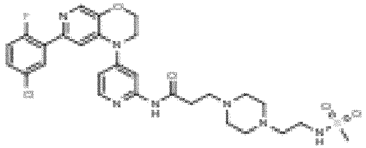 | N-{4-[7-(5-chloro-2-fluorophenyl)-1H,2H,3H-pyrido[3,4-b][1,4]oxazin-1-yl]pyridin-2-yl}-3-[4-(2-methanesulfonamidoethyl)piperazin -1-yl]propanamide |
| 31 | 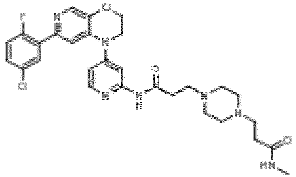 | 3-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-1-yl}-N-methylpropanamide |
| 32 | 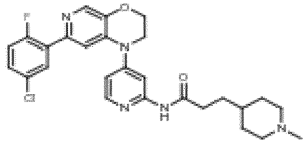 | N-{4-[7-(5-chloro-2-fluorophenyl)-1H,2H,3H-pyrido[3,4-b][1,4]oxazin-1-yl]pyridin-2-yl}-3-(1-methylpiperidin-4-yl)propanamide |
| 33 | 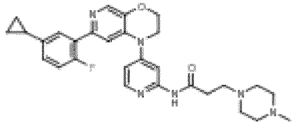 | N-{4-[7-(5-cyclopropyl-2-fluorophenyl)-1H,2H,3H-pyrido[3,4-b][1,4]oxazin-1-yl]pyridin-2-yl}-3-(4-methylpiperazin-1-yl)propanamide |
| 34 | 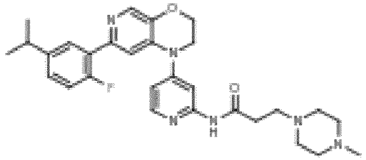 | N-(4-{7-[2-fluoro-5-(propan-2-yl)phenyl]-1H,2H,3H-pyrido[3,4-b][1,4]oxazin-1-yl}pyridin-2-yl)-3-(4-methylpiperazin-1- yl)propanamide |
| 35 | 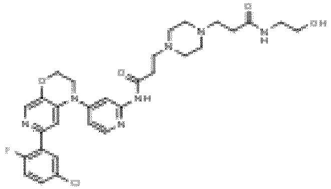 | 3-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-1-yl}-N-(2-hydroxyethyl)propanamide |
| 36 | 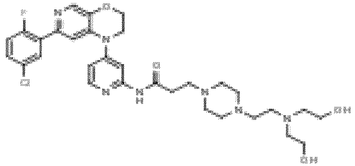 | 3-(4-{2-[bis(2-hydroxyethyl)amino]ethyl}piperazin -1-yl)-N-{4-[7-(5-chloro-2-fluorophenyl)-1H,2H,3H-pyrido[3,4-b][1,4]oxazin-1-yl]pyridin-2-yl}propanamide |
| 37 | 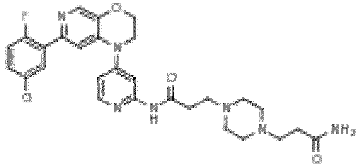 | 3-[4-(2-carbamoylethyl)piperazin-1-yl]-N-{4-[7-(5-chloro-2-fluorophenyl)-1H,2H,3H-pyrido[3,4-b][1,4]oxazin-1-yl]pyridin-2-yl}propanamide |
| 38 | 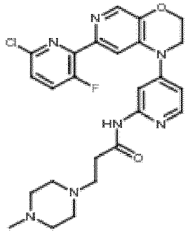 | N-{4-[7-(6-chloro-3 -fluoropyridin-2-yl)-1H,2H,3H-pyrido[3,4-b][1,4]oxazin-1-yl]pyridin-2-yl(-3-(4-methylpiperazin-1- yl)propanamide |
| 39 | 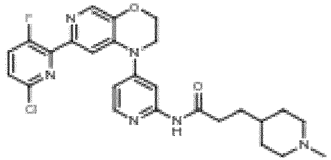 | N-{4-[7-(6-chloro-3-fluoropyridin-2-yl)-1H,2H,3H-pyrido[3,4-b][1,4]oxazin-1-yl]pyridin-2-yl}-3-(1-methylpiperidin-4-yl)propanamide |
| 40 | 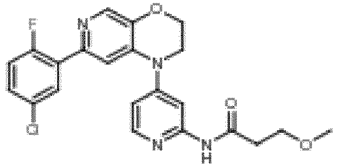 | N-{4-[7-(5-chloro-2-fluorophenyl)-1H,2H,3H-pyrido[3,4-b][1,4]oxazin-1-yl]pyridin-2-yl}-3-methoxypropanamide |
| 41 | 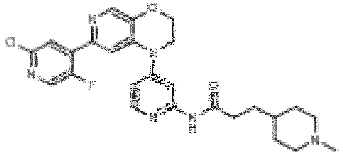 | N-{4-[7-(2-chloro-5-fluoropyridin-4-yl)-1H,2H,3H-pyrido[3,4-b][1,4]oxazin-1-yl]pyridin-2-yl}-3-(1-methylpiperidin-4-yl)propanamide |
| 42 | 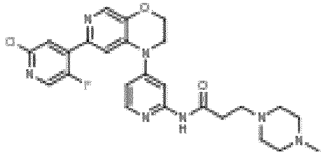 | N-{4-[7-(2-chloro-5-fluoropyridin-4-yl)-1H,2H,3H-pyrido[3,4-b][1,4]oxazin-1-yl]pyridin-2-yl}-3-(4-methylpiperazin-1-yl)propanamide |
| 43 | 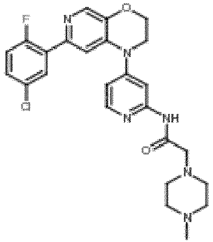 | N-{4-[7-(5-chloro-2-fluorophenyl)-1H,2H,3H-pyrido[3,4-b][1,4]oxazin-1-yl]pyridin-2-yl}-2-(4-methylpiperazin-1-yl)acetamide |
| 44 | 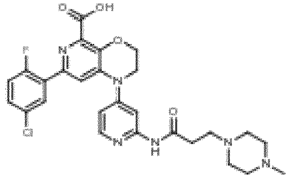 | 7-(5-chloro-2-fluorophenyl)-1-{2-[3-(4-methylpiperazin-1-yl)propanamido]pyridin-4-yl}-1H,2H,3H-pyrido[3,4-b][1,4]oxazine-5-carboxylic acid |
| 45 | 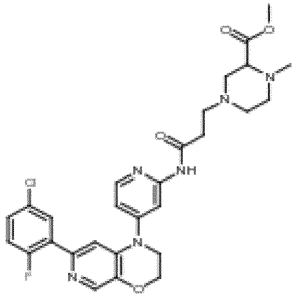 | Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazine-2-carboxylate |
| 46 | 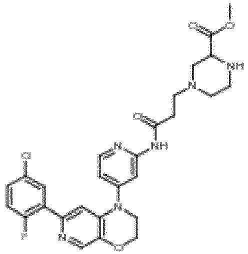 | Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]piperazine-2-carboxylate |
| 47 | 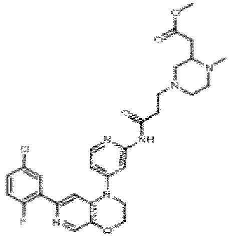 | Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazin-2-yl}acetate |
| 48 | 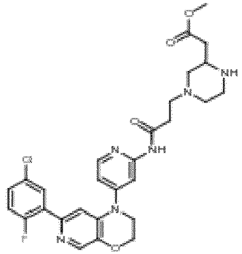 | Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-2-yl}acetate |
| 49 | 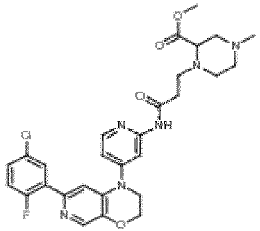 | Methyl 1-[2-({4-[7-(5-chloro-2-fluorophenyl)-1H,2H,3H-pyrido[3,4-b][1,4]oxazin-1-yl]pyridin-2-yl}carbamoyl)ethyl]-4-methylpiperazine-2-carboxylate |
| 50 | 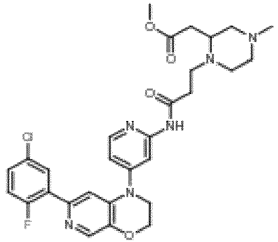 | Methyl 2-{1-[2-({4-[7-(5-chloro-2-fluorophenyl)-1H,2H,3H-pyrido[3,4-b][1,4]oxazin-1-yl]pyridin-2-yl}carbamoyl)ethyl]-4-methylpiperazin-2-yl } acetate |
| 51 | 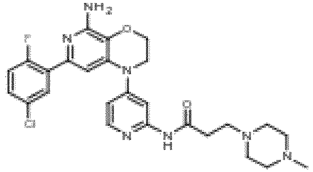 | N-{4-[5-amino-7-(5-chloro-2-fluorophenyl)-1H,2H,3H-pyrido[3,4-b][1,4]oxazin-1-yl]pyridin-2-yl}-3-(4-methylpiperazin-1-yl)propanamide |
| 52 | 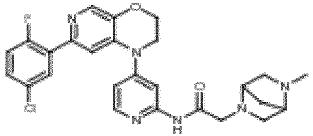 | N-{4-[7-(5-chloro-2-fluorophenyl)-1H,2H,3H-pyrido[3,4-b][1,4]oxazin-1-yl]pyridin-2-yl}-2-{ 5-methyl-2,5-diazabicyclo[2.2.1]heptan-2-yl} acetamide |
| 53 | 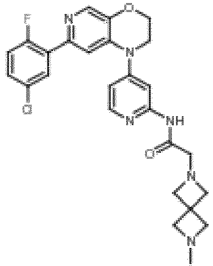 | N-{4-[7-(5-chloro-2-fluorophenyl)-1H,2H,3H-pyrido[3,4-b][1,4]oxazin-1-yl]pyridin-2-yl}-2-{6-methyl-2,6-diazaspiro[3.3]heptan-2-yl}acetamide |
| 54 | 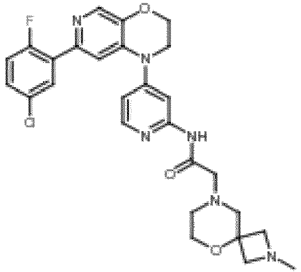 | N-{4-[7-(5-chloro-2-fluorophenyl)-1H,2H,3H-pyrido[3,4-b][1,4]oxazin-1-yl]pyridin-2-yl}-2-{2-methyl-5-oxa-2,8-diazaspiro[3.5]nonan-8-yl} acetamide |
| 55 | 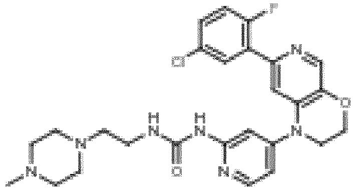 | 1-(4-(7-(5-chloro-2-fluorophenyl)-2,3-dihydro-1H-pyrido[3,4-b][1,4]oxazin-1-yl)pyridin-2-yl)-3- (2-(4-methylpiperazin-1- yl)ethyl)urea |
| 56 | 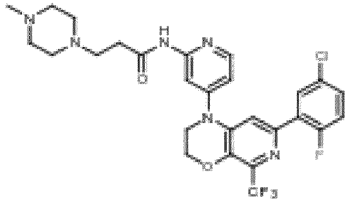 | N-(3-(7-(5-chloro-2-fluorophenyl)-5-(trifluoromethyl)-2,3-dihydro-1H-pyrido[3,4-b][1,4]oxazin-1-yl)phenyl)-3-(4-methylpiperazin-1-yl)propanamide |

In a more preferred embodiment the present invention refers to a compound of formula (I), wherein **A** is **A1a** 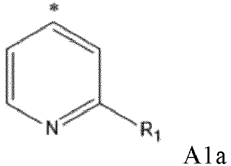 represented by the formula (Iaa) 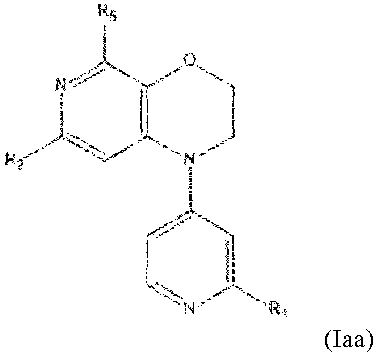
**R₁** is selected from the group consisting of -NR₃C(O)R₄ and -NR₃R_{3'};
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄** is selected from the group consisting of -(C₁-C₆)alkyl, -NR_{A}R_{B}, -(C₁₋C₆)alkylene-NR_{A}R_{B}, -(C₁-C₆)alkylene-O-(C₁₋C₆)alkyl, -NH(C₁-C₆)alkylene-heterocycloalkyl and -(C₁-C₆)alkylene-heterocycloalkyl, wherein said heterocycloalkyl is optionally substituted by one or more groups selected from oxo, alkoxy, -(C₁₋C₆)alkylene-OH, -C(O)O-(C₁-C₆)alkyl, -(C₁₋C₆)alkylene-C(O)O-(C₁-C₆)alkyl, -(C₁₋C₆)alkylene-C(O)NH-(C₁₋C₆)alkyl, -(C₁₋C₆)alkylene-C(O)NH-(C₁₋C₆)alkyl-OH, -(C₁₋C₆)alkylene-NHSO₂-(C₁₋C₆)alkyl, -(C₁₋C₆)alkylene-CONH₂, -(C₁₋C₆)alkylene-NR_{A}R_{B}, and -(C₁-C₆)alkyl;
**R_{A}** is H or is selected from the group consisting of -(C₁-C₆)alkyl, heterocycloalkyl and -(C₁-C₆)hydroxyalkyl;
**R_{B}** is selected from the group consisting of -(C₁-C₆)alkyl, -(C₁-C₆)alkylene-C(O)O-(C₁-C₆)alkyl, heterocycloalkyl, -(C₁-C₆)alkylene-heterocycloalkyl, -SO₂-(C₁-C₆)alkyl, -(C₁-C₆)alkylene-NH-SO₂-(C₁-C₆)alkyl, -(C₁-C₆)alkylene-SO₂-(C₁₋C₆)alkyl and -(C₁-C₆)hydroxyalkyl, wherein said heterocycloalkyl of the -(C₁-C₆)alkylene-heterocycloalkyl may be optionally substituted by one or more -(C₁-C₆)alkyl;
**R₅** is H or selected from the group consisting of -NH₂, -C(O)OH, -C(O)O-(C₁₋C₆)alkyl, -(C₁-C₆)haloalkyl and -OH;
and pharmaceutically acceptable salts thereof.

In a even more preferred embodiment the present invention refers to a compound of formula (Iaa), wherein
**R₁** is -NHC(O)R₄;
**R₄** is selected from the group consisting of methyl, 4-ethylmorpholine, (dimethylamino)ethyl, 4-ethylpiperazine, 1-ethyl-4-methylpiperazine, 2-[bis(2-hydroxyethyl)amino]ethyl, -[(2-hydroxyethyl)(methyl)amino]ethyl, 3-[bis(oxetan-3-yl)amino]ethyl, -3-(4-methyl-3-oxopiperazin-1-yl)ethyl, methyl-2-(ethyl(methyl)amino)acetate, -ethylmethanesulfonamide, -3-(4-methyl-2-oxopiperazin-1-yl)ethyl, -3-[(2-methanesulfonylethyl)amino]ethyl, -3-[(2-methanesulfonylethyl)(methyl)amino]ethyl, -3-[4-(2-hydroxyethyl)piperazin-1-yl]ethyl, -3-[(2-methanesulfonamidoethyl)(methyl)amino]ethyl, -3-[4-(2-methanesulfonamidoethyl)piperazin-1-yl]ethyl, -(4-ethyl piperazin-1-yl)-N-methylpropanamide, -3-(1-methylpiperidin-4-yl)ethyl, -(4-ethyl)piperazin-1-yl-N-(2-hydroxyethyl)propanamide, 3-(4{2-[bis(2-hydroxyethyl)amino]ethyl}piperazin-1-yl)ethyl, 3-[4-(2-carbamoylethyl)piperazin-1-yl]ethyl, -3-methoxyethyl, -2-(4-methylpiperazin-1-yl)methyl, methyl 4-(ethyl)-1-methylpiperazine-2-carboxylate, methyl 4-(ethyl)-piperazine-2-carboxylate, methyl 2-[4-(ethyl)-1-methylpiperazin-2-yl]acetate, methyl 2-[4-(ethyl)-piperazin-2-yl]acetate, methyl 1- (ethyl)-4-methylpiperazine-2-carboxylate, methyl 2-[1-(ethyl)-4-methylpiperazin-2-yl]acetate, 2-{5-methyl-2,5-diazabicyclo[2.2.1]heptan-2-yl}methyl, 2-{6-methyl-2,6-diazaspiro[3.3]heptan-2-yl}methyl, 2-{2-methyl-5-oxa-2,8-diazaspiro[3.5]nonan-8-yl}methyl and -3-(2-(4-methylpiperazin-1-yl)ethyl)urea.

According to a preferred embodiment, the invention refers to at least one of the compounds of Formula (Iaa) listed in the Table 2 below and pharmaceutically acceptable salts thereof. These compounds are particularly active on receptor ALK5, as shown in Table 4.

**Table 2: List of preferred compounds of Formula (Iaa)**

| **Example No.** | **Structure** | **Chemical Name** |
|---|---|---|
| 6 | 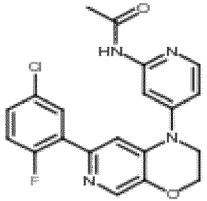 | N-{4-[7-(5-chloro-2-fluorophenyl)-1H,2H,3H-pyrido[3,4-b][1,4]oxazin-1-yl]pyridin-2-yl} acetamide |
| 8 | 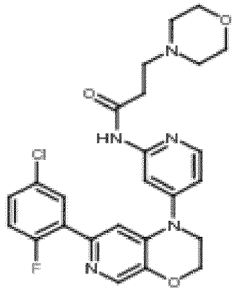 | N-{4-[7-(5-chloro-2-fluorophenyl)-1H,2H,3H-pyrido[3,4-b][1,4]oxazin-1-yl]pyridin-2-yl}-3-(morpholin-4-yl)propanamide |
| 9 | 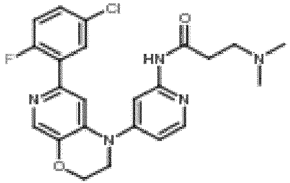 | N-{4-[7-(5-chloro-2-fluorophenyl)-1H,2H,3H-pyrido[3,4-b][1,4]oxazin-1-yl]pyridin-2-yl}-3-(dimethylamino)propanamide |
| 10 | 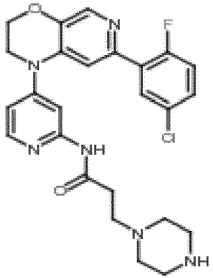 | N-{4-[7-(5-chloro-2-fluorophenyl)-1H,2H,3H-pyrido[3,4-b][1,4]oxazin-1-yl]pyridin-2-yl}-3-(piperazin-1-yl)propanamide |
| 14 | 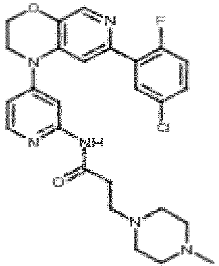 | N-{4-[7-(5-chloro-2-fluorophenyl)-1H,2H,3H-pyrido[3,4-b][1,4]oxazin-1-yl]pyridin-2-yl}-3-(4-methylpiperazin-1-yl)propanamide |
| 18 | 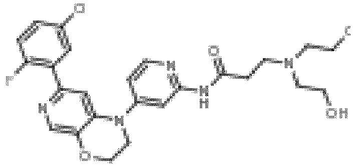 | 3-[bis(2-hydroxyethyl)amino]-N-{4-[7-(5-chloro-2-fluorophenyl)-1H,2H,3H-pyrido[3,4-b][1,4]oxazin-1-yl]pyridin-2-yl }propanamide |
| 19 | 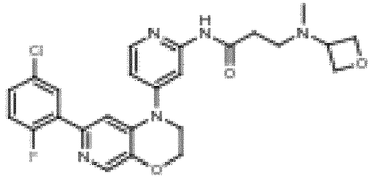 | N-{4-[7-(5-chloro-2-fluorophenyl)-1H,2H,3H-pyrido[3,4-b][1,4]oxazin-1-yl]pyridin-2-yl}-3-[methyl(oxetan-3-yl)amino]propanamide |
| 20 | 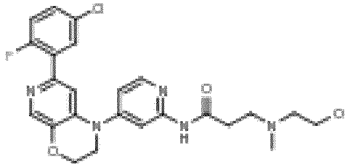 | N-{4-[7-(5-chloro-2-fluorophenyl)-1H,2H,3H-pyrido[3,4-b][1,4]oxazin-1-yl]pyridin-2-yl}-3-[(2-hydroxyethyl)(methyl)amino]propan amide |
| 21 | 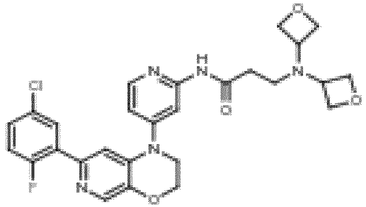 | 3-[bis(oxetan-3-yl)amino]-N-{4-[7-(5-chloro-2-fluorophenyl)-1H,2H,3H-pyrido[3,4-b][1,4]oxazin-1-yl]pyridin-2-yl }propanamide |
| 22 | 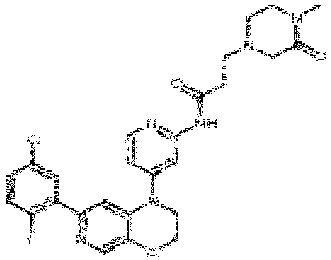 | N-{4-[7-(5-chloro-2-fluorophenyl)-1H,2H,3H-pyrido[3,4-b][1,4]oxazin-1-yl]pyridin-2-yl}-3-(4-methyl-3-oxopiperazin-1-yl)propanamide |
| 23 | 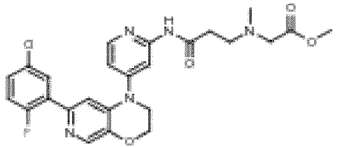 | methyl 2-{[2-({4-[7-(5-chloro-2-fluorophenyl)-1H,2H,3H-pyrido[3,4-b][1,4]oxazin-1-yl]pyridin-2-yl}carbamoyl)ethyl](methyl)amino} acetate |
| 24 | 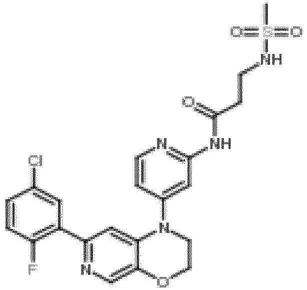 | N-{4-[7-(5-chloro-2-fluorophenyl)-1H,2H,3H-pyrido[3,4-b][1,4]oxazin-1-yl]pyridin-2-yl}-3-methanesulfonamidopropanamide |
| 25 | 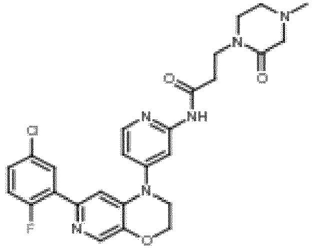 | N-{4-[7-(5-chloro-2-fluorophenyl)-1H,2H,3H-pyrido[3,4-b][1,4]oxazin-1-yl]pyridin-2-yl}-3-(4-methyl-2-oxopiperazin-1-yl)propanamide |
| 26 | 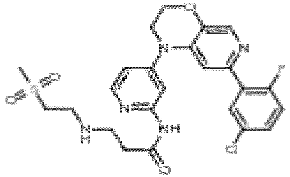 | N-{4-[7-(5-chloro-2-fluorophenyl)-1H,2H,3H-pyrido[3,4-b][1,4]oxazin-1-yl]pyridin-2-yl}-3-[(2-methanesulfonylethyl)amino]propan amide |
| 27 | 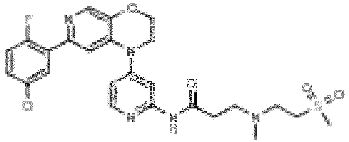 | N-{4-[7-(5-chloro-2-fluorophenyl)-1H,2H,3H-pyrido[3,4-b][1,4]oxazin-1-yl]pyridin-2-yl}-3-[(2-methanesulfonylethyl)(methyl)amin o]propanamide |
| 28 | 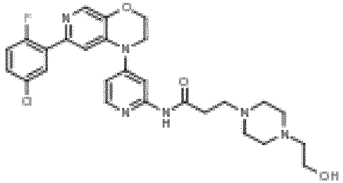 | N-{4-[7-(5-chloro-2-fluorophenyl)-1H,2H,3H-pyrido[3,4-b][1,4]oxazin-1-yl]pyridin-2-yl}-3-[4-(2-hydroxyethyl)piperazin-1-yl]propanamide |
| 29 | 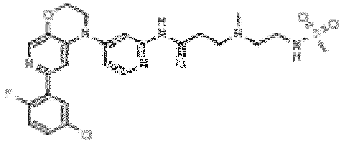 | N-{4-[7-(5-chloro-2-fluorophenyl)-1H,2H,3H-pyrido[3,4-b][1,4]oxazin-1-yl]pyridin-2-yl}-3-[(2-methanesulfonamidoethyl)(methyl)a mino]propanamide |
| 30 | 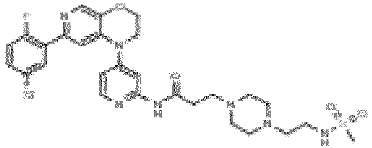 | N-{4-[7-(5-chloro-2-fluorophenyl)-1H,2H,3H-pyrido[3,4-b][1,4]oxazin-1-yl]pyridin-2-yl}-3-[4-(2-methanesulfonamidoethyl)piperazin-1-yl]propanamide |
| 31 | 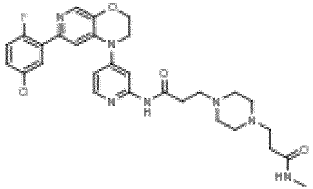 | 3-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-1-yl}-N-methylpropanamide |
| 32 | 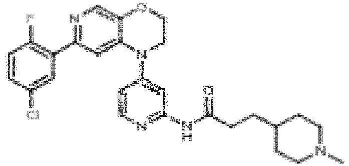 | N-{4-[7-(5-chloro-2-fluorophenyl)-1H,2H,3H-pyrido[3,4-b][1,4]oxazin-1-yl]pyridin-2-yl}-3-(1-methylpiperidin-4-yl)propanamide |
| 33 | 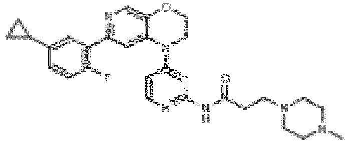 | N-{4-[7-(5-cyclopropyl-2-fluorophenyl)-1H,2H,3H-pyrido[3,4-b][1,4]oxazin-1-yl]pyridin-2-yl}-3-(4-methylpiperazin-1-yl)propanamide |
| 34 | 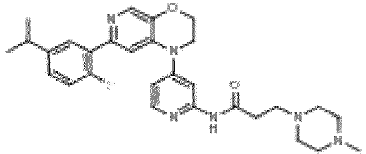 | N-(4-{7-[2-fluoro-5-(propan-2-yl)phenyl]-1H,2H,3H-pyrido[3,4-b][1,4]oxazin-1-yl}pyridin-2-yl)-3-(4-methylpiperazin-1- yl)propanamide |
| 35 | 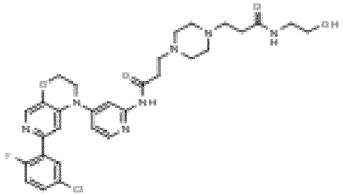 | 3-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-1-yl}-N-(2-hydroxyethyl)propanamide |
| 36 | 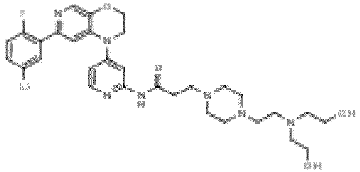 | 3-(4-{2-[bis(2-hydroxyethyl)amino]ethyl}piperazin -1-yl)-N-{4-[7-(5-chloro-2-fluorophenyl)-1H,2H,3H-pyrido[3,4-b][1,4]oxazin-1-yl]pyridin-2-yl}propanamide |
| 37 | 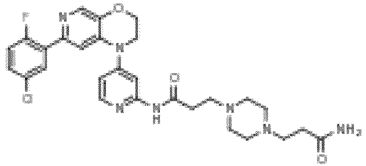 | 3-[4-(2-carbamoylethyl)piperazin-1-yl]-N-{4-[7-(5-chloro-2-fluorophenyl)-1H,2H,3H-pyrido[3,4-b][1,4]oxazin-1-yl]pyridin-2-yl}propanamide |
| 38 | 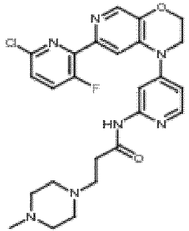 | N-{4-[7-(6-chloro-3-fluoropyridin-2-yl)-1H,2H,3H-pyrido[3,4-b][1,4]oxazin-1-yl]pyridin-2-yl}-3-(4-methylpiperazin-1-yl)propanamide |
| 39 | 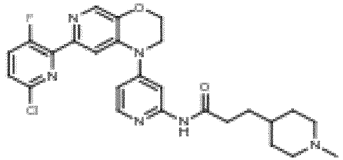 | N-{4-[7-(6-chloro-3-fluoropyridin-2-yl)-1H,2H,3H-pyrido[3,4-b][1,4]oxazin-1-yl]pyridin-2-yl}-3-(1-methylpiperidin-4-yl)propanamide |
| 40 | 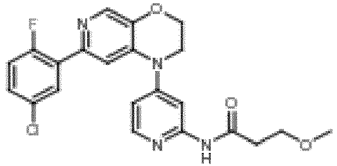 | N-{4-[7-(5-chloro-2-fluorophenyl)-1H,2H,3H-pyrido[3,4-b][1,4]oxazin-1-yl]pyridin-2-yl}-3-methoxypropanamide |
| 41 | 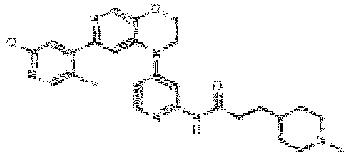 | N-{4-[7-(2-chloro-5-fluoropyridin-4-yl)-1H,2H,3H-pyrido[3,4-b][1,4]oxazin-1-yl]pyridin-2-yl}-3-(1-methylpiperidin-4-yl)propanamide |
| 42 | 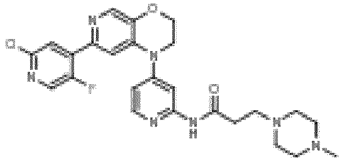 | N-{4-[7-(2-chloro-5-fluoropyridin-4-yl)-1H,2H,3H-pyrido[3,4-b][1,4]oxazin-1-yl]pyridin-2-yl}-3-(4-methylpiperazin-1-yl)propanamide |
| 43 | 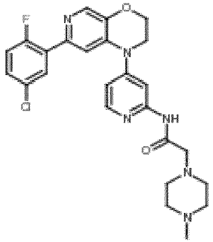 | N-{4-[7-(5-chloro-2-fluorophenyl)-1H,2H,3H-pyrido[3,4-b][1,4]oxazin-1-yl]pyridin-2-yl}-2-(4-methylpiperazin-1-yl)acetamide |
| 44 | 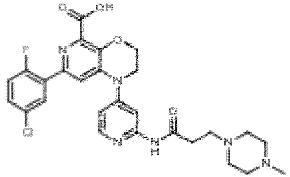 | 7-(5-chloro-2-fluorophenyl)-1-{2-[3-(4-methylpiperazin-1-yl)propanamido]pyridin-4-yl}-1H,2H,3H-pyrido[3,4-b][1,4]oxazine-5-carboxylic acid |
| 45 | 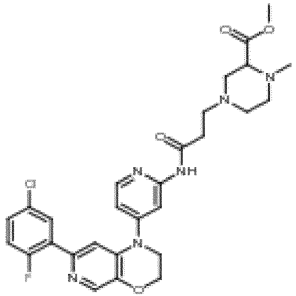 | Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazine-2-carboxylate |
| 46 | 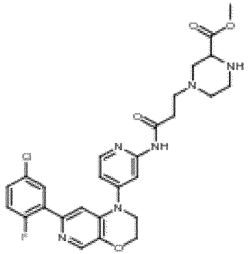 | Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]piperazine-2-carboxylate |
| 47 | 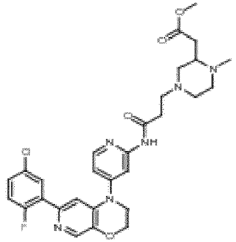 | Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazin-2-yl}acetate |
| 48 | 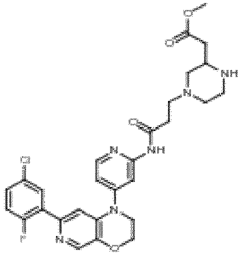 | Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-2-yl}acetate |
| 49 | 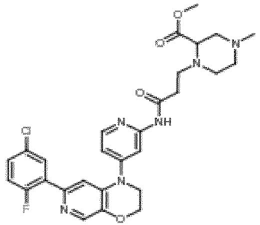 | Methyl 1-[2-({4-[7-(5-chloro-2-fluorophenyl)-1H,2H,3H-pyrido[3,4-b][1,4]oxazin-1-yl]pyridin-2-yl}carbamoyl)ethyl]-4-methylpiperazine-2-carboxylate |
| 50 | 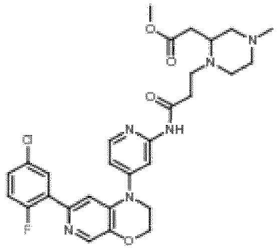 | Methyl 2-{1-[2-({4-[7-(5-chloro-2-fluorophenyl)-1H,2H,3H-pyrido[3,4-b][1,4]oxazin-1-yl]pyridin-2-yl}carbamoyl)ethyl]-4-methylpiperazin-2-yl}acetate |
| 51 | 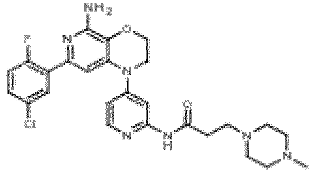 | N-{4-[5-amino-7-(5-chloro-2-fluorophenyl)-1H,2H,3H-pyrido[3,4-b][1,4]oxazin-1-yl]pyridin-2-yl}-3-(4-methylpiperazin-1-yl)propanamide |
| 52 | 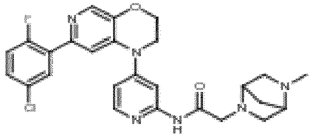 | N-{4-[7-(5-chloro-2-fluorophenyl)-1H,2H,3H-pyrido[3,4-b][1,4]oxazin-1-yl]pyridin-2-yl}-2-{5-methyl-2,5-diazabicyclo[2.2.1]heptan-2-yl} acetamide |
| 53 | 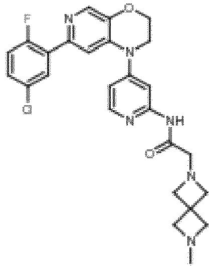 | N-{4-[7-(5-chloro-2-fluorophenyl)-1H,2H,3H-pyrido[3,4-b][1,4]oxazin-1-yl]pyridin-2-yl}-2-{6-methyl-2,6-diazaspiro[3.3]heptan-2-yl}acetamide |
| 54 | 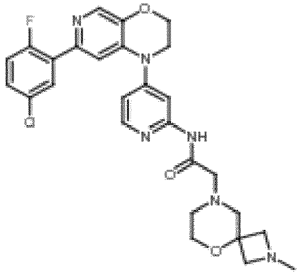 | N-{4-[7-(5-chloro-2-fluorophenyl)-1H,2H,3H-pyrido[3,4-b][1,4]oxazin-1-yl]pyridin-2-yl}-2-{2-methyl-5-oxa-2,8-diazaspiro[3.5]nonan-8-yl}acetamide |
| 55 | 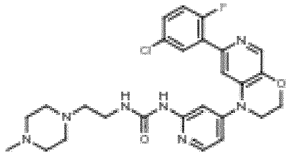 | 1-(4-(7-(5-chloro-2-fluorophenyl)-2,3-dihydro-1H-pyrido[3,4-b][1,4]oxazin-1-yl)pyridin-2-yl)-3- (2-(4-methylpiperazin-1- yl)ethyl)urea |
| 56 | 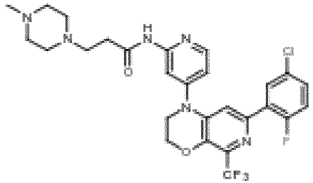 | N-(3-(7-(5-chloro-2-fluorophenyl)-5-(trifluoromethyl)-2,3-dihydro-1H-pyrido[3,4-b][1,4]oxazin-1-yl)phenyl)-3-(4methylpiperazin-1-yl)propanamide |

In another preferred embodiment the present invention refers to a compound of formula (I), wherein **A** is **A2** 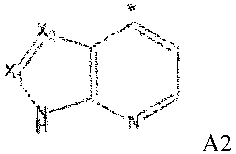 represented by the formula (Ib) 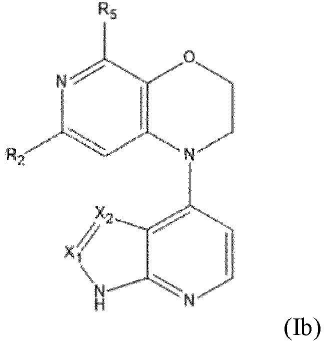
**X₁** and **X₂** are independently N or CH;
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₅** is H;
and pharmaceutically acceptable salts thereof.

According to a preferred embodiment, the invention refers to at least one of the compounds of Formula (Ib) listed in the Table 3 below and pharmaceutically acceptable salts thereof.

**Table 3: List of preferred compounds of Formula (Ib)**

| **Example No.** | **Structure** | **Chemical Name** |
|---|---|---|
| 15 | 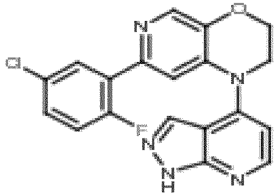 | 7-(5-chloro-2-fluorophenyl)-1-{1H-pyrazolo[3,4-b]pyridin-4-yl}-1H,2H,3H-pyrido[3,4-b][1,4]oxazine |
| 16 | 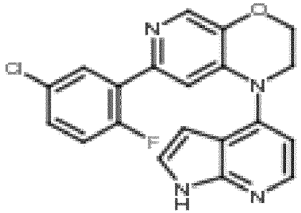 | 7-(5-chloro-2-fluorophenyl)-1-{1H-pyrrolo[2,3-b]pyridin-4-yl}-1H,2H,3H-pyrido[3,4-b][1,4]oxazine |
| 17 | 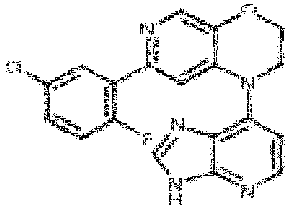 | 7-(5-chloro-2-fluorophenyl)-1-{3H-imidazo[4,5-b]pyridin-7-yl}-1H,2H,3H-pyrido[3,4-b][1,4]oxazine |

In a even more preferred embodiment the present invention refers to a compound of formula (Ib), wherein **X₁** and **X₂** are CH.

The compounds of the invention, including all the compounds here above listed, can be prepared from readily available starting materials using the following methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

Thus, processes described below should not be viewed as limiting the scope of the synthetic methods available for the preparation of the compounds of the invention.

In some cases a step is needed in order to mask or protect sensitive or reactive moieties, generally known protective groups (PG) could be employed, in accordance to general principles of chemistry (Protective group in organic syntheses, 3rd ed. T. W. Greene, P. G. M. Wuts).

The compounds of formula (I) of the present invention have surprisingly been found to effectively inhibit the receptor ALK5. Advantageously, the inhibition of ALK5 may result in efficacious treatment of the diseases or condition wherein the ALK5 receptor is involved.

In this respect, it has now been found that the compounds of formula (I) of the present invention have an antagonist drug potency expressed as half maximal inhibitory concentration (IC₅₀) on ALK5 lower or equal than 10 nM as shown in the present experimental part.

Preferably, the compounds of the present invention have an IC₅₀ on ALK5 between 5 and 10 nM.

Even more preferably, the compounds of the present invention have an IC₅₀ on ALK5 lower than 1 nM.

In one aspect, the present invention refers to a compound of formula (I) or a pharmaceutically acceptable salt thereof, for use as a medicament. Thus, the invention refers to a compound of formula (I) in the preparation of a medicament, preferably for use in the prevention and/or treatment of a disease, disorder or condition associated with ALK5 signaling pathway.

In a preferred embodiment, the invention refers to a compound of formula (I) or a pharmaceutically acceptable salt thereof, for use in the prevention and/or treatment of a disease, disorder or condition associated with ALK5 signaling pathwayIn one embodiment, the present invention refers to a compound of formula (I) useful for the prevention and/or treatment of fibrosis and/or diseases, disorders, or conditions that involve fibrosis.

The terms "fibrosis" or "fibrosing disorder," as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract.

Preferably, the compounds of formula (I) of the present invention, or a pharmaceutical composition comprising a compound of formula (I), are useful for the treatment and/or prevention of fibrosis such as pulmonary fibrosis, idiopathic pulmonary fibrosis (IPF), hepatic fibrosis, renal fibrosis, ocular fibrosis, cardiac fibrosis, arterial fibrosis and systemic sclerosis.

More preferably, the compounds of formula (I) of the present invention, or a pharmaceutical composition comprising a compound of formula (I), are useful for the treatment of idiopathic pulmonary fibrosis (IPF).

As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan.

The compounds of formula (I) may be administered once or according to a dosing regimen wherein a number of doses are administered at varying intervals of time for a given period of time. Typical daily dosages may vary depending upon the route of administration chosen.

The present invention also refers to a pharmaceutical composition comprising a compound of formula (I) in admixture with at least one or more pharmaceutically acceptable carrier or excipient.

In one embodiment, the invention refers to a pharmaceutical composition of compounds of formula (I) in admixture with one or more pharmaceutically acceptable carrier or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion) and by inhalation.

Preferably, the compounds of the present invention are administered orally or by inhalation.

More preferably, the compounds of the present invention are administered by inhalation.

In one preferred embodiment, the pharmaceutical composition comprising the compound of formula (I) is a solid oral dosage form such as tablets, gelcaps, capsules, caplets, granules, lozenges and bulk powders.

In one embodiment, the pharmaceutical composition comprising the compound of formula (I) is a tablet.

The compounds of the invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like.

In a further embodiment, the pharmaceutical composition comprising a compound of formula (I) is a liquid oral dosage forms such as aqueous and nonaqueous solutions, emulsions, suspensions, syrups, and elixirs. Such liquid dosage forms can also contain suitable known inert diluents such as water and suitable known excipients such as preservatives, wetting agents, sweeteners, flavorants, as well as agents for emulsifying and/or suspending the compounds of the invention.

In a further embodiment, the pharmaceutical composition comprising the compound of formula (I) is an inhalable preparation such as inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as cosolvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers.

The compounds of the invention can be administered as the sole active agent or in combination with other pharmaceutical active ingredients.

The dosages of the compounds of the invention depend upon a variety of factors including among others the particular disease to be treated, the severity of the symptoms, the route of administration and the like.

The invention is also directed to a device comprising a pharmaceutical composition comprising a compound of formula (I) according to the invention, in form of a single- or multi-dose dry powder inhaler or a metered dose inhaler.

All preferred groups or embodiments described above for compounds of formula (I) may be combined among each other and apply as well *mutatis mutandis.*

The compounds of the invention, including all the compounds here above listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

Thus, processes described below and reported in the following schemes should not be viewed as limiting the scope of the synthetic methods available for the preparation of the compounds of the invention.

The compounds of formula (I) including all the compounds or at least one of the here above listed can be generally prepared according to the procedure outlined in detail in the Schemes shown below, using generally known methods.

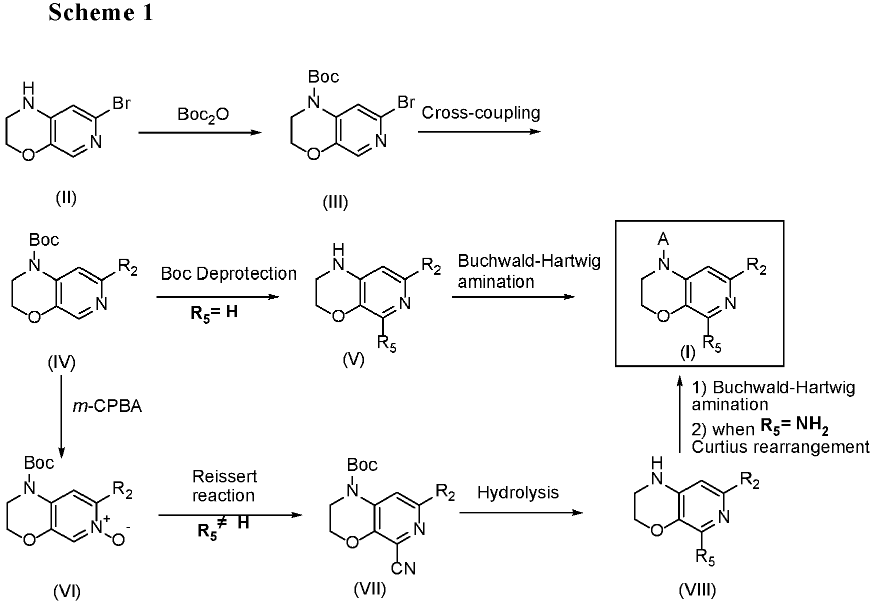

Scheme 1 provides a possible synthetic route for the preparation of a compound of formula (I), wherein A, R₂ and R₅ are as defined above.

A compound of formula (III) may be prepared by protection of the nitrogen atom of compound of formula (II) with suitable protecting groups, such as di-*tert-*butyl dicarbonate (Boc anhydride, Boc₂O) following well known procedures to a person skilled in the art.

A compound of formula (IV) may be prepared from compound (III) by metal-catalyzed cross coupling reactions such as Stille or Suzuki couplings, or similar as described in "Transition Metals for 15 Organic Synthesis", 2nd Ed, 1, 2004, with a suitable reagent. Typical Suzuki reaction conditions comprise reacting compound (III) with appropriate boronic acid or ester, in the presence of a Pd catalyst, such as Pd(dppf)Cl₂ or Pd(Ph₃P)₄, a suitable base, as for example CsF, K₂CO₃ or NaHCO₃, in a mixture of solvents, such as 1,4-dioxane and water, at an appropriate temperature under conventional heating or microwave irradiation. Typical Stille cross coupling conditions comprise reacting compound (III) with a suitable organo-tin reagent, in the presence of a Pd catalyst, such as Pd(Ph₃P)₄, in a suitable solvent, such as toluene or DMF, and at an appropriate temperature under conventional heating or microwave irradiation.

In some cases, for example wherein R₅ is hydrogen, a compound of formula (I) may be obtained from compound (IV) by removal of nitrogen protecting group, for example, under acidic conditions, followed by Buchwald-Hartwig amination in the presence of suitable halide. Typical Buchwald-Hartwig conditions include the use of a base, as for example potassium *tert*-butoxide, proper palladium catalyst, such as Pd₂dba₃, in the presence of a suitable ligand as Xantphos, in appropriate solvent such as 1,4-dioxane or toluene and at an appropriate temperature, for example, 120 °C.

In some other cases, wherein R₅ is different from hydrogen as defined above, a compound of formula (IV) may be converted into the corresponding N-oxide (VI) in the presence of a suitable oxidant, such as for example m-chloroperbenzoic acid, and treated under Reissert conditions to obtain compounds (VII). Reissert conditions comprise the use of potassium cyanate and a methylating agent, such as dimethyl sulfate, in a suitable solvent such as 1,4-dioxane and at an appropriate temperature, for example, 70 °C.

A compound of formula (VIII) may be prepared through hydrolysis of compounds (VII) under basic conditions, such as for example, aqueous NaOH solution in methanol at an appropriate temperature, as for example 80 °C.

Compounds (VIII) were finally converted in compounds of formula (I) by Buchwald-Hartwig amination as described above.

In some selected cases, wherein R₅ is different from hydrogen as defined above and, for example, is equal to NH₂, a compound of formula (I) may be prepared by means of Curtius rearrangement of the Buchwald-Hartwig amination product. Typical conditions include the use of trimethylsilyl azide in the presence of a coupling reagent, such as for example T3P, and of an organic base as triethylamine, diisopropylamine and the like. As well known to a person skilled in the art, the reaction may be carried in polar aprotic solvent such as THF and at a suitable temperature, as for instance 80 °C.

A compound of formula (II) may be prepared as described in Scheme 2, using generally known methods.

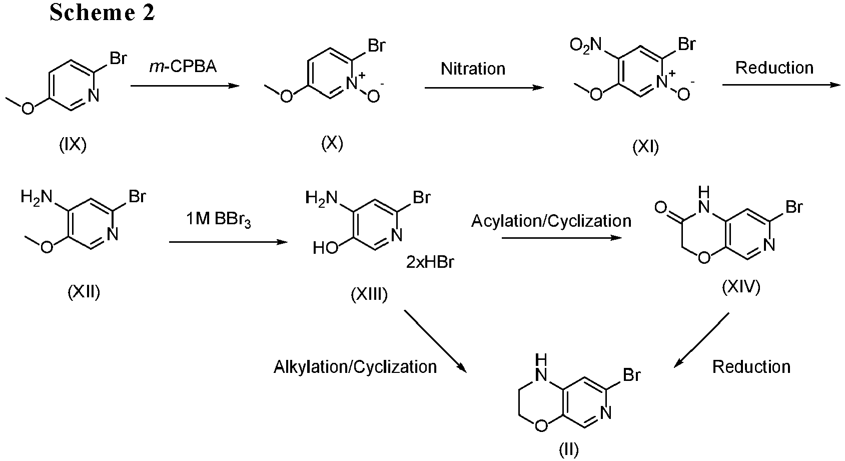

A compound of formula (X) may be prepared by treating commercially available, suitably substituted pyridine (IX) with a proper oxidant, such as for example *m*-chloroperbenzoic acid. The resulting *N*-oxide (X) may undergo nitration in the presence of a mixture of strong acids, as nitric acid and sulfuric acid, at appropriate temperature ranging for example between 0 and 70 °C, following well known procedures to a person skilled in the art.

A compound of formula (XII) may be obtained from compound (XI) by reduction using, for example, iron powder in the presence of acids, such as acetic acid and hydrochloridric acid, at a suitable temperature in a protic polar solvent, such as ethanol.

A compound of formula (XIII) may be prepared by demethylation of compound (XII) according to known procedures taking advantage of reagents such as boron tribromide in apolar solvents, as dichloromethane.

Lastly, a compound of formula (II) may be obtained from compound (XIII) by alkylation followed by intramolecular cyclization. Typical conditions include alkylating agents, such as 1,2-dibromoethane, in the presence of a base as for example potassium carbonate, in polar aprotic solvents, as DMF, and at a suitable temperature, such as 80 °C. Alternatively, a compound of formula (II) may be prepared from compound (XIII) in two steps comprising tandem acylation/cyclization, followed by lactam reduction.

A compound of formula (XIII) may undergo acylation when treated with reagents as for example chloro- or bromacetyl chloride in the presence of a base, in an appropriate solvent, such as ACN, and at a suitable temperature, as for example 80 °C. Under the same conditions, intramolecular cyclization may lead to compound (XIV).

A compound of formula (II) may be prepared from compound of formula (XIV) by reduction using, for example, borane complexes, such as borane tetrahydrofuran complex or borane dimethylsulfide complex in polar aprotic solvent, such as tetrahydrofuran at appropriate temperature. In some cases, the reduction for example may be also carried out in the presence of Vaska complex in combination with tertramethyldisiloxane in a suitable solvent, such as DCM and at room temperature.

Finally, a compound of formula (II) may also be obtained from the commercially available compound (XIV) by reduction of the lactam, as described above.

In some embodiments of the present invention, compounds of formula (Iaa) may be prepared following Route A or Route B as depicted in Scheme 3, wherein A, R₁, R₂ and R₅ are as defined above.

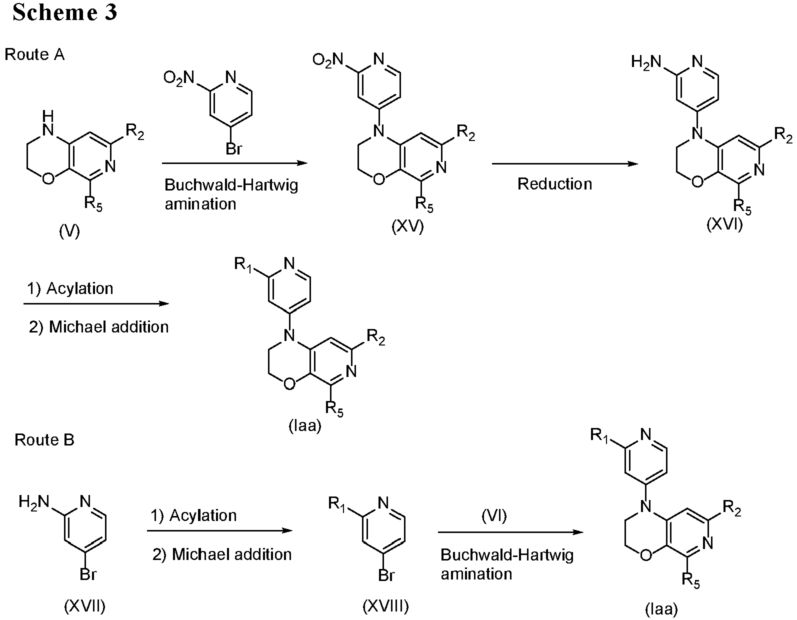

A compound of formula (XV) may be prepared from compound (V) by means of Buchwald-Hartwig amination in the presence of suitable halide, such as for example 4-bromo-2-nitropyridine. Standard amination conditions were described above for Scheme 1.

A compound of formula (XVI) may be prepared from compound (XV) by reduction of the nitro group under conditions well known to a person skilled in the art, as for example, iron powder in the presence of an acid in a protic polar solvent, such as ethanol, at a suitable temperature ranging from room temperature to 85 °C.

A compound of formula (Iaa) can be obtained from compound (XV) *via* two-step preparation. First, compound (XV) can be reacted with a suitable acylating agent, such as for example chloroacetyl chloride or 3-bromoproprionyl chloride, in the presence of a base, such as triethylamine or similar, in a solvent as DCM at an appropriate temperature such as 0 °C or below. Next, addition of suitable reagents under the conditions of Michael addition may lead to compounds of formula (Iaa). Typical conditions include the use of a suitable nucleophile, such as an amine or an alcohol, a base such as for example diisopropylethylamine and the like, in an appropriate polar protic solvent as methanol or ethanol, at room temperature.

According to Route B in Scheme 3, a compound of formula (Iaa) can also be obtained starting from commercial 4-bromo-2-amino pyridina (XVII) and following a two-step synthesis to introduce R1, as defined above. Acylation and Michael addition using the typical conditions described for Route A Scheme 3 may be used to obtain compounds of formula (XVIII).

A compound of formula (Iaa) may be obtained from compound (XVIII) by means of Hartwig-Buchwald amination according to the conditions described above.

The various aspects of the invention described in this application are illustrated by the following examples which are not meant to limit the invention in any way.

### PREPARATIONS OF INTERMEDIATES AND EXAMPLES

Chemical Names of the compounds were generated with MarvinSketch Deuterium. 3.

All reagents, for which the synthesis is not described in the experimental part, are either commercially available, or are known compounds or may be formed from known compounds by known methods by a person skilled in the art.

### ABBREVIATION - MEANING (IN ALPHABETICAL ORDER)

ACN= acetonitrile
AcOH= acetic acid
Aq.= aqueous
BBr₃ = boron tribromide
BINAP= 2,2-bis(diphenylphosphino)-1,1-binaphthalene
Boc₂O= di-tert-butyl dicarbonate
c-Hex= cyclohexane
Cs₂CO₃= cesium carbonate
CsF= cesium fluoride
DCE= dichloroethane
DCM= dichloromethane
DMF= dimethylformamide
DMAP= 4-dimethylaminopyridine
DMSO= dimethylsulfoxide
DIPEA= *N,N*-diisopropylethylamine
EtOAc= ethyl acetate
h= hour
HATU= 1-[bis(dimethylamino)methylene]-1H-1,2,3-triazolo[4,5-b]pyridinium 3-oxide hexaneafluorophosphate
HBr= hydrobromidric acid
HCl= hydrochloridric acid
HPLC= high performance liquid chromatography
I₂= iodine
IPA= isopropyl alcohol
K₂CO₃= potassium carbonate
LDA= lithium diisopropylamide
LCMS= liquid chromatography mass spectrometry
*m*-CPBA= *meta*-chloroperoxybenzoic acid
MeOH= methanol
MOMCl= chloromethyl methy ether
Na₂CO₃= sodium carbonate
NaHCO₃= sodium bicarbonate
NaOH= sodium hydroxide
Na₂SO₃= sodium sulfite
Na₂SO₄= sodium sulfate
NH₃= ammonia
NH₄Cl= ammonium chloride
NH₄OH= ammonium hydroxide
NMR= nuclear magnetic resonance
Pd/C= palladium on carbon
Pd dba₂= bis(dibenzylideneacetone)palladium(0)
PdCl₂dppf= [1,1'-bis(diphenylphosphino)ferrocene]palladium(II) dichloride
Pd₂dba₃= tris(dibenzylideneacetone)dipalladium(0)
(Ph₃P)₄Pd= tetrakis(triphenylphosphine)palladium(0)
pTLC= preparative thin layer chromatography
RM= reaction mixture
r.t.= retention time
RT= room temperature
Sat.= saturated
SCX= Strong Cation Exchange
SEM-Cl= (2-chloromethoxyethyl)trimethylsilane
T3P= propylphosphonic anhydride
TEA= triethylamine
TFA= trifluoroacetic acid
THF= tetrahydrofuran
TMSN₃= trimethylsilyl azide
UPLC-MS= ultra-performance liquid chromatography mass spectrometry
Xantphos= 4,5-bis(diphenylphosphino)-9,9-dimethylxanthene
XantphosPdG3= [(4,5-Bis(diphenylphosphino)-9,9-dimethylxanthene)-2-(2-amino-1,1-biphenyl)] palladium(II) methanesulfonate
Y= yield

### General Experimental Details and methods

### Analytical method

### Instruments, materials and methods employed for analyses:

¹H-NMR spectra were recorded on Bruker Avance III HD 400 MHz or on Bruker Fourier 300 MHz, or on a Varian MR-400 spectrometer. Chemical shifts are reported as δ values in ppm relative to signals from residual non-deuterated solvents used for sample preparation as an internal standard. Coupling constants (J values) are given in hertz (Hz) and multiplicities are reported using the following abbreviation (br= broad signal, s= singlet, d= doublet, dd= doublet of doublets, ddd= doublet of doublet of doublets, t= triplet, dt= doublet of triplets, ddt= doublet of doublet of triplets, td= triplet of doublets, q= quartet, qd= quartet of doublets, m= multiplet).

Preparative HPLC purification was performed on a Shimadzu FCV-200AL, coupled with the LC-20AP preparative pump.

Microwave reactions were carried out using an Anton Parr Monowave 450 System.

### LC/UV/MS Analytical Methods

Materials: Formic acid ≥ 98% (Sigma-Aldrich), acetonitrile for HPLC UV/gradient grade (Baker), µQ-water for LCMS

UPLC-MS measurements were performed on Waters ACQUITY UPLC I-Class PLUS System with Waters SQ Detector 2 (ESI-MS, capillary voltage: 3000 V, cone voltage: 40 V, de-solvation gas: 1000 L/h, de-solvation temp.: 500 °C), equipped with Acquity UPLC BEH C18 1.7 µm (2.1 × 100 mm), column no. 186002352, using 20 - 100% ACN in water gradient with 0.1% formic acid (flow: 0.5 mL/min).

### LC-MS methods:

LC-MS measurements were performed on Dionex UHPLC Ultimate 3000 with DAD detector/Thermo Scientific MSQ Plus system (Methods A - H) or with DAD detector/Thermo Scientific ISQ EC - MS (Methods I - M), equipped with Kinetex^{®} 2.6 µm XB-C18 (4.6x50mm), 110A, column no. 00B-4496-E0 (Methods A - G and K - M), column no. 00B-4496-E0 (Methods I and J) or ACQUITY UPLC BEH C8 1.7 µm (2.1x150mm), 130 A, column no. 186003377 (Method H). Detection range: 190 - 350 nm ± 4 nm. Flow 1.0 mL/min (Methods A - G and I - M), 0.5 mL/min (Method H). Column temperature: 25 °C (Methods A - G and I - M), 55 °C (Method H). Autosampler temperature: 20 °C.

*Method A.* Analysis time: 6 min. Mobile phase A: 0.1% v/v MQ water solution of formic acid. Mobile phase B: 0.1% v/v acetonitrile solution of formic acid. Elution gradient: 0.00 min (90% A, 10% B), 3.35 - 3.75 min (40% A, 60% B), 3.90 - 4.75 min (5% A, 95% B), 5.00 - 6.00 min (90% A, 10% B).

*Method B.* Analysis time: 6 min. Mobile phase A: 0.1% v/v MQ water solution of formic acid. Mobile phase B: 0.1% v/v acetonitrile solution of formic acid. Elution gradient: 0.00 min (90% A, 10% B), 3.35 - 3.75 min (30% A, 70% B), 3.90 - 4.75 min (5% A, 95% B), 5.00 - 6.00 min (90% A, 10% B).

*Method C.* Analysis time: 7 min. Mobile phase A: 0.1% v/v MQ water solution of formic acid. Mobile phase B: 0.1% v/v acetonitrile solution of formic acid. Elution gradient: 0.00 min (80% A, 20% B), 3.35 - 3.75 min (20% A, 80% B), 3.90 - 4.75 min (5% A, 95% B), 5.00 - 6.00 min (80% A, 20% B).

*Method D.* Analysis time: 7 min. Mobile phase A: 0.1% v/v MQ water solution of formic acid. Mobile phase B: 0.1% v/v acetonitrile solution of formic acid. Elution gradient: 0.00 - 1.00 min (95% A, 5% B), 4.75 - 5.25 min (40% A, 60% B), 6.00 - 7.00 min (95% A, 5% B).

*Method E.* Analysis time: 7 min. Mobile phase A: 0.1% v/v MQ water solution of formic acid. Mobile phase B: 0.1% v/v acetonitrile solution of formic acid. Elution gradient: 0.00 - 1.00 min (95% A, 5% B), 4.75 - 5.25 min (20% A, 80% B), 6.00 - 7.00 min (95% A, 5% B).

*Method F.* Analysis time: 6 min. Mobile phase A: 0.1% v/v MQ water solution of formic acid. Mobile phase B: 0.1% v/v acetonitrile solution of formic acid. Elution gradient: 0.00 min (80% A, 20% B), 3.35 - 3.75 min (60% A, 40% B), 3.90 - 4.75 min (5% A, 95% B), 5.00 - 6.00 min (80% A, 20% B).

*Method G.* Analysis time: 6 min. Mobile phase A: 0.1% v/v MQ water solution of formic acid. Mobile phase B: 0.1% v/v acetonitrile solution of formic acid. Elution gradient: 0.00 min (95% A, 5% B), 3.35 - 3.75 min (60% A, 40% B), 3.90 - 4.75 min (5% A, 95% B), 5.00 - 6.00 min (95% A, 5% B).

*Method H.* Analysis time: 10 min. Mobile phase A: 0.1% v/v MQ water solution of formic acid. Mobile phase B: 0.1% v/v acetonitrile solution of formic acid. Elution gradient: 0.00 - 0.50 min (99% A, 1% B), 3.00 min (70% A, 30% B), 6.5 min (50% A, 50% B), 7.50 - 8.00 min (20% A, 80% B), 8.1 - 10 min (99% A, 1%B).

*Method I.* Analysis time: 6 min. Mobile phase A: 0.1% v/v MQ water solution of formic acid. Mobile phase B: 0.1% v/v acetonitrile solution of formic acid. Elution gradient: 0.00 min (80% A, 20% B), 3.35 - 3.75 min (20% A, 80% B), 3.90 - 4.75 min (5% A, 95% B), 5.00 - 6.00 min (80% A, 20% B).

*Method J.* Analysis time: 7 min. Mobile phase A: 0.1% v/v MQ water solution of formic acid. Mobile phase B: 0.1% v/v acetonitrile solution of formic acid. Elution gradient: 0.00 - 1.00 min (95% A, 5% B), 4.75 - 5.25 min(20% A, 80% B), 6.00 - 7.00 min (95% A, 5% B).

*Method K.* Analysis time: 6 min. Mobile phase A: 0.1% v/v MQ water solution of formic acid. Mobile phase B: 0.1% v/v acetonitrile solution of formic acid. Elution gradient: 0.00 min (90% A, 10% B), 3.35 - 3.75 min (40% A, 60% B), 3.90 - 4.75 min (5% A, 95% B), 5.00 - 6.00 min (90% A, 10% B).

*Method L.* Analysis time: 6 min. Mobile phase A: 0.1% v/v MQ water solution of formic acid. Mobile phase B: 0.1% v/v acetonitrile solution of formic acid. Elution gradient: 0.00 min (90% A, 10% B), 3.35 - 3.75 min (30% A, 70% B), 3.90 - 4.75 min (5% A, 95% B), 5.00 - 6.00 min (90% A, 10% B).

*MethodM.* Analysis time: 7 min. Mobile phase A: 0.1% v/v MQ water solution of formic acid. Mobile phase B: 0.1% v/v acetonitrile solution of formic acid. Elution gradient: 0.00 - 1. 00 min (95% A, 5% B), 4.75 - 5.25 min (20% A, 80% B), 6.00 - 7.00 min (95% A, 5% B).

LCMS may be recorded under the following conditions: diode array DAD chromatographic traces, mass chromatograms and mass spectra may be taken on UPLC/PDA/MS AcquityTM system coupled with Micromass ZQTM or Waters SQD single quadrupole mass spectrometer operated in positive and/or negative electron spray ES ionization mode and/or Fractionlynx system used in analytical mode coupled with ZQTM single quadrupole operated in positive and/or negative ES ionisation mode. Quality Control methods used operated under low pH conditions or under high pH conditions:
*Method 1.* Low pH conditions column: Acquity CSH C18 2.1x50mm 1.7um, the column temperature was 40°C; mobile phase solvent A was milliQ water+0.1% formic acid, mobile phase solvent B ACN+0.1% formic acid. The flow rate was 1 mL/min.

The gradient table was t=0 min 97% A 3% B, t=1.5 min 0.1% A 99.9% B, t=1.9 min 0.1% A 99.9% B and t=2 min 97% A 3% B. The UV detection range was 210-350 nm and ES+/ES- range was 100 to 1500 AMU.

*Method 2.* High pH conditions: column: Acquity Kinetex 1.7 um EVO C18 100A, 2.1x50mm, the column temperature was 40°C; mobile phase solvent A was 10 mM aqueous solution of NH₄HCO₃ adjusted to pH=10 with ammonia, mobile phase solvent B ACN. The flow rate was 1 mL/min. The gradient table was t=0 min 97% A 3% B, t=1.5 min 0.1% A 99.9% B, t=1.9 min 0.1% A 99.9% B and t=2 min 97% A 3% B. The UV detection range was 210-350 nm and ES+/ES- range was 100 to 1500 AMU.

### PREPARATIONS OF INTERMEDIATES

### Intermediate 1: 7-bromo-2,3-dihydro- 1H-Pyrido[3,4-b][1,4]oxazine

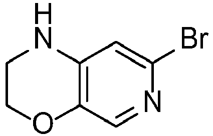

69.0 g (0.52 mol, 1.8 eq.) of m-CPBA was dissolved in 1 L DCE. 50.0 g (0.27 mol, 1.0 eq.) of commercial 2-bromo-5-methoxypyridine was added to the RM dropwise under argon. RM was heated to 60 °C for 16 h. Additional portion (4.6 g) of *m*-CPBA was added and the mixture was stirred in the same temperature for 4h. After that time, RM was cooled to RT and half of it was partitioned between 600 mL DCM and 600 mL of saturated aqueous solution of Na₂SO₃. The organic layer was separated and the aqueous extracted 2×500 mL DCM. The procedure was repeated with the second part of the RM. 1000 mL of sat. aq. NaHCO₃ was added to the combined organic layers and the mixture was stirred for one night in RT. The organic layer was separated and the aqueous layer was extracted with DCM (5×500 mL). Organic layers were combined, dried over Na₂SO₄, filtered and volatiles were removed under reduced pressure to give the corresponding N-oxide (55.0 g, Y= 97%), which was used in the next step without purification.

55.0 g of N-oxide was dissolved in concentrated sulfuric acid (115 mL). 53 mL of 65% nitric acid was added slowly to the solution at 0 °C and the RM was heated to 40 °C and stirred for 15 min. After that time, when clear solution was obtained, the mixture was heated to 75 °C and stirred for 2.75 h. The RM was cooled to RT and poured onto ice. The cold mixture was basified to pH=11 using solid NaOH. The precipitated solid was filtered off and washed with methanol. Obtained nitro-derivative (44.0 g, Y= 66%) which was used in the next step without purification. 40.0 g of nitro-derivative was suspended in 2 L of ethanol (96%) with 84 mL of AcOH and 40.0 g (4.5 eq.) of iron powder was added to the suspension. The RM was heated to 80 °C and 28 mL of 6 N HCl was added. After 0.75 h, the RM was cooled to RT and filtered through Celite^{®}, which was washed with methanol and DCM sequentially. The combined filtrates were concentrated under reduced pressure, diluted with DCM:IPA (4:1 mixture) and washed with aq. 6 M NaOH. The aqueous layer was back-extracted with the above mixture 3 times. The combined organic layers were dried over Na₂SO₄ and dried under reduced pressure to give the amino-derivative (32.0 g, Y= 98%). 32.0 g of amino-derivative was suspended in dry DCM (220 mL) and cooled to -30 °C. 110 mL of BBr₃ (1 M solution in DCM) was added dropwise under Ar atmosphere with vigorous stirring. RM was heated to RT and stirred for 18 h. The precipitated solid was filtered off, washed with DCM and dried on air for 20 h to give the hydroxy-derivative as HBr salt (56.0 g, Y= quant.). 40.0 g of HBr salt of the hydroxy-derivative and 500 g of Cs₂CO₃ (5.0 eq.) were suspended in 1.0 L of dry DMF and heated to 70 °C with mechanical stirring. Dibromoethane (3.0 eq.) was added dropwise and the RM was stirred at the same temperature for 18 h. 300 g of K₂CO₃ was added and the mixture was stirred for additional 24 h at 70 °C. After that time, 50.0 g of NaOH was added and the RM was stirred for 6 h at 70 °C. The suspension was diluted with DCM, filtered through Celite^{®} and concentrated under reduced pressure. The slurry residue was partitioned between DCM and water. The organic phase was separated and the aqueous extracted with DCM (3×500 mL). The combined organic layers were dried over Na₂SO₄, filtered and dried under reduced pressure to give the crude Intermediate 1 (20.0 g, crude) as a brown solid. The title compound was used as such in next steps. UPLC-MS: 100% (280 nm); r.t.= 1.14 min; m/z(+)= 214.9.

### Intermediate 2: 7-(5-chloro-2-fluorophenyl)-1H,2H,3H-pyrido[3,4-b][1,4]oxazine

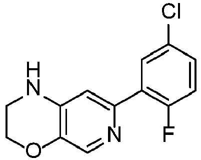

500 mg (1.0 eq.) of Intermediate 1 and 809 mg (2.0 eq.) of 5-chloro-2-fluoro-phenylboronic acid were suspended in 10 mL of dioxane and 4 mL of water. RM was degassed for at least 10 minutes with argon in ultrasonic bath. PdCl₂dppf (0.2 eq.) and CsF (3.0 eq.) were added. RM was heated to 150 °C under microwave irradiation for 15 min. The mixture was diluted with DCM and filtered through Celite^{®}. The solvents were evaporated under reduced pressure and the residue was dissolved in DCM and washed with water. Purification by silica gel column chromatography eluting with gradient from 0 to 80% of EtOAc in hexane +2%TEA yielded Intermediate 2 (440 mg, Y= 72%). UPLC-MS: 100% (280 nm); r.t.= 1.32 min; m/z(+)= 265.5.

### Intermediate 3: 4-[7-(5-chloro-2-fluorophenyl)-1H,2H,3H-pyrido[3,4-b][1,4]oxazin-1-yl]-2-nitropyridine

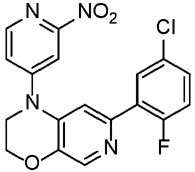

Intermediate 2 (160 mg, 1.0 eq.) and 4-bromo-2-nitropyridine (1.15 eq.) were suspended in dry toluene (16 mL). RM was degassed by bubbling with argon in ultrasonic bath for at least 10 minutes. Xantphos (0.2 eq.), Pd₂dba₃ (0.1 eq.) and sodium tert-butoxide (2.0 eq.) were added. Reaction was heated to 100 °C under microwave irradiation for 45 min. Subsequently, RM was degassed by bubbling with argon in ultrasonic bath for at least 10 minutes and additional 4-bromo-2-nitropyridine (1.15 eq.), xantphos (0.2 eq.) and Pd₂dba₃ (0.1 eq.) were added. RM was heated to 100 °C under microwave irradiation for 30 min. RM was diluted with DCM and filtered through Celite^{®}. The filtrate was dried. Purification by silica gel column chromatography eluting with gradient from 0 to 100% of EtOAc in hexane + 2% TEA yielded Intermediate 3 (200 mg, Y = 85%). UPLC-MS: 100% (280 nm); r.t.= 1.64 min; m/z(+)= 387.4.

### Intermediate 4: N-{4-[7-(5-chloro-2-fluorophenyl)-1H,2H,3H-pyrido[3,4-b] [1,4]oxazin-1-yl]pyridin-2-yl}prop-2-enamide

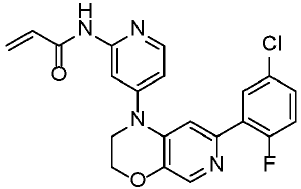

Example 3 (80 mg, 1.0 eq.) was dissolved in dry DCM (4.0 mL) and TEA (5.0 eq.) was added. RM was cooled to -45 °C and 3-bromopropionyl chloride (1.0 eq.) was added. RM was stirred for 20 min in the same temperature. Subsequently, 3-bromopropionyl chloride (0.5 eq.) was added and RM was stirred for 40 min. The mixture was diluted with DCM (5 mL) and washed with water (5 mL). The aqueous layer was extracted with DCM (3×7 mL). Combined organics dried over Na₂SO₄, filtered and evaporated at RT to give the crude Intermediate 4 (125 mg) which was used as such in the next step. UPLC-MS: 85% (280 nm); r.t.= 1.48 min; m/z(+)= 411.4.

### Intermediate 5: 2-chloro-6-(5-chloro-2-fluorophenyl)pyridin-3-ol

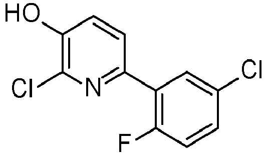

2-Chloro-6-iodopyridin-3-ol (2.0 g, 1.0 eq.) and 5-chloro-2-fluorophenylboronic acid (1.0 eq.) were dissolved in 96% EtOH (4.0 mL) and water (2.0 mL). To the mixture toluene (4.0 mL) was added. RM was degassed by bubbling with argon in ultrasonic bath for at least 10 minutes before the addition of Pd(PPh₃)₄ (0.05 eq.) and NaHCO₃ (1.5 eq.). RM was stirred at 100 °C for 1 h. The suspension was cooled to RT, diluted with DCM and filtered through Celite^{®}. The filtrate was dried under reduced pressure and the residue was partitioned between water (50 mL) and DCM (150 mL). Organics were washed with water (2×50 mL). Water layers were combined and 50 mL of brine was added. The aqueous layer was extracted with DCM (3×100 mL). Combined organics were dried over Na₂SO₄, filtered and volatiles were removed under reduced pressure to give the crude Intermediate 5 (2.4 g), which was used as such in the next step. UPLC-MS: 91% (280 nm); r.t.= 1.72 min; m/z(+)= 258.4.

### Intermediate 6: 2-chloro-6-(5-chloro-2-fluorophenyl)-4-iodopyridin-3-ol

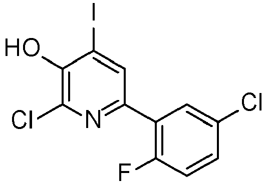

Intermediate 5 (2.3 g, 1.0 eq.) and Na₂CO₃ (2.5 eq.) were suspended in water (35 mL) and RM was stirred for 40 min at RT. I₂ (1.3 eq.) was added and the suspension was stirred at RT for 19 h. After that time, RM was partitioned between 100 mL of sat. aq. sodium bisulfite and 150 mL of DCM. Water layer was extracted with 2×150 mL of DCM. The aqueous phase was filtered through Celite^{®}, which was washed with a mixture DCM:MeOH:TEA (1:1:0.05 v/v/v). The filtrate was dried to give Intermediate 6 (2.0 g, Y= 60%). UPLC-MS: 100% (280 nm); r.t.= 1.84 min; m/z(+)= 384.3.

### Intermediate 7: 2-chloro-6-(5-chloro-2-fluorophenyl)-4-iodo-3-(methoxymethoxy)pyridine

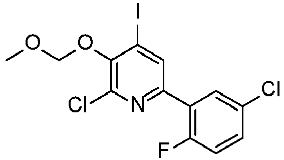

Intermediate 6 (1.9 g, 1.0 eq.) was dissolved in dry DCM (57 mL). RM was cooled to 0 °C and MOMCl (1.1 eq.) was added dropwise. RM was stirred for 5 min and DIPEA (1.3 eq.) was added. After stirring for 20 min, the mixture was partitioned between 70 mL water and 70 mL DCM. The aqueous layer was extracted with DCM (2×70 mL). Combined organics were dried over Na₂SO₄, filtered and evaporated under reduced pressure to give Intermediate 6 (1.58 g, Y = 75%). UPLC-MS: 100% (280 nm); r.t.= 1.96 min; not ionizing.

### Intermediate 8: 2-chloro-6-(5-chloro-2-fluorophenyl)-3-(methoxymethoxy)-N-(pyridin-4-yl)pyridin-4-amine

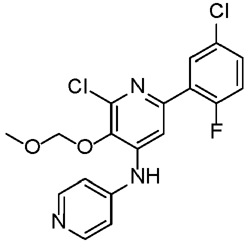

The title compound was prepared following procedure used for Intermediate 3, starting from Intermediate 7 (150 mg, 1.0 eq.) and 4-aminopyridine (1.0 eq.). The suspension was diluted with EtOAc and MeOH and filtered through Celite^{®}. Volatiles were removed under reduced pressure, and the residue was taken up with EtOAc (50 mL) and washed with water (50 mL). Water layer was extracted EtOAc (3×50 mL). Combined organics were dried over Na₂SO₄, filtered and evaporated. Purification by silica gel column chromatography eluting with gradient from 20 to 100% of EtOAc in hexane + 2%TEA yielded Intermediate 8 (90 mg, Y = 60%). UPLC-MS: 100% (280 nm); r.t.= 1.49 min; m/z(+)= 394.2.

### Intermediate 9: 2-chloro-6-(5-chloro-2-fluorophenyl)-4-[(pyridin-4-yl)amino]pyridin-3-ol

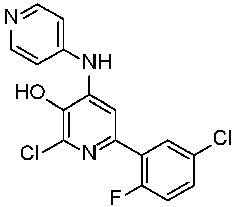

Intermediate 8 (624 mg, 1.0 eq.) was suspended in DCM (12.0 mL) and TFA (3.1 mL) was added. After stirring for 20 minutes, the RM was dried under reduced pressure to yield the crude Intermediate 9 (994 mg, Y = 100%) (calculated for 3×TFA salt). UPLC-MS: 100% (280 nm); r.t.= 1.43 min; m/z(+)= 350.1.

### Intermediate 10: 4-[5-chloro-7-(5-chloro-2-fluorophenyl)-1H,2H,3H-pyrido[3,4-b][1,4]oxazin-1-yl]pyridine

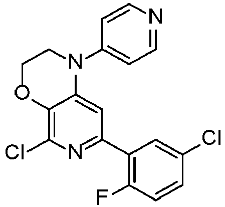

Intermediate 9 (916 mg, 1.0 eq.) and Cs₂CO₃ (5.0 eq.) were suspended in dry DMF (27.5 mL). The suspension was heated to 60 °C. Subsequently, 1,2-dibromoethane (3.0 eq.) was added dropwise and the RM was stirred for 30 min at the same temperature. After that time, RM was evaporated to dryness. The residue was dissolved in DCM (100 mL) and washed with water (2×50 mL). Water layers were combined and extracted with DCM (3×50 mL). Combined organics were dried over Na₂SO₄ and evaporated to dryness to give the crude Intermediate 10 (620 mg, Y = quant). UPLC-MS: 100% (280 nm); r.t.= 1.56 min; m/z(+)= 376.2.

### Intermediate 11: 7-(5-chloro-2-fluorophenyl)-N-[(2,4-dimethoxyphenyl)methyl]-1-(pyridin-4-yl)-1H,2H,3H-pyrido[3,4-b][1,4]oxazin-5-amine

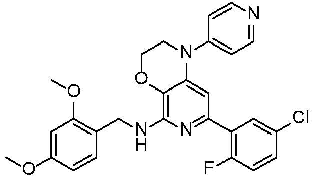

Intermediate 10 (170 mg, 1.0 eq.) and of 2,4-dimetoxybenzylamine (68 µL) were dissolved in dry toluene (17 mL). RM was degassed by bubbling with argon in ultrasonic bath for at least 10 minutes. BINAP (0.2 eq.), Pddba₂ (0.1 eq.) and sodium *tert*-butoxide (2.0 eq.) were added. Reaction was heated to 115 °C under microwave irradiation for 3.5 h. RM was diluted with DCM:MeOH (9:1) and filtered through Celite^{®}. Organic layer was evaporated under reduced pressure. The residue was purified by pH-dependent extraction (6M HCl, DCM, 6M NaOH) yielding a mixture of Intermediate 11 and Example 2 (300 mg). UPLC-MS: 65% (280 nm); r.t.= 1.88 min; not ionizing.

### Intermediate 12: 7-(2,5-difluorophenyl)-1H,2H,3H-pyrido[3,4-b][1,4]oxazine

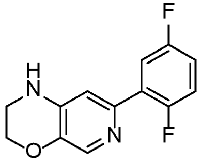

The title compound was prepared following the procedure used for the synthesis of Intermediate 2, starting from Intermediate 1 (300 mg, 1.0 eq.) and using 2,5-difluorophenylboronic acid (2.0 eq.). Purification by silica gel column chromatography using gradient from 0 to 100% of EtOAc in hexane +2.5%TEA yielded Intermediate 12 (210 mg, Y = 61%). UPLC-MS: 100% (280 nm); r.t.= 1.50 min; m/z(+)= 249.3.

### Intermediate 13: 4-[7-(2,5-difluorophenyl)-1H,2H,3H-pyrido[3,4-b][1,4]oxazin-1-yl]-2-nitropyridine

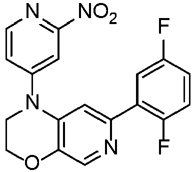

The title compound was prepared following the procedure used for the synthesis of Intermediate 3, starting from Intermediate 12 (90 mg, 1.0 eq.) and using 4-bromo-2-nitropyridine (1.15 eq.). Purification by silica gel column chromatography using gradient from 0 to 100% of EtOAc in hexane +2.5%TEA yielded Intermediate 13 (100 mg, Y = 75%). UPLC-MS: 100% (280 nm); r.t.= 1.62 min; m/z(+)= 371.3.

### Intermediate 14: 7-(3-chlorophenyl)-1H,2H,3H-pyrido[3,4-b][1,4]oxazine

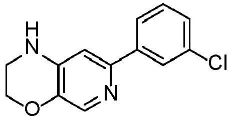

The title compound was prepared following the procedure used for the synthesis of Intermediate 2, starting from Intermediate 1 (300 mg, 1.0 eq.) and using 3-chlorophenylboronic acid (2.0 eq.). Purification by silica gel column chromatography using gradient from 0 to 100% of EtOAc in hexane +2.5%TEA yielded Intermediate 14 (200 mg, Y = 58%). UPLC-MS: 100% (280 nm); r.t.= 1.40 min; m/z(+)= 247.2.

### Intermediate 15: 4-[7-(3-chlorophenyl)-1H,2H,3H-pyrido[3,4-b][1,4]oxazin-1-yl]-2-nitropyridine

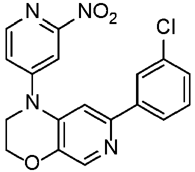

The title compound was prepared following the procedure used for the synthesis of Intermediate 3, starting from Intermediate 14 (120 mg, 1.0 eq.) and using 4-bromo-2-nitropyridine (1.15 eq.). Purification by silica gel column chromatography using gradient from 0 to 100% of EtOAc+2.5%TEA in hexane yielded Intermediate 15 (140 mg, Y = 85%). UPLC-MS: 100% (280 nm); r.t.= 1.63 min; m/z(+)= 369.6.

### Intermediate 16: tert-butyl 7-bromo-1H,2H,3H-pyrido[3,4-b][1,4]oxazine-1-carboxylate

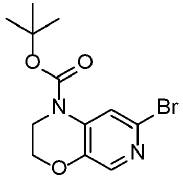

2.0 g of Intermediate 1 was dissolved in dry DCM. 5.5 mL (2.5 eq.) of TEA and 100 mg (0.1 eq.) of DMAP were added to the solution. Subsequently, 4.1 g (2.0 eq.) of Boc₂O was added to the mixture and it was stirred at RT for 72 h. The RM was diluted with DCM and washed with water and 0.5M aq. solution of HCl. The two phases were separated and the organic layer was dried over Na₂SO₄, filtered and dried. Purification by silica gel column chromatography using gradient from 0 to 100% of EtOAc in hexane yielded Intermediate 16 (1.9 g, Y = 65%). UPLC-MS: 100% (280 nm); r.t.= 1.74 min; m/z(+)= 316.9.

### Intermediate 17: 6-chloro-3-fluoro-2-(trimethylstannyl)pyridine

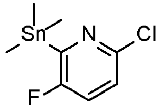

200 mg of 6-chloro-3-fluoro-2-iodopyridine (1.0 eq.) was dissolved in 8 mL of toluene, followed by the addition of 0.2 mL (1.2 eq.) of hexaneamethylditin and 4 mg (0.01 eq.) of Pd(PPh₃)₄. The mixture was stirred at 110 °C for 3 h. RM was cooled to RT, diluted with hexane and filtered through Celite^{®}. The filtrate was dried under reduced pressure to give the crude Intermediate 17 (280 mg), which was used as such in the next step. UPLC-MS: 100% (280 nm); r.t.= 1.86 min; m/z(+)= 295.9.

### Intermediate 18: tert-butyl 7-(6-chloro-3-fluoropyridin-2-yl)-1H,2H,3H-pyrido[3,4-b][1,4]oxazine-1-carboxylate

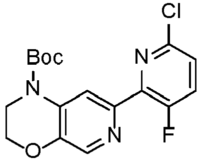

200 mg of Intermediate 16 (1.0 eq.) and 225 mg of Intermediate 17 were dissolved in 10 mL of dry DMF. The solution was degassed by bubbling with argon in ultrasonic bath for at least 10 minutes, followed by the addition of 24 mg (0.2 eq.) of CuI and 22 mg (0.05 eq.) of Pd[(PPh)₃]₂Cl₂. The RM was stirred at 100 °C for 1 h under microwave irradiation. The mixture was diluted with EtOAc, filtered through Celite^{®} and dried under reduced pressure. The residue was dissolved in EtOAc and washed with aq. NH₄ solution. The two phases were separated and the organic layer was dried over Na₂SO₄, filtered and evaporated. Purification by silica gel column chromatography eluting with hexane:EtOAc (4:1) yielded Intermediate 18 (45 mg, Y = 19%). UPLC-MS: 90% (280 nm); r.t.= 1.70 min; m/z(+)= 366.6.

### Intermediate 19: 6-chloro-3-fluoro-2-{1H,2H,3H-pyrido[3,4-b] [1,4]oxazin-7-yl}pyridine

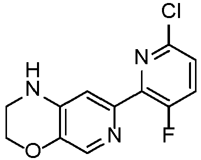

To a solution of 45 mg of Intermediate 18 in 2 mL of MeOH, 1 mL of 4M HCl in dioxane was added. The RM was stirred at 50 °C for 1 h. Then, volatiles were removed under reduced pressure, and the residue was dissolved in 5 mL water. Neutralization with sat. aq. NaHCO₃ and extraction with DCM afforded Intermediate 19 (31 mg, Y = quant). UPLC-MS: 94% (280 nm); r.t.= 1.27 min; m/z(+)= 266.5.

### Intermediate 20: 6-chloro-3-fluoro-2-[1-(2-nitropyridin-4-yl)-1H,2H,3H-pyrido[3,4-b][1,4]oxazin-7-yl]pyridine

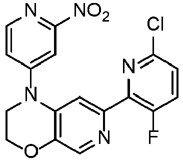

The title compound was prepared following the procedure used for the synthesis of Intermediate 3, starting from Intermediate 19 (12 mg, 1.0 eq.). RM was heated at 150 °C for 20 minutes under microwave irradiation. RM was diluted with EtOAc and washed with water. The organic phase was dried over Na₂SO₄, filtered and evaporated. Purification by pTLC (SiO₂, EtOAc:Hexane (4:1)) yielded Intermediate 20 (6 mg, Y = 33%). UPLC-MS: 100% (280 nm); r.t.= 1.43 min; m/z(+)= 388.6.

### Intermediate 21: 7-(2-fluoro-5-methylphenyl)-1H,2H,3H-pyrido[3,4-b] [1,4]oxazine

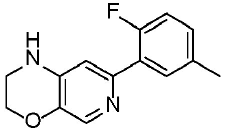

The title compound was prepared following the procedure used for the synthesis of Intermediate 2, starting from Intermediate 1 (50 mg, 1.0 eq.) and using 39 mg (1.3 eq.) of 5-methyl-2-fluorophenyl boronic acid. RM was heated to 150 °C under microwave irradiation for 15 min. RM was diluted with DCM and filtered through Celite^{®}. The filtrate was extracted with 5×10 mL of 6 M HCl. Acid layers were combined, filtered through Celite^{®}, basified with 6M NaOH and extracted with 5×20 mL of DCM. Combined organics were dried over Na₂SO₄ and evaporated to give Intermediate 21 (46 mg, Y = 82%). UPLC-MS: 100% (280 nm); r.t.= 1.30 min; m/z(+)= 245.6.

### Intermediate 22: 4-[7-(2-fluoro-5-methylphenyl)-1H,2H,3H-pyrido[3,4-b][1,4]oxazin-1-yl]-2-nitropyridine

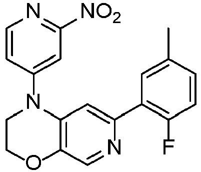

The title compound was prepared following the procedure used for the synthesis of Intermediate 3, starting from Intermediate 21 (54 mg, 1.0 eq.). Reaction was carried out under microwave irradiation for 45 min at 100 °C. Next, the mixture was degassed again and 52 mg (1.1 eq.) of 4-bromo-2-nitropyridine, 26 mg (0.2 eq.) xantphos, 20 mg (0.1 eq.) Pd₂dba₃ were added. RM was heated under the same conditions for 30 min. Subsequently, RM was diluted with DCM and filtered through Celite^{®}. The filtrate was evaporated to dryness. Purification by silica gel column chromatography using gradient from 0 to 100% of EtOAc+2.5%TEA in hexane yielded Intermediate 22 (58 mg, Y = 77%). UPLC-MS: 94% (280 nm); r.t.= 1.44 min; m/z(+)= 367.6.

### Intermediate 23: 7-(5-cyclopropyl-2-fluorophenyl)-1H,2H,3H-pyrido[3,4-b][1,4]oxazine

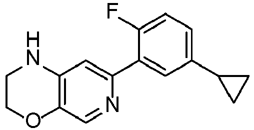

The title compound was prepared following the procedure used for the synthesis of Intermediate 2, starting from Intermediate 1 (50 mg, 1.0 eq.) and using 46 mg (1.3 eq.) of 5-cyclopropyl-2-fluorophenyl boronic acid. RM was heated to 150 °C under microwave irradiation for 15 min. RM was diluted with DCM and filtered through Celite^{®}. The filtrate was extracted with 5×10 mL of 6 M HCl. Acid layers were combined, filtered through Celite^{®}, basified with 6M NaOH and extracted with 5×20 mL of DCM. Combined organics were dried over Na₂SO₄ and evaporated to give Intermediate 23 (47 mg, Y = 75%). UPLC-MS: 90% (280 nm); r.t.= 1.41 min; m/z(+)= 271.5.

### Intermediate 24: 4-[7-(5-cyclopropyl-2-fluorophenyl)-1H,2H,3H-pyrido[3,4-b][1,4]oxazin-1-yl]-2-nitropyridine

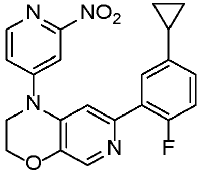

The title compound was prepared following the procedure used for the synthesis of Intermediate 3, starting from Intermediate 23 (64 mg, 1.0 eq.). Obtained Intermediate 24 (82 mg, Y = 88%). UPLC-MS: 97% (280 nm); r.t.= 1.49 min; m/z(+)= 393.7.

### Intermediate 25: 7-[2-fluoro-5-(propan-2-yl)phenyl]-1H,2H,3H-pyrido[3,4-b][1,4]oxazine

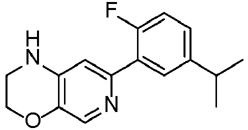

The title compound was prepared following the procedure used for the synthesis of Intermediate 2, starting from Intermdiate 1 (70 mg, 1.0 eq.) and using 5-isopropyl-2-fluorophenylboronic acid. Obtained Intermediate 25 (70 mg, Y = 79%). UPLC-MS: 100% (280 nm); r.t.= 1.46 min; m/z(+)= 273.5.

### Intermediate 26: 4-{7-[2-fluoro-5-(propan-2-yl)phenyl]-1H,2H,3H-pyrido[3,4-b][1,4]oxazin-1-yll-2-nitropyridine

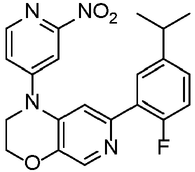

The title compound was prepared following the procedure used for the synthesis of Intermediate 3, starting from Intermediate 25 (63 mg, 1.0 eq.). Obtained Intermediate 26 (57 mg, Y = 63%). UPLC-MS: 89% (280 nm); r.t.= 1.60 min; m/z(+)= 395.8.

### Intermediate 27: 4-bromo-1-{[2-(trimethylsilyl)ethoxy]methyl}-1H-pyrazolo [3,4-b]pyridine

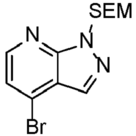

300 mg of 4-bromo-1*H*-pyrazolo[3,4-b]pyridine (1.0 eq.) was dissolved in 4.50 mL of dry THF. 0.53 mL (2.5 eq.) of TEA was added to the solution followed by the addition of 0.274 mL (1.5 eq.) of SEM-Cl at 0 °C. RM was stirred at RT for 18 h. The mixture was diluted with 20 mL of DCM and washed with 20 mL of water. The aqueous layer was extracted with DCM (3×20 mL). Combined organics were dried over Na₂SO₄ and evaporated. Purification by silica gel column chromatography eluting with gradient from 0 to100% of EtOAc+2.5%TEA in hexane yielded Intermediate 17 (150 mg, Y = 30%). UPLC-MS: 90% (280 nm); r.t.= 1.91 min; m/z(+)= 328.6.

### Intermediate 28: 4-bromo-1-{[2-(trimethylsilyl)ethoxy]methyl}-1H-pyrrolo[2,3-b]pyridine

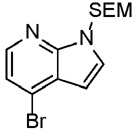

The title compound was prepared following the procedure used for the synthesis of Intermediate 27, starting from of 4-bromo-1H-pyrrolo[2,3-b]pyridine (1.0 g, 1.0 eq.). Obtained Intermediate 28 (341 mg, Y = 21%). UPLC-MS: 93% (280 nm); r.t.= 2.00 min; m/z(+)= 329.3.

### Intermediate 29: 7-bromo-3/4-{[2-(trimethylsilyl)ethoxy]methyl}-3/4H-imidazo[4,5-b]pyridine

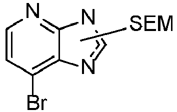

The title compound was prepared following the procedure used for the synthesis of Intermediate 27, starting from 7-bromo-3*H*-imidazo[4,5-b]pyridine (200 mg, 1.0 eq.). Obtained Intermediate 29 as mixture of regioisomers (280 mg, Y = 85%). UPLC-MS: 95% - sum of both regioisomers (280 nm); r.t.= 1.71 and 1.79 min; m/z(+)= 328.5.

### Intermediate 30: 3-amino-N-{4-[7-(5-chloro-2-fluorophenyl)-1H,2H,3H-pyrido[3,4-b][1,4]oxazin-1-yl]pyridin-2-yl}propanamide

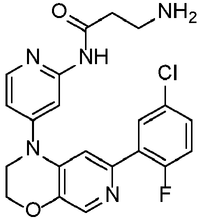

Example 3 (125 mg, 1.0 eq.) was dissolved in dry THF (6.3 mL) and TEA (8.0 eq.) was added. RM was cooled to -40 °C and a solution of 3-(1,3-dioxo-1,3-dihydro-2H-isoindol-2-yl)propanoyl chloride (2.3 eq.) in dry THF (6.3 mL) was added. RM was stirred at RT for 72 h. Additional TEA (5.0 eq.) was added followed by 3-(1,3-dioxo-1,3-dihydro-2H-isoindol-2-yl)propanoyl chloride (0.5 eq.). RM was heated to 60 °C for 18 h. Volatiles were removed and the residue (400 mg) was dissolved in 20 mL of dry THF. Methylhydrazine (225 µL) was added the RM was stirred at 60 °C for 18 h, then additional methylhydrazine (75 µL) was added allowing the mixture to stir at the same temperature for 24 h. Volatiles were removed under reduced pressure and the crude partitioned between water (25 mL) and DCM (10 mL). Aqueous layer was extracted with DCM (3×10 mL). Combined organics were washed with brine and dried over Na₂SO₄, filtered and evaporated. The residue was triturated with diethyl ether to afford Intermediate 30 (90 mg, Y = 60%). UPLC-MS: 90% (280 nm); r.t.= 1.71 and 1.49 min; m/z(+)= 428.2.

### Intermediate 31: 5-bromo-1-{[2-(trimethylsilyl)ethoxy]methyl}-1H-pyrazole

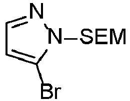

The title compound was prepared following the procedure used for the synthesis of Intermediate 27, starting from 5-bromo-1H-pyrazole (200 mg, 1.0 eq.). Obtained Intermediate 31 (300 mg, Y= 79%). UPLC-MS: 95% (254 nm); r.t.= 1.83; not ionizing.

### Intermediate 32: N-[2-(piperazin-1-yl)ethyl]methanesulfonamide

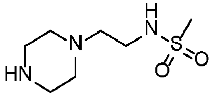

Methanesulfonyl chloride (0.7 mL, 1.55 eq.) was added to a cold (0 °C) solution of 4-(2-amino-ethyl)-piperazine-1-carboxylic acid tert-butyl ester (1.33 g, 1.0 eq.) in pyridine (25.0 mL). The reaction was stirred for 12 h at RT and partitioned between aq. sat. NaHCOs and DCM. The organic layer was separated, dried over Na₂SO₄, filtered and dried under reduced pressure. Purification by silica gel column chromatography eluting with gradient from 0 to 5% of MeOH in DCM yielded Boc-protected Intermediate 32 (0.97 g, Y = 54%) as a white solid. 0.5 g of the solid was dissolved in 20 mL of DCM, cooled to 0 °C and TFA (1.1 mL, 9.0 eq.) was added to the solution. After 4 h at RT, the RM was diluted with 5 mL of MeOH and basified with solid NaHCO₃. The solids were filtered off and volatiles were removed under reduced pressure. Purification by silica gel column chromatography eluting with gradient from 5 to 20% of MeOH + 2% TEA in DCM yielded Intermediate 32 (130 mg, Y = 38%) as a colorless, hygroscopic oil. ¹H NMR (300 MHz, Chloroform-*d*) δ 3.25 - 3.15 (m, 2H), 2.98 (s, 3H), 2.88 (t, *J* = 4.9 Hz, 4H), 2.58 - 2.48 (m, 2H), 2.44 (t, *J* = 4.8 Hz, 4H).

### Intermediate 33: N-methyl-3-(piperazin-1-yl)propanamide

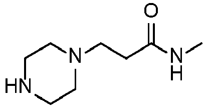

500 mg of 3-{4-[(tert-butoxy)carbonyl]piperazin-1-yl}propanoic acid (1.0 eq.), 830 mg (1.2 eq.) of HATU and 700 mg (3.0 eq.) of DIPEA were dissolved in 20 mL of DMF. 1.1 mL (1.0 eq.) of methylamine (2.0 M in THF) was added to the solution and the RM was stirred for 18 h at RT. Volatiles were removed under reduced pressure and the residue was partitioned between EtOAc and water. The aqueous layer was extracted with EtOAc (3 × 20 mL) and the combined organics were washed with brine. After drying over Na₂SO₄ and filtration, the organic layers were evaporated to give crude Boc-protected Intermediate 33 (450 mg), which was dissolved in 10 mL of DCM and cooled to 0 °C before addition of 2 mL (15.0 eq.) of TFA. RM was stirred at RT for 2 h. Removal of volatiles under reduced pressure afforded the crude Intermediate 33 (1020 mg) as TFA salt, which was used as such in the synthesis of Example 31. UPLC-MS: 53% (MS⁺ detector), r.t.= 0.53 min and 0.61 min; m/z(+) = 172.1.

### Intermediate 34: N-(2-hydroxyethyl)-3-(piperazin-1-yl)propanamide

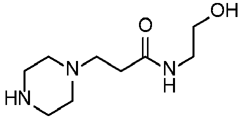

550 mg of 3-{4-[(tert-butoxy)carbonyl]piperazin-1-yl}propanoic acid (1.0 eq.) was dissolved in 30 mL of dry DCM and cooled to 0 °C. 2.8 mL (16 eq.) of thionyl chloride was added dropwise to the solution. RM was allowed to warm to RT and stirred for 3 h. Volatiles were removed under reduced pressure to give the crude acyl chloride (590 mg). The chloride was suspended in 24 mL of dry THF and 330 mg (3.0 eq.) of ethanolamine was added to the suspension. The mixture was stirred at RT for 2 h and evaporated to dryness under reduced pressure to give crude, Boc-protected Intermediate 34 (450 mg), which was dissolved in dry DCM (10 mL) and cooled to 0 °C before addition of 2 mL (15.0 eq.) of TFA. RM was stirred at RT for 2 h. Removal of volatiles under reduced pressure afforded the crude Intermediate 34 (1360 mg) as a TFA salt, which was used as such in the synthesis of Example 35. UPLC-MS: 80% (MS⁺ detector), r.t.= 1.15 min; m/z(+) = 202.2.

### Intermediate 35: 2-[(2-hydroxyethyl)[2-(piperazin-1-yl)ethyl]amino]ethan-1-ol

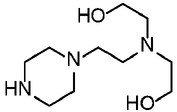

230 mg of *tert*-butyl 4-(2-aminoethyl)piperazine-1-carboxylate (1.0 eq.) was dissolved in 1.8 mL of dry THF under argon atmosphere. Oxirane, 2.5-3.3 M solution in THF, (0.7 mL, 2.0 eq.) was added dropwise to the solution and RM was stirred at RT for 18 h. After that time, the volatiles were removed under reduced pressure to give 760 mg of crude product. 350 mg of crude, Boc-protected Intermediate 35 was dissolved in 7 mL of dry DCM and cooled to 0 °C under argon atmosphere. 2 mL (3.0 eq.) of TFA was added dropwise and the RM was stirred at RT for 2 h. Volatiles were removed under reduced pressure and the residue was filtered through silica using methanol as eluent to yield Intermediate 35 (320 mg, Y = 88%) as TFA salt. UPLC-MS: 80% (MS⁺ detector), r.t.= 1.27 min; m/z(+) = 259.1 (ACN adduct).

### Intermediate 36: 3-(piperazin-1-yl)propanamide

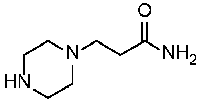

Following literature procedure *Collect.* Czech. Chem. Commun., 1986, 51(11), 2598 - 2616, starting from commercial piperazine (4.3g, 1.0 eq.), Intermediate 36 (2.2 g, Y = 28%) was obtained.¹H NMR (300 MHz, Chloroform-*d*) δ 8.13 (s, 1H), 5.69 (s, 1H), 2.88 (t, *J* = 4.9 Hz, 4H), 2.59 (dd, *J* = 6.7, 5.4 Hz, 2H), 2.46 (s, 4H), 2.38 (dd, *J* = 6.7, 5.4 Hz, 2H).

### Intermediate 37: 2-chloro-5-fluoro-4-(trimethylstannyl)pyridine

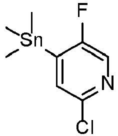

The title compound was prepared following the procedure used for the synthesis of Intermediate 17, starting from 2-chloro-5-fluoro-4-iodopyridine (1g, 1.0 eq). The mixture was diluted with hexane and filtered through Celite^{®}. Purification by silica gel column chromatography eluting with hexane with 5% TEA yielded Intermediate 37 (945 mg, Y = 82%) as colorless oil. ¹H NMR (300 MHz, Chloroform-*d*) δ 8.14 (d, *J=* 1.4 Hz, 1H), 7.33 (d, *J* = 2.2 Hz, 1H),5.69 (s, 1H), 0.44 (s, 9H).

### Intermediate 38: tert-butyl 7-(2-chloro-5-fluoropyridin-4-yl)-1H,2H,3H-pyrido[3,4-b][1,4] oxazine-1-carboxylate

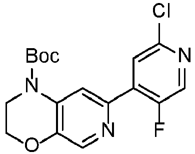

The title compound was prepared following the procedure used for the synthesis of Intermediate 18, starting from Intermediate 16 (100 mg, 1.0 eq.) and Intermediate 37 (102 mg, 1.1 eq). The mixture was diluted with DCM and filtered through Celite^{®}. The filtrate was concentrated under reduced pressure and the residue was dissolved in DCM. The organic phase was washed with 1:1 mixture of aq. sat. NH₄Cl and aq. NH₄ (3 × 15 mL) and with brine. Combined organics were dried over Na₂SO₄, filtered and volatiles were removed under reduced pressure to give Intermediate 38 (107 mg, crude), which was used as such in next steps. UPLC-MS (254 nm): 60% Intermediate 38 (r.t.= 1.82, not ionizing), 30% Intermediate 39 (r.t.= 1.25 min; m/z(+) = 266.4).

### Intermediate 39: 2-chloro-5-fluoro-4-{1H,2H,3H-pyrido[3,4-b] [1,4]oxazin-7-yl}pyridine

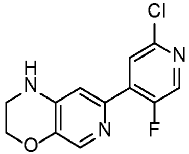

123 mg of crude Intermediate 38 (1.0 eq.) was dissolved in 5 mL of MeOH and 2.5 mL (2.6 eq.) of 4N HCl in dioxane was added to the solution. The RM was stirred at 50 °C for 1 h. The mixture was dried under reduced pressure and the residue was dissolved in aq. 1M HCl and washed with DCM. The aqueous phase was basified using aq. 6M NaOH and extracted with DCM. Combined organics were dried over Na₂SO₄, filtered and volatiles were removed under reduced pressure to give Intermediate 39 (71 mg, Y = 80%) as a white powder. UPLC-MS (254 nm): 100%, r.t.= 1.25 min; m/z(+) = 266.4.

### Intermediate 40: 2-chloro-5-fluoro-4-[1-(2-nitropyridin-4-yl)-1H,2H,3H-pyrido[3,4-b][1,4]oxazin-7-yl]pyridine

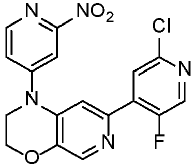

The title compound was prepared following the procedure used for the synthesis of Intermediate 3, starting from Intermediate 39 (125 mg, 1.0 eq.). RM was diluted with DCM, filtered through Celite^{®} and dried under reduced pressure. Purification by silica gel column chromatography using isocratic elution with EtOAc:hexane:TEA:MeOH (47:46:5:3, v/v/v/v) mixture yielded Intermediate 40 (122 mg, Y = 61%) as a yellow solid. UPLC-MS: 100% (280 nm); r.t.= 1.62 min; m/z(+)= 388.3.

### Intermediate 41: 2-chloro-5-fluoro-4-[1-(2-aminopyridin-4-yl)-1H,2H,3H-pyrido[3,4-b][1,4]oxazin-7-yl]pyridine

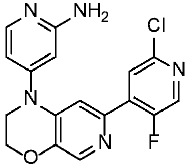

To a stirred solution of 100 mg of Intermediate 40 (1.0 eq.) in ethanol (25 mL) with AcOH (0.15 mL), 58 mg (4.0 eq.) of iron powder was added at 85 °C, followed by addition of 1 drop of concentrated HCl. RM was stirred at the same temperature for 1h, cooled to RT and diluted with DCM. The solution was filtered through Celite^{®}, which was washed with MeOH. The filtrate was dried under reduced pressure and the residue was partitioned between DCM and aq. 6M NaOH. The aqueous phase was extracted with DCM. Combined organics were dried over Na₂SO₄, filtered and volatiles were removed under reduced pressure to give the crude Intermediate 41 (47 mg) as a brown solid. UPLC-MS: 100% (280 nm); r.t.= 1.40 min; m/z(+)= 358.4.

### Intermediate 42: N-(4-bromopyridin-2-yl)-2-(4-methylpiperazin-1-yl)acetamide

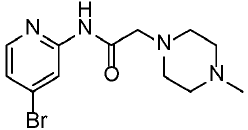

To a stirred solution of 250 mg of 2-amino-4-bromopyridine (1.0 eq.) and 1.0 mL (5.0 eq.) of TEA in 12.5 mL of THF, chloroacetyl chloride (0.2 mL, 1.8 eq.) was added dropwise at 0 °C. After 40 minutes stirring at the same temperature, N-methylpiperazine (1.15 mL, 4.0 eq.) was added and the RM was allowed to warm to RT and further stirred at 50 °C for 30 minutes. Volatiles were removed under reduced pressure and the residue was partitioned between DCM and water. The aqueous phase was extracted with DCM. Combined organics were dried over Na₂SO₄, filtered and volatiles were removed under reduced pressure to give the crude Intermediate 42 (480 mg) as a brown solid, which was used as such in synthesis of Example 43. UPLC-MS (254 nm): 100%; r.t.= 1.29 min; m/z(+)= 313.4.

### Intermediate 43: tert-butyl 7-(5-chloro-2-fluorophenyl)-1H,2H,3H-pyrido[3,4-b][1,4] oxazine-1-carboxylate

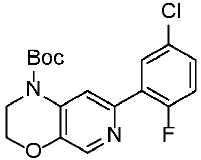

The title compound was prepared following the procedure used for the synthesis of Intermediate 16, using Intermediate 2 (361 mg, 1.0 eq.). The mixture was diluted with DCM, washed with water, extracted to aq. 1M HCl, basified with sat. aq. K₂CO₃ solution, and back-extracted to DCM. Combined organics were dried over Na₂SO₄, filtered and dried under reduced pressure to give the crude Intermediate 43 (435 mg), which was used as such in the next steps. UPLC-MS (254 nm): 92%; r.t.= 1.86 min; m/z(+)= 365.8.

### Intermediate 44: 1-[(tert-butoxy)carbonyl]-7-(5-chloro-2-fluorophenyl)-1H,2H,3H-pyrido[3,4-b] [1,4]oxazin-6-ium-6-olate

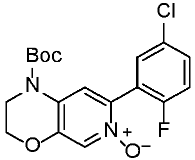

880 mg of Intermediate 43 (1.0 eq.) was dissolved in 17.6 mL of DCE and heated to 60 °C before addition of 1200 mg (3.0 eq.) of *m*-CPBA. RM was stirred at the same temperature for 3 h. The solution was cooled to RT, diluted with DCM and washed with water. The organic layer was separated, dried over Na₂SO₄, filtered and dried under reduced pressure to give Intermediate 44 (825 mg, Y = 90%) which was used as such in the next steps. UPLC-MS (280 nm): 81%; r.t.= 1.65 min; m/z(+)= 381.2.

### Intermediate 45: tert-butyl 7-(5-chloro-2-fluorophenyl)-5-cyano-1H,2H,3H-pyrido [3,4-b] [1,4] oxazine-1-carboxylate-6-olate

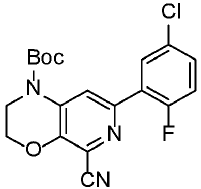

641 mg of Intermediate 44 (1.0 eq.) was suspended in 12.8 mL of dioxane and 0.64 mL (4.0 eq.) of dimethyl sulfate was added dropwise. RM was stirred for 30 minutes at 70 °C. Then, it was cooled to RT and 1100 mg (10 eq.) of KCN was added and the mixture was stirred at 70 °C for 1 h. RM was diluted with EtOAc, filtered through Celite^{®} and dried under reduced pressure to give crude Intermediate 45 (1030 mg) which was used as such in the next steps. ¹H NMR (400 MHz, DMSO-*d*₆) δ 8.68 (s, 1H), 7.84 (dd, *J* = 6.7, 2.8 Hz, 1H), 7.58 (ddd, *J* = 8.8, 4.2, 2.8 Hz, 1H), 7.44 (dd, *J=* 11.0, 8.8 Hz, 1H), 4.51 (dd, *J=* 5.2, 3.8 Hz, 2H), 4.01 - 3.93 (m, 2H), 1.53 (s, 9H).

### Intermediate 46: 7-(5-chloro-2-fluorophenyl)-1H,2H,3H-pyrido[3,4-b][1,4]oxazine-5-carboxylic acid

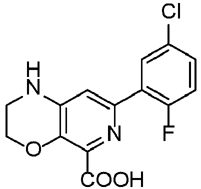

1030 mg of crude Intermediate 45 (1.0 eq.) was dissolved in 21.0 mL of MeOH and 21 mL of aq. 6M NaOH. RM was stirred for 80 minutes at 70 °C. RM was dried under reduced pressure and the residue was partitioned between aq. 1M HCl and DCM. The aqueous layer was dried and triturated with dry MeOH to give the crude Intermediate 46 (996 mg). UPLC-MS (254 nm): 100%; r.t.= 1.29 min; m/z(+)= 309.7.

### Intermediate 47: N-(4-bromopyridin-2-yl)-3-(4-methylpiperazin-1-yl)propanamide

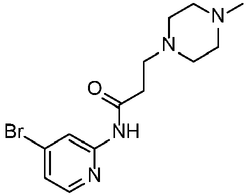

To a stirred solution of 2-amino-4-bromopyridine (2.5g, 1.0 eq.) and TEA (10 mL, 5.0 eq.) in THF (125 mL), 3-bromopropionyl chloride (1.9 mL, 1.3 eq.) was added dropwise at 0 °C. After 30 minutes stirring at the same temperature, N-methylpiperazine (6.4 mL, 4.0 eq.) was added and the RM was allowed to warm to RT and further stirred at 60 °C for 60 minutes. The mixture was filtered through Celite^{®} and dried under reduced pressure. The residue was dissolved in DCM and washed with water three times. Combined organics were dried over Na₂SO₄, filtered and volatiles removed under reduced pressure. Purification by silica gel column chromatography eluting with gradient from 10% to 50% of MeOH in EtOAc yielded Intermediate 47 (3130 mg, Y = 70%) as yellow crystals. UPLC-MS (254 nm): 100%; r.t.= 1.27 min; m/z(+)= 327.2.

### Intermediate 48: methyl 7-(5-chloro-2-fluorophenyl)-1H,2H,3H-pyrido[3,4-b] [1,4]oxazine-5-carboxylate

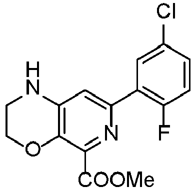

996 mg of crude Intermediate 46 (1.0 eq.) was dissolved in 30 mL of dry MeOH and 10 mL of 4 M HCl in dioxane was added to the solution. RM was stirred at 60 °C for 18 h and dried under reduced pressure. The residue was partitioned between DCM and water. The aqueous phase was basified with sat. aq. K₂CO₃ and extracted with DCM. Combined organics were dried over Na₂SO₄, filtered and dried under reduced pressure to give the crude Intermediate 48 (148 mg). UPLC-MS (254 nm): 80%; r.t.= 1.47 min; m/z(+)= 323.4.

### Intermediate 49: 7-(5-chloro-2-fluorophenyl)-1-{2-[3-(4-methylpiperazin-1-yl)propanamido]pyridin-4-yl}-1H,2H,3H-pyrido[3,4-b][1,4]oxazin-6-ium-6-olate

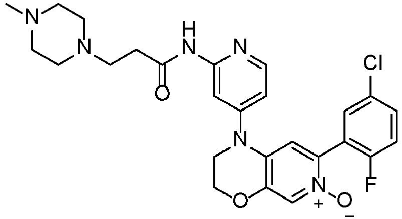

150 mg of Intermediate 44 (1.0 eq.) was suspended in 5 mL of dioxane and 0.7 mL of concentrated HCl was added to the suspension. The RM was stirred at 60 °C for 1 h. Volatiles were removed and the residue was partitioned between DCM and water. Solid NaHCO₃ was added to slightly alkalize the aqueous phase. The organic phase was separated and the water phase was extracted with DCM three times. Combined organics were dried over Na₂SO₄, filtered and dried under reduced pressure to give deprotected Intermediate 44 (107 mg, Y = 97%). 57 mg of this intermediate (1.0 eq.) was suspended in 1 mL dimethylacetamide and 133 mg (2.0 eq.) of Cs₂CO₃ with 133 mg (2.0 eq.) of Intermediate 47 was added to the suspension. The mixture was degassed and saturated with Ar before the addition of XantphosPdG3 (0.1 eq.). The RM was stirred in a sealed vial at 100 °C for 1h. The mixture was cooled to RT, diluted with DCM, filtered through Celite^{®} and dried under reduced pressure. The residue was triturated with ACN and dried to give Intermediate 49 (42 mg, Y = 39%) which was used as such in the synthesis of Example 46. UPLC-MS (254 nm): 90%; r.t.= 1.32 min; m/z(+)= 527.5.

### Intermediate 50: N-(4-bromopyridin-2-yl)prop-2-enamide

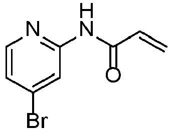

To an ice-cooled stirred suspension of 4-bromo-2-pyridinamine (3 g, 1.0 eq.) and TEA (3.0 eq.) in dry DCM (80 mL), 3-chloropropanoyl chloride (1.1 eq.) in DCM (20 mL) was added dropwise. The solution became a suspension and this was stirred at 0 °C for 1 h. The mixture was diluted with sat. NH₄Cl. Phases were separated and the organic phase was washed with brine, filtered through a phase separator and concentrated under vacuum. Purification by silica gel flash chromatography using gradient from 0% to 30% of EtOAc in c-Hex affording Intermediate 2 (2.86 g, Y = 73%) as pale yellow solid. LC-MS (ESI): *m*/*z* (M+2): 229.0 (Method 1)

### Intermediate 51: 1-Tert-butyl 3-methyl 4-methylpiperazine-1,3-dicarboxylate

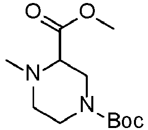

To a suspension of commercially available methyl-4-boc-piperazine-2-carboxylate (150 mg, 1.0 eq.) in MeOH (2.05 mL), acetic acid (3.0 eq.) and formaldehyde 37%w/w in water (5.0 eq.) were added. This mixture was stirred at RT for 30 min, before adding sodium cyanoborohydride (2.0 eq.). The suspension quickly turned into a solution. After 1h volatiles were removed under vacuum, the residual material was taken up with DCM, washed with sat. aq. NaHCO₃. The organic phase was filtered through a phase separator and concentrated under vacuum. Purification by silica gel flash chromatography using gradient from 0% to 5% of MeOH in DCM yielded Intermediate 50 (124 mg, Y =78%) as a pale yellow oil. LC-MS (ESI): *m*/*z* (M+1): 259.2 (Method 2)

### Intermediate 52: Methyl 1-methylpiperazine-2-carboxylate hydrochloride

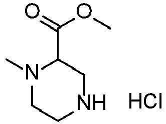

A solution of Intermediate 51 (124 mg, 1.0 eq.) in HCl 4.0 M in dioxane (10.0 eq.) and MeOH (1.2 mL) was stirred at RT for 2 h. Volatiles were removed under vacuum affording methyl Intermediate 52 (160 mg, recovery assumed quantitative) that was used as such in the next step. LC-MS (ESI): *m*/*z* (M+1): 159.1 (Method 2)

### Intermediate 53: Methyl 4-{2-[(4-bromopyridin-2-yl)carbamoyl]ethyl}-1-methylpiperazine-2-carboxylate

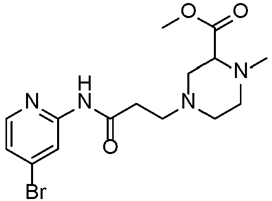

The title compound was prepared following the procedure used for the synthesis of Example 8, starting from Intermediate 50 (83 mg, 1.0 eq.) and using Intermediate 52 (1.3 eq.). Purification by silica gel flash chromatography using gradient from 0% to 4% of MeOH in DCM yielded Intermediate 53 (135 mg, Y= 96%) as pale yellow oil. LC-MS (ESI): *m*/*z* (M+1): 385.1 (Method 1)

### Intermediate 54: 1-Tert-butyl 2-methyl 4-{2-[(4-bromopyridin-2-yl)carbamoyl]ethyl}piperazine-1,2-dicarboxylate

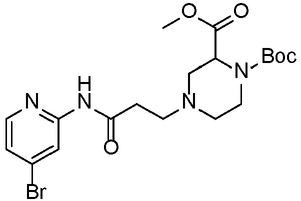

The title compound was prepared following the procedure used for the synthesis of Example 8, starting from Intermediate 50 (160 mg, 1.0 eq.) and using commercially available N-Boc-piperazine-2-carboxylic acid methyl ester (1.3 eq.). Obtained Intermediate 54 (248 mg, Y = 80%). LC-MS (ESI): m/z (M+1): 471.1 (Method 1)

### Intermediate 55: 1-Tert-butyl 2-methyl 4-[2-({4-[7-(5-chloro-2-fluorophenyl)-1H,2H,3H-pyrido [3,4-b] [1,4] oxazin-1-yl] pyridin-2-yl}carbamoyl)ethyl]piperazine-1,2-dicarboxylate

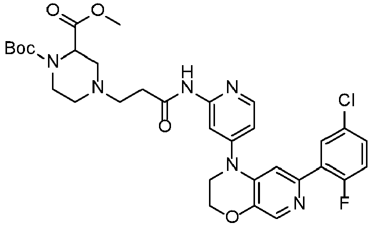

The title compound was prepared following similar procedure to the one used for the synthesis of Intermediate 3, starting from Intermediate 2 (44 mg, 1.0 eq.) and using Intermediate 54 (1.05 eq.), Cs₂CO₃ (1.9 eq.), XanthPhos (0.11 eq.) and Pd(OAc)₂ (0.05 eq.) in dry 1,4-Dioxane (2.16 mL). Purification by NH-modified flash chromatography using gradient from 0% to 40% of EtOAc in c-Hex yielded Intermediate 55 (80 mg, Y = 73%) as an off- white solid. LC-MS (ESI): *m*/*z* (M+1): 655.0 (Method 2)

### Intermediate 56: Ethyl (2-E/Z)-4-[benzyl(12-[(triphenylmethyl)amino] ethyl})amino] but-2-enoate

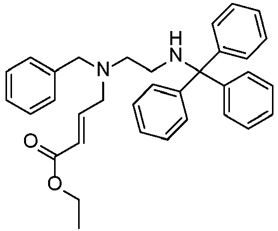

To a solution of N-benzyl-N'-tritylethane-1,2-diamine (1.4 g, 1.0 eq.) in ACN (10 mL) K₂CO₃ (2.0 eq.), and ethyl (2-*E*/*Z*)-4-bromo-2-butenoate (1.0 eq.) were added. The mixture was heated at 50 °C for 90 min. EtOAc and water were added, the product was extracted with EtOAc 2×, organic phases were collected, dried, and evaporated. Purification by silica gel flash chromatography using gradient from 0 to 10% EtOAc in c-Hex yielded Intermediate 56 (1.57 g, Y= 87%) as white wax. LC-MS (ESI): *m*/*z* (M+1): 505.1 (Method 1).

### Intermediate 57: Methyl 2-(4-benzylpiperazin-2-yl)acetate

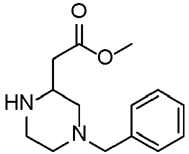

HCl 4M in dioxane (5.0 eq.) was added to a solution of Intermediate 56 (1.57 g, 1.0 eq.) in MeOH (6 mL) and the mixture was refluxed for 1h. Volatiles were removed under vacuum, the residual material was charged in SCX washed with MeOH and eluted with 1N NH₃ in MeOH. Evaporation of basic fractions afforded Intermediate 57 (560 mg, Y = 72%) used as such in the next step. LC-MS (ESI): *m*/*z* (M+1): 248.7 (Method 2)

### Intermediate 58: Tert-butyl 4-benzyl-2-(2-methoxy-2-oxoethyl)piperazine-1-carboxylate

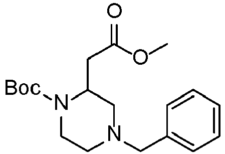

The title compound was prepared following the procedure for the synthesis of Intermediate 16, using Intermediate 57 (560 mg, 1.0 eq.). Purification by silica gel flash chromatography using gradient from 0 to 15% of EtOAc in c-Hex yielded Intermediate 58 (410 mg, Y = 52%) as colorless oil. LC-MS (ESI): *m*/*z* (M+1): 349.2 (Method 1)

### Intermediate 59: Tert-butyl 2-(2-methoxy-2-oxoethyl)piperazine-1-carboxylate

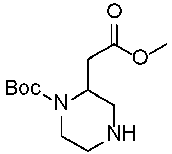

A solution of Intermediate 58 (410 mg, 1.0 eq.) in MeOH (8 mL) was bubbled with N₂, then 10% Pd/C (1.0 eq.) was added, the resulting suspension was stirred for 12h at RT under H₂ atmosphere. Catalyst was filtered over Celite^{®} pad, rinsing with MeOH, the solvent was removed under redueced pressure yielding Intermediate 59 (280 mg, Y= 92%) as colorless oil. LC-MS (ESI): *m*/*z* (M+1): 259.1 (Method 1)

### Intermediate 60: Tert-butyl 4-{2-[(4-bromopyridin-2-yl)carbamoyl]ethyl}-2-(2-methoxy-2-oxoethyl)piperazine-1-carboxylate

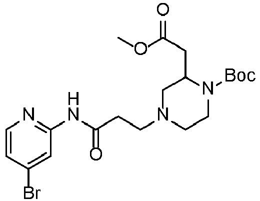

The title compound was prepared following the procedure used for the synthesis of Example 8, starting Intermediate 50 (200 mg, 1.0 eq.) and using Intermediate 59 (1.15 eq.). LC-MS (ESI): m/z (M+1): 487.1 (Method 1)

### Intermediate 61: Methyl 2-(4-{2-[(4-bromopyridin-2-yl)carbamoyl]ethyl}piperazin-2-yl)acetate

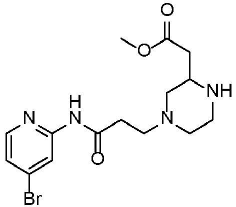

TFA (10.0 eq.) was added to a stirred solution of Intermediate 60 (100 mg, 1.0 eq.) in DCM (3 mL). The mixture was stirred at RT 3 h, volatiles were removed under vacuum, the residue was charged on SCX, washing with MeOH and eluting with 1N NH3 in MeOH. Evaporation of basic fraction afforded Intermediate 61 (100 mg, recovery assumed quantitative) used as such in the next step. LC-MS (ESI): *m*/*z* (M+1): 387.1 (Method 2)

### Intermediate 62: Methyl 2-(4-{2-[(4-bromopyridin-2-yl)carbamoyl]ethyl}-1-methylpiperazin-2-yl)acetate

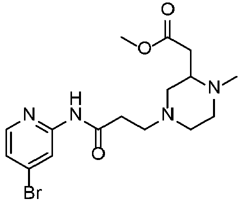

The title compound was prepared following the procedure used for the synthesis of Intermediate 51, starting from Intermediate 61 (100 mg, 1.0 eq.). Obtained Intermediate 62 (100 mg, Y = 97%). LC-MS (ESI): m/z (M+1): 399.1 (Method 1)

### Intermediate 63: Tert-butyl 4-[2-({4-[7-(5-chloro-2-fluorophenyl)-1H,2H,3H-pyrido[3,4-b][1,4]oxazin-1-yl]pyridin-2-yllcarbamoyl)ethyl]-2-(2-methoxy-2-oxoethyl)piperazine-1-carboxylate

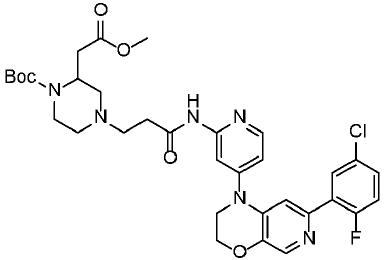

The title compound was prepared following the procedure used for the synthesis of Intermediate 55, starting from Intermediate 2 (45 mg, 1.0 eq.) and using Intermediate 60 (1.15 eq.). LC-MS (ESI): m/z (M+1): 669.5 (Method 2)

### Intermediate 64: 1-Tert-butyl 2-methyl 4-methylpiperazine-1,2-dicarboxylate

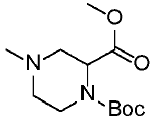

The title compound was prepared following the procedure used for the synthesis of Intermediate 51, starting from commercially available N-Boc-piperazine-2-carboxylic acid methyl ester (600 mg, 1.0 eq.). LC-MS (ESI): m/z (M+1): 259.9 (Method 2)

### Intermediate 65: Methyl 4-methylpiperazine-2-carboxylate hydrochloride

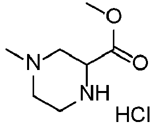

The title compound was prepared following the procedure for the synthesis of Intermediate 52, using Intermediate 64 (470 mg, 1.0 eq.). Obtained Intermediate 65 (557 mg, recovery assumed quantitative) which was used as such in the next step. LC-MS (ESI): m/z (M+1): 159.7 (Method 2)

### Intermediate 66: Methyl 1-{2-[(4-bromopyridin-2-yl)carbamoyl]ethyl}-4-methylpiperazine-2-carboxylate

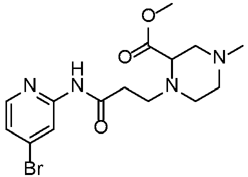

The title compound was prepared following the procedure used for the synthesis of Example 8, starting Intermediate 50 (200 mg, 1.0 eq.) and using Intermediate 65 (1.15 eq.). Obtained Intermediate 66 (191 mg, Y = 38%). LC-MS (ESI): m/z (M+1): 385.6 (Method 2)

### Intermediate 67: Tert-butyl 4-{2-[(4-bromopyridin-2-yl)carbamoyl]ethyl}-3-(2-methoxy-2-oxoethyl)piperazine-1-carboxylate

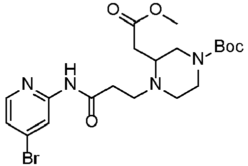

The title compound was prepared following the procedure used for the synthesis of Example 8, starting from Intermediate 50 (200 mg, 1.0 eq.) and using commercially available N-4-Boc-2-piperazineacetic acid methyl ester (1.15 eq.). Obtained Intermediate 67 (250 mg, Y = 78%). LC-MS (ESI): m/z (M+1): 485.0 (Method 2)

### Intermediate 68: Methyl 2-(1-{2-[(4-bromopyridin-2-yl)carbamoyl]ethyl}piperazin-2-yl)acetate

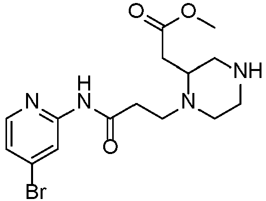

The title compound was prepared following the procedure for the synthesis of Intermediate 52, using Intermediate 67 (250 mg, 1.0 eq.). Obtained Intermediate 65 (185 mg, Y = 93%) which was used as such in the next step. LC-MS (ESI): m/z (M+1): 384.8 (Method 2)

### Intermediate 69: Methyl 2-(1-{2-[(4-bromopyridin-2-yl)carbamoyl] ethyl) -4-methylpiperazin-2-yl)acetate

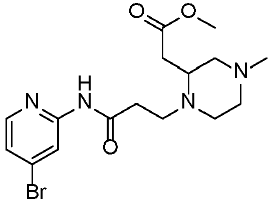

The title compound was prepared following the procedure used for the synthesis of Intermediate 51, starting from Intermediate 68 (185 mg, 1.0 eq.). Obtained Intermediate 69 (175 mg, Y = 92%). LC-MS (ESI): m/z (M+1): 399.6 (Method 2)

### Intermediate 70: N-(4-bromopyridin-2-yl)-2-{5-methyl-2,5-diazabicyclo [2.2.1]heptan-2-yl}acetamide

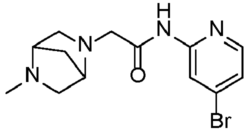

The title compound was prepared following the procedure used for the synthesis of Intermediate 42, starting from freshly-prepared N-(4-bromopyridin-2-yl)-2-chloroacetamide (50 mg, 1.0 eq.) and using 2-methyl-2,5-diazabicyclo[2.2.1]heptane (2.0 eq.) dissolved in MeOH (1.0 mL). Purification by silica gel flash chromatography eluting with hexane7EtOAc/meOH/tea (5:4:1:0.2 v/v/v/v) yielded Intermediate 70 (21 mg, Y = 32%) as yellow solid. UPLC-MS (254 nm): 100%; r.t.= 1.24 min; m/z(+)= 325.2.

### Intermediate 71: N-(4-bromopyridin-2-yl)-2-{6-methyl-2,6-diazaspiro [3.3] heptan- 2-yl} acetamide

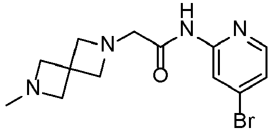

The title compound was prepared following the procedure used for the synthesis of Intermediate 42, starting from freshly-prepared N-(4-bromopyridin-2-yl)-2-chloroacetamide (50 mg, 1.0 eq.) and using 2-methyl-2,6-diazaspiro[3.3]heptane bis(trifluoromethyl formate) (3.5 eq.) dissolved in MeOH (2.0 mL). Purification by silica gel flash chromatography eluting with hexane7EtOAc/meOH/tea (5:4:1:0.5 v/v/v/v) yielded Intermediate 71 (49 mg, Y = 57%) as brown solid. UPLC-MS (254 nm): 94%; r.t.= 1.13 min; m/z(+)= 325.3.

### Intermediate 72: N-(4-bromopyridin-2-yl)-2-{2-methyl-5-oxa-2,8-diazaspiro[3.5] nonan-8-yl}acetamide

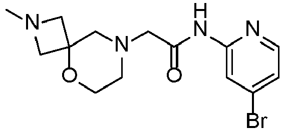

Commercially available *tert*-butyl 5-oxa-2,8-diazaspiro[3.5]nonane-2-carboxylate (550 mg, 1.0 eq.) was dissolved in dry THF (15 mL) and the solution was cooled to 0 °C. LiAlH₄ 1.0M in THF (7.2 mL, 3.0 eq.) was added dropwise and the resulting mixture was heated to 45 °C and stirred for 30 minutes. Then, 20% aq. solution of NaOH was added, the precipitated product was filtered, washed with THF and dried to give the crude product (294 mg) which was reacted with freshly prepared N-(4-bromopyridin-2-yl)-2-chloroacetamide (117 mg, 1.0 eq.) following the procedure described for the synthesis of Intermediate 70. Purification by silica gel flash chromatography eluting with hexane/EtOAc/MeOH/TEA (5:4:1:0.5 v/v/v/v) yielded Intermediate 72 (83 mg, Y = 45%) as brown solid. UPLC-MS (254 nm): 79%; r.t.= 1.50 min; m/z(+)= 355.6.

### Intermediate 73: 1-(4-bromopyridin-2-yl)-3-(2-(4-methylpiperazin-1-yl)ethyl)urea

Step 1. To a solution of bis(2,5-dioxopyrrolidin-1-yl) carbonate (537 mg, 1.0 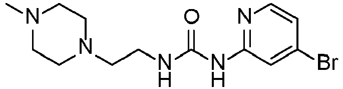 eq.) in dry THF (10 mL), 2-(4-methylpiperazin-1-yl)ethan-1-amine (300 mg, 1.0 eq.) was added and the reaction was stirred at 80 °C for 1h.

Step 2. To a solution of 4-bromopyridin-2-amine (1.0 eq.) in dry THF (10 mL), cooled to -78 °C, LDA 2M in THF (3.0 eq.) was added dropwise. The reaction was stirred for 10 min, then the suspension from Step 1 was added dropwise over a period of 30 minutes to the mixture. The reaction was stirred for 30 minutes at -78 °C and for 12h at RT. The reaction was poured into brine (20 mL) and the organic phase was extracted with DCM (30 mL × 3). Combined organics were filtered through a phase separator tube and volatiles removed under reduced pressure. Purification by NH-modified flash chromatography using gradient from 0 to 5% of EtOH in DCM yielded Intermediate 73 (120 mg, Y = 24%) as an orange solid. LC-MS (ESI): m/z (M+1): 342.2 - 344.2 (Method 1).

### Intermediate 74: 7-Bromo-5-(trifluoromethyl)-2,3-dihydro-1H-pyrido[3,4-b][1,4] oxazine

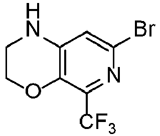

To a suspension of Intermediate 1 (200 mg, 1.0 eq.) in DCM (1.0 mL), TFA (2.0 eq.), zinc trifluoromethanesulfinate (1.0 eq.) and water (1.0 mL) were added. Then, 2-hydroperoxy-2-methylpropane (2.0 eq.) was added and the resulting suspension was heated to 60 °C and stirred for 12h. Volatiles were removed under reduced pressure and Intermediate 74 was used as such in the next step. LC-MS (ESI): m/z (M+1): 282.7 - 284.7 (Method 1).

### Intermediate 75: 7-(5-Chloro-2-fluorophenyl)-5-(trifluoromethyl)-2,3-dihydro-1H-pyrido[3,4-b][1,4]oxazine

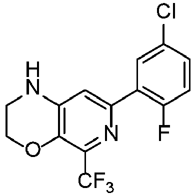

The title compound was prepared following the procedure used for the synthesis of Intermediate 2, starting from Intermediate 74 (263 mg, 1.0 eq.) and using the commercial (5-chloro-2-fluorophenyl)boronic acid (1.5 eq.), potassium carbonate (3.0 eq.) and PdCl₂(dppf) (0.2 eq.). Purification by reverse phase flash chromatography using gradient from 0 to 50% of B in A (A:water/ACN 95:5 + 0.1% formic acid, B: ACN/water 95:5 + 0.1% formic acid) yielded Intermediate 75 (40 mg, Y = 13%) as brownish solid. LC-MS (ESI): m/z (M+1): 332.9 (Method 1).

### PREPARATIONS OF EXAMPLES

### Example 1: 4-[7-(5-Chloro-2-fluorophenyl)-1H,2H,3H-pyrido[3,4-b][1,4]oxazin-1-yl]pyridine

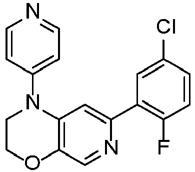

The title compound was prepared following the procedure used for the synthesis of Intermediate 3, using Intermediate 2 (50 mg, 1.0 eq.) and 4-bromopyridine hydrochloride (1.5 eq.). The RM was diluted with DCM and filtered through Celite^{®}. The organic layer was concentrated and partitioned between water (25 mL) and DCM (25 mL).The two phases were separated and the aqueous layer was further extracted DCM (2 × 25 mL). Combined organics were dried over Na₂SO₄, filtered and volatiles were removed under reduced pressure. Purification by preparative HPLC using ACN:water gradient with 0.1% of formic acid yielded Example 1 (25 mg, Y= 40%) as a white powder. ¹H NMR (300 MHz, DMSO-*d*₆) δ 8.56 - 8.48 (m, 2H), 8.24 (s, 1H), 7.92 (dd, *J* = 6.9, 2.8 Hz, 1H), 7.60 (d, *J* = 1.0 Hz, 1H), 7.46 (ddd, *J=* 8.8, 4.2, 2.8 Hz, 1H), 7.40 - 7.26 (m, 3H), 4.39 (dd, *J* = 5.2, 3.6 Hz, 2H), 3.89 (t, *J* = 4.3 Hz, 2H). LCMS (Method E): 210 nm: 93.73%, 254 nm: 95.02%, 220 nm: 97.36%; r.t.= 3.300 min; m/z(+)= 342.13.

### Example 2: 7-(5-Chloro-2-fluorophenyl)-1-(pyridin-4-yl)-1H,2H,3H-pyrido[3,4-b][1,4]oxazin-5-amine

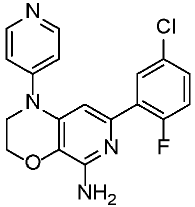

300 mg of Intermediate 11 was suspended in 4.5 mL DCM and 0.91 mL (20.0 eq.) TFA was added. RM was stirred for 2 h at RT. After that time, the mixture was dried under reduced pressure and the residue was dissolved in 30 mL DCM and washed with 10 mL of 6M NaOH. The aqueous layer was extracted 3 × 30 mL of DCM. Combined organics were dried over Na₂SO₄, filtered and evaporated. Purification by pTLC on silica gel, using DCM:MeOH:TEA (9.6:0.4:0.1) mixture as eluent yielded Example 2 (48 mg, Y = 30%) as a beige powder. ¹H NMR (400 MHz, DMSO-*d*₆) δ 8.50 - 8.38 (m, 2H), 7.97 (dd, *J* = 6.9, 2.8 Hz, 1H), 7.40 (ddd, *J* = 8.7, 4.1, 2.8 Hz, 1H), 7.31 - 7.23 (m, 3H), 6.99 (d, *J* = 1.0 Hz, 1H), 5.93 (s, 2H), 4.35 (dd, *J* = 5.1, 3.5 Hz, 2H), 3.93 - 3.81 (m, 2H). LCMS (Method E): 254 nm: 98.44, 220 nm: 98.44%, 205 nm: 98.70%; r.t.= 2.877 min; m/z(+)= 497.78.

### Example 3: 4-[7-(5-Chloro-2-fluorophenyl)-1H,2H,3H-pyrido[3,4-b][1,4]oxazin-1-yl]-2-aminopyridine

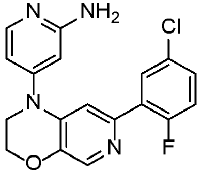

The title compound was prepared following the procedure used for the synthesis of Intermediate 41, starting from Intermediate 3 (800 mg, 1.0 eq.). RM was diluted with MeOH and filtered through Celite^{®}. Organic layer was evaporated to dryness and the residue was partitioned between 250 mL DCM and 100 mL 6 M NaOH. The resulting emulsion was filtered and washed with DCM. Basic layer was extracted with 5 × 100 mL of DCM. Combined organics were dried over Na₂SO₄. 60 mL MeOH and 10 mL of TEA were added to the DCM and the suspension was filtered through Celite^{®} and a thin layer of silica gel. The filtrate was dried under reduced pressure to give Example 3 (728 mg, Y= 99%) as a white powder. ¹H NMR (300 MHz, DMSO-*d*₆) δ 8.19 (s, 1H), 7.97 - 7.86 (m, 2H), 7.54 (d, *J* = 1.0 Hz, 1H), 7.47 (ddd, *J* = 8.8, 4.2, 2.8 Hz, 1H), 7.34 (dd, *J* = 11.1, 8.8 Hz, 1H), 6.50 (dd, *J* = 5.7, 2.0 Hz, 1H), 6.34 (d, *J* = 2.0 Hz, 1H), 5.99 (s, 2H), 4.37 (dd, *J* = 5.2, 3.4 Hz, 2H), 3.80 (t, *J* = 4.3 Hz, 2H). LCMS (Method B): 220 nm: 92.37%, 254 nm: 90.05%; r.t.= 2.047 min; m/z(+)= 356.97.

### Example 4: 4-[7-(2,5-Difluorophenyl)-1H,2H,3H-pyrido[3,4-b][1,4]oxazin-1-yl]pyridin-2-amine

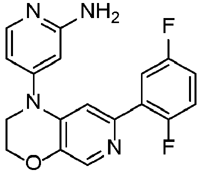

The title compound was prepared following the procedure used for the synthesis of Intermediate 41, starting from Intermediate 13 (100 mg, 1.0 eq.). RM was worked-up as for Example 3. Purification by pTLC on silica gel, using DCM:MeOH:TEA (9.6:0.4:0.25) mixture as eluent, eluted 3 times, yielded Example 4 (54 mg, Y = 60%) as a white powder. ¹H NMR (400 MHz, DMSO-*d*₆) δ 8.19 (s, 1H), 7.89 (d, *J* = 5.7 Hz, 1H), 7.69 (ddd, *J* = 9.6, 6.2, 3.3 Hz, 1H), 7.55 (d, *J* = 0.9 Hz, 1H), 7.37 - 7.21 (m, 2H), 6.50 (dd, *J* = 5.7, 2.1 Hz, 1H), 6.35 (d, *J* = 2.0 Hz, 1H), 6.00 (s, 2H), 4.41 - 4.31 (m, 2H), 3.79 (t, *J* = 4.3 Hz, 2H). LCMS (Method B): 220 nm: 99.33%, 254 nm: 98.71%; r.t.= 1.740 min; m/z(+)= 340.97.

### Example 5: 4-[7-(3-Chlorophenyl)-1H,2H,3H-pyrido[3,4-b][1,4]oxazin-1-yl]pyridin-2-amine

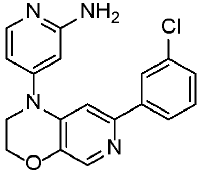

The title compound was prepared following the procedure used for the synthesis of Intermediate 41, starting from Intermediate 15 (120 mg, 1.0 eq.). RM was worked-up as for Example 3. Purification by pTLC on silica gel, using DCM:MeOH:TEA (9.6:0.4:0.25) mixture as eluent, eluted 2 times yielded Example 5 (38 mg, Y = 30%) as a beige powder. ¹H NMR (400 MHz, DMSO-*d*₆) δ 8.16 (s, 1H), 7.94 - 7.88 (m, 2H), 7.77 (dt, *J* = 7.5, 1.5 Hz, 1H), 7.55 (s, 1H), 7.48 - 7.38 (m, 2H), 6.52 (dd, *J* = 5.7, 2.1 Hz, 1H), 6.40 (d, *J* = 2.0 Hz, 1H), 6.00 (s, 2H), 4.42 - 4.30 (m, 2H), 3.79 (dd, *J* = 5.9, 2.9 Hz, 2H). LCMS (Method E): 254 nm: 95.90%, 220 nm: 98.64%; r.t.= 3.020 min; m/z(+)= 338.97.

### Example 6: N-{4-[7-(5-chloro-2-fluorophenyl)-1H,2H,3H-pyrido[3,4-b][1,4]oxazin-1-yl]pyridin-2-yl}acetamide

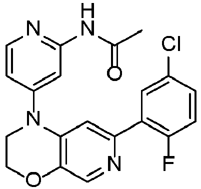

145 mg of Example 3 (1.0 eq.) was suspended in 8.7 mL of dry DCM and cooled to 0 °C. 0.57 mL (10.0 eq.) of TEA and 0.043 (1.5 eq.) acetyl chloride were added to RM and the mixture was stirred for 25 min at RT. RM was evaporated to dryness under reduced pressure and the residue was suspended in dry toluene. 0.21 mL of TEA was added to the mixture and it was stirred at 100 °C for 72 h. RM was cooled to RT and diluted with 20 mL DCM and washed with 20 mL of water. The aqueous layer was extracted with DCM (3 × 20 mL). Combined organics were dried over Na₂SO₄, filtered and dried under reduced pressure. Purification by pTLC on silica gel, using DCM:MeOH:TEA (9.6:0.4:0.25) mixture as eluent, followed by trituration with ACN afforded Example 6 (85 mg, Y = 52%) as a beige powder. ¹H NMR (400 MHz, DMSO-*d*₆) δ 10.54 (s, 1H), 8.29 - 8.22 (m, 2H), 8.11 (s, 1H), 7.93 (dd, *J* = 6.8, 2.8 Hz, 1H), 7.62 (s, 1H), 7.46 (ddd, *J* = 8.8, 4.1, 2.8 Hz, 1H), 7.32 (dd, *J* = 11.0, 8.8 Hz, 1H), 7.07 (dd, *J* = 5.7, 2.2 Hz, 1H), 4.43 - 4.36 (m, 2H), 3.88 (t, *J* = 4.3 Hz, 2H), 2.09 (s, 3H). LCMS (Method C): 220 nm: 98.65%, 235 nm: 98.56%, 254 nm: 98.36%; r.t.= 1.617 min; m/z(+)= 398.87

### Example 7: 4-[7-(6-Chloro-3-fluoropyridin-2-yl)-1H,2H,3H-pyrido[3,4-b][1,4]oxazin-1-yl]pyridin-2-amine

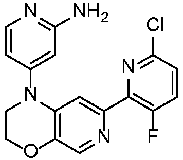

The title compound was prepared following the procedure used for the synthesis of Intermediate 41, starting from Intermediate 20 (15 mg, 1.0 eq.). RM was worked-up as for Example 3. Purification by pTLC on NHz-modified silica using DCM as eluent yielded Example 7 (9 mg, Y = 65%) as a beige powder. ¹H NMR (400 MHz, DMSO-*d*₆) δ 8.17 (s, 1H), 7.94 - 7.82 (m, 2H), 7.64 (s, 1H), 7.56 (dd, *J* = 8.6, 2.9 Hz, 1H), 6.50 (dd, *J* = 5.7, 2.1 Hz, 1H), 6.34 (d, *J* = 2.0 Hz, 1H), 5.99 (s, 2H), 4.44 - 4.30 (m, 2H), 3.85 - 3.72 (m, 2H). LCMS (Method E): 220 nm: 97.57%, 254 nm: 97.13%, 310 nm: 97.30%; r.t.= 2.630 min; m/z(+)= 357.96.

### Example 8: N-{4-[7-(5-chloro-2-fluorophenyl)-1H,2H,3H-pyrido[3,4-b][1,4]oxazin-1-yl]pyridin-2-yl}-3-(morpholin-4-yl)propanamide

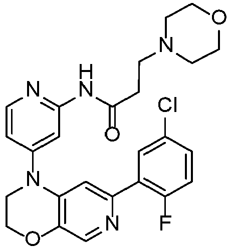

Freshly-prepared Intermediate 4 (88 mg, 1.0 eq.) was dissolved in MeOH (3.7 mL). Morpholine (1.7 eq.) and TEA (1.25 eq.) were added and the resulting RM was stirred for 18 h at RT. The mixture was evaporated to dryness. Purification by preparative HPLC using ACN:water gradient with 0.1% of formic acid yielded Example 8 (9 mg, Y= 8%) as a beige powder. ¹H NMR (400 MHz, DMSO-*d*₆) δ 10.72 (s, 1H), 8.29 - 8.19 (m, 2H), 8.14 (d, *J* = 2.1 Hz, 1H), 7.93 (dd, *J* = 6.8, 2.8 Hz, 1H), 7.61 (s, 1H), 7.46 (ddd, *J* = 8.8, 4.2, 2.8 Hz, 1H), 7.31 (dd, *J* = 11.0, 8.8 Hz, 1H), 7.07 (dd, *J* = 5.7, 2.2 Hz, 1H), 4.40 (dd, *J* = 5.1, 3.5 Hz, 2H), 3.88 (t, *J* = 4.3 Hz, 2H), 3.57 (t, *J* = 4.6 Hz, 4H), 2.61 (dd, *J* = 7.4, 4.9 Hz, 2H), 2.56 (d, *J* = 6.1 Hz, 2H), 2.40 (t, *J* = 4.6 Hz, 4H). LCMS (Method E): 254 nm: 98.44, 220 nm: 98.44%, 205 nm: 98.70%; r.t.= 2.877 min; m/z(+)= 497.78.

### Example 9: N-{4-[7-(5-chloro-2-fluorophenyl)-1H,2H,3H-pyrido[3,4-b][1,4]oxazin-1-yl]pyridin-2-yl}-3-(dimethylamino)propanamide

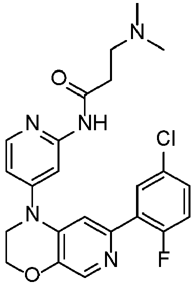

The title compound was prepared following the procedure used for the synthesis of Example 8, starting from freshly-prepared Intermediate 4 (100 mg, 1.0 eq.) and using dimethylamine hydrochloride (1.7 eq.). Purification by preparative HPLC using ACN:water gradient with 0.1% of formic acid yielded Example 9 (13 mg, Y= 12%) as a beige powder. ¹H NMR (400 MHz, Chloroform-*d*) δ 8.29 (s, 1H), 8.21 (d, *J* = 5.7 Hz, 1H), 8.11 (d, *J* = 2.2 Hz, 1H), 7.93 (dd, *J* = 6.7, 2.8 Hz, 1H), 7.61 (d, *J* = 1.3 Hz, 1H), 7.25 - 7.22 (m, 1H), 7.07 - 6.96 (m, 2H), 4.40 (t, *J* = 4.4 Hz, 2H), 3.88 (t, *J* = 4.4 Hz, 2H), 2.87 (t, *J* = 6.2 Hz, 2H), 2.68 (t, *J* = 6.3 Hz, 2H), 2.49 (s, 6H). LCMS (Method H): 254 nm: 93.80%, 220 nm: 92.80%, 240 nm: 94.42%; r.t.= 4.197 min; m/z(+)= 456.00.

### Example 10: N-{4-[7-(5-chloro-2-fluorophenyl)-1H,2H,3H-pyrido[3,4-b][1,4]oxazin-1-yl]pyridin-2-yl}-3-(piperazin-1-yl)propanamide

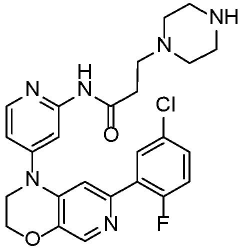

The title compound was prepared following the procedure used for the synthesis of Example 8, starting from freshly-prepared Intermediate 4 (100 mg, 1.0 eq.) and using *tert*-butyl piperazine-1-carboxylate (1.7 eq.). Purification by silica gel column chromatography eluting with gradient from 0 to 100% of EtOAc in hexane yielded Boc-protected Example 10 (77 mg, Y=54%). Boc-protected product (30 mg, 1.0 eq.) was dissolved in 1.26 mL of 1.25M HCl in MeOH and stirred at RT for 24 h. Example 10 as HCl salt was collected by filtration (28 mg, Y= 98%) as a white powder. ¹H NMR (400 MHz, Methanol-*d*₄) δ 8.45 (d, *J* = 6.3 Hz, 1H), 8.42 (s, 1H), 7.97 (s, 1H), 7.85 (dd, *J* = 6.4, 2.6 Hz, 1H), 7.70 (s, 1H), 7.66 (ddd, *J* = 8.9, 4.4, 2.7 Hz, 1H), 7.48 (dd, *J* = 6.2, 2.2 Hz, 1H), 7.39 (dd, *J* = 10.1, 8.9 Hz, 1H), 4.64 (t, *J* = 4.5 Hz, 2H), 4.21 - 4.15 (m, 2H), 3.66 (br. s, 10H), 3.18 (t, *J* = 6.7 Hz, 2H). LCMS (Method H): 220 nm: 95.26%, 254 nm: 94.91%, 240 nm: 95.52%, r.t.= 4.043, m/z(+)= 496.99.

### Example 11: 4-[7-(2-Fluoro-5-methylphenyl)-1H,2H,3H-pyrido[3,4-b][1,4]oxazin-1-yl]pyridin-2-amine

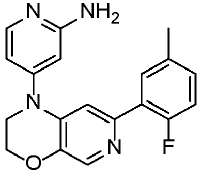

The title compound was prepared following the procedure used for the synthesis of Intermediate 41, starting from Intermediate 22 (74 mg, 1.0 eq.). RM was worked-up as for Example 3. Purification by pTLC on silica gel, using DCM:MeOH:TEA (9.6:0.4:0.25) mixture as eluent, eluted 2 times, yielded Example 11 (31 mg, Y = 44%) as a beige powder. ¹H NMR (400 MHz, DMSO-*d*₆) δ 8.17 (s, 1H), 7.88 (d, *J* = 5.7 Hz, 1H), 7.69 (dd, *J* = 7.9, 2.3 Hz, 1H), 7.48 (d, *J* = 1.2 Hz, 1H), 7.21 - 7.16 (m, 1H), 7.12 (dd, *J* = 11.4, 8.3 Hz, 1H), 6.50 (dd, *J* = 5.7, 2.0 Hz, 1H), 6.34 (d, *J* = 2.0 Hz, 1H), 6.00 (s, 2H), 4.35 (t, *J =* 4.2 Hz, 2H), 3.79 (t, *J* = 4.4 Hz, 2H), 2.32 (s, 3H). LCMS (Method B): 210 nm: 87.93%, 220 nm: 92.67%, 254 nm: 97.43%, r.t.= 1.537 min; m/z(+)= 337.02.

### Example 12: 4-[7-(5-Cyclopropyl-2-fluorophenyl)-1H,2H,3H-pyrido[3,4-b][1,4]oxazin-1-yl]pyridin-2-amine

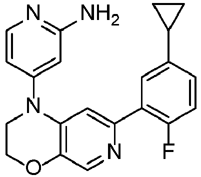

The title compound was prepared following the procedure used for the synthesis of Intermediate 41, starting from Intermediate 24 (82 mg, 1.0 eq.). RM was worked-up as for Example 3 to afford Example 12 (22 mg, Y = 29%) as a beige powder. ¹H NMR (400 MHz, DMSO-*d*₆) δ 8.17 (s, 1H), 7.88 (d, *J* = 5.6 Hz, 1H), 7.59 (dd, *J* = 7.7, 2.4 Hz, 1H), 7.47 (d, *J* = 1.3 Hz, 1H), 7.19 - 7.02 (m, 2H), 6.49 (dd, *J* = 5.7, 2.0 Hz, 1H), 6.33 (d, *J* = 2.0 Hz, 1H), 5.96 (s, 2H), 4.35 (dd, *J* = 5.0, 3.6 Hz, 2H), 3.78 (t, *J* = 4.4 Hz, 2H), 1.98 (ddd, *J* = 13.4, 8.5, 5.1 Hz, 1H), 1.02 - 0.86 (m, 2H), 0.72 - 0.58 (m, 2H). LCMS (Method B): 220 nm: 96.65%, 254 nm: 97.36%; r.t.= 1.870 min; m/z(+)= 363.04.

### Example 13: 4-{7-[2-Fluoro-5-(propan-2-yl)phenyl]-1H,2H,3H-pyrido[3,4-b][1,4]oxazin-1-yl}pyridin-2-amine

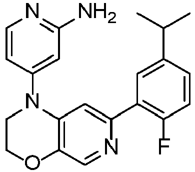

The title compound was prepared following the procedure used for the synthesis of Intermediate 41, starting from Intermediate 26 (55 mg, 1.0 eq.). RM was worked-up as for Example 3 to afford Example 13 (27 mg, Y = 53%) as a beige powder. ¹H NMR (400 MHz, DMSO-*d*₆) δ 8.18 (s, 1H), 7.88 (d, *J* = 5.7 Hz, 1H), 7.74 (dd, *J* = 7.7, 2.5 Hz, 1H), 7.49 (d, *J* = 1.2 Hz, 1H), 7.26 (ddd, *J* = 7.5, 4.7, 2.4 Hz, 1H), 7.15 (dd, *J* = 11.4, 8.5 Hz, 1H), 6.49 (dd, *J* = 5.7, 2.1 Hz, 1H), 6.34 (d, *J* = 2.0 Hz, 1H), 5.96 (s, 2H), 4.35 (t, *J* = 4.3 Hz, 2H), 3.79 (t, *J* = 4.4 Hz, 2H), 2.94 (p, *J* = 6.9 Hz, 1H), 1.21 (d, *J* = 6.9 Hz, 6H). LCMS (Method B): 220 nm: 95.95%, 254 nm: 96.34%; r.t.= 1.997 min; m/z(+)= 365.03.

### Example 14: N-{4-[7-(5-chloro-2-fluorophenyl)-1H,2H,3H-pyrido[3,4-b][1,4]oxazin-1-yl]pyridin-2-yl}-3-(4-methylpiperazin-1-yl)propanamide

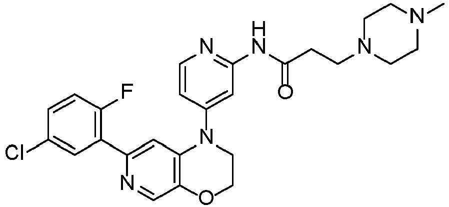

The title compound was prepared following the procedure used for the synthesis of Example 8, starting from freshly-prepared Intermediate 4 (130 mg, 1.0 eq.) and using N-methylpiperazine (1.7 eq.). Purification by preparative HPLC using ACN:water gradient with 0.1% of formic acid yielded a single formic salt of Example 14 (63 mg, Y = 40%) as a beige powder. ¹H NMR (400 MHz, Chloroform-d) δ 8.32 (s, 1H), 8.16 - 8.08 (m, 2H), 7.95 (dd, *J* = 6.9, 2.7 Hz, 1H), 7.63 (d, *J* = 1.2 Hz, 1H), 7.26 (dd, *J* = 12.9, 2.8 Hz, 1H), 7.09 (dd, *J* = 5.9, 2.2 Hz, 1H), 7.03 (dd, *J =* 10.7, 8.7 Hz, 1H), 4.45 - 4.37 (m, 2H), 3.90 (t, *J* = 4.5 Hz, 2H), 3.5 - 3.0 (m, 8H), 2.94 (t, *J* = 6.1 Hz, 2H), 2.80 (s, 3H), 2.64 (d, *J* = 6.3 Hz, 2H). LCMS (Method J): 254 nm: 98.51%, 220 nm: 98.02%, 240 nm: 98.52%; r.t.= 3.313 min; m/z(+)= 511.28.

### Example 15: 7-(5-Chloro-2-fluorophenyl)-1-{1H-pyrazolo[3,4-b]pyridin-4-yl}-1H,2H,3H-pyrido[3,4-b][1,4]oxazine

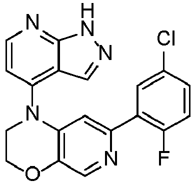

The title compound was prepared following the procedure used for the synthesis of Intermediate 3, starting from Intermediate 2 (60 mg, 1.0 eq.) and using Intermediate 27 (1.15 eq.). Purification by silica gel column chromatography eluting with gradient from 0 to 100% of EtOAc in hexane +2.5%TEA yielded pure SEM-protected Example 15 (96 mg, Y = 83%). The protected product was dissolved in 2.5 mL of DCM and 1.25 mL of TFA was added to the solution. The mixture was stirred for 1.5 h at RT. RM was dried under reduced pressure and the residue was partitioned between 5 mL DCM and 5 mL sat. aq. NaHCO₃. The aqueous layer was extracted with DCM (4 × 5 mL). Combined organics were dried over Na₂SO₄, filtered and volatiles removed under reduced pressure. The residue was suspended in MeOH and acidified using formic acid. Purification by preparative HPLC using ACN:water gradient with 0.1% of formic acid yielded Example 15 (36 mg, Y = 46%) as a white powder. ¹H NMR (400 MHz, DMSO-*d*₆) δ 13.71 (s, 1H), 8.45 (d, *J* = 5.2 Hz, 1H), 8.30 (s, 1H), 8.05 (s, 1H), 7.91 (dd, *J* = 6.8, 2.8 Hz, 1H), 7.51 (d, *J* = 0.9 Hz, 1H), 7.44 (ddd, *J* = 8.8, 4.1, 2.8 Hz, 1H), 7.28 (dd, *J* = 11.1, 8.8 Hz, 1H), 7.12 (d, *J* = 5.2 Hz, 1H), 4.46 (dd, *J* = 5.2, 3.5 Hz, 2H), 4.13 (t, *J* = 4.3 Hz, 2H). LCMS (Method F): 220 nm: 99.41%, 254 nm: 99.79%, 205 nm: 99.08%; r.t.= 2.323 min; m/z(+)= 381.91.

### Example 16: 7-(5-Chloro-2-fluorophenyl)-1-{1H-pyrrolo[2,3-b]pyridin-4-yl}-1H,2H,3H-pyrido[3,4-b][1,4]oxazine

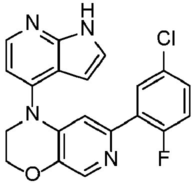

The title compound was prepared following the procedure used for the synthesis of Intermediate 3, starting from Intermediate 2 (50 mg, 1.0 eq.) and using Intermediate 28 (1.15 eq.). Purification by pTLC on silica gel, using hexane:AcOEt:TEA (8:2:1), eluted two times, yielded the SEM-protected Example 16 (75 mg, Y= 78%). The product was dissolved in DCM (5.66 mL) and 0.5 mL of TFA was added to the stirred solution. After 24 h, the RM was dried under reduced pressure and the residue was suspended in 4.5 mL of dioxane. 4.5 mL of 25% NH_{3(aq.)} was added to the mixture and it was stirred for 2 h at RT. RM was evaporated to dryness and the residue was partitioned between 5 mL of water and 5 mL of DCM. The aqueous layer was extracted with DCM (3 × 5 mL). Combined organics were dried over Na₂SO₄, filtered and volatiles removed under reduced pressure. Trituration with MeOH yielded Example 16 (15 mg, Y= 32%) as a white powder. ¹H NMR (400 MHz, DMSO-*d*₆) δ 11.81 (s, 1H), 8.29 - 8.17 (m, 2H), 7.88 (dd, *J* = 6.8, 2.8 Hz, 1H), 7.49 - 7.37 (m, 2H), 7.28 - 7.16 (m, 2H), 7.07 (d, *J* = 5.2 Hz, 1H), 6.31 (d, *J* = 3.5 Hz, 1H), 4.45 (dd, *J* = 5.2, 3.4 Hz, 2H), 4.03 (q, *J* = 4.2, 3.1 Hz, 2H). LCMS (Method B): 220 nm: 98.95%, 254 nm: 98.94%; r.t.= 2.140 min; m/z(+)= 380.90.

### Example 17: 7-(5-Chloro-2-fluorophenyl)-1-{3H-imidazo[4,5-b]pyridin-7-yl}-1H,2H,3H-pyrido[3,4-b][1,4]oxazine

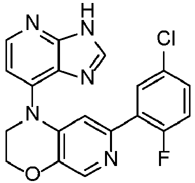

The title compound was prepared following the procedure used for the synthesis of Intermediate 3, starting from Intermediate 2 (40 mg, 1.0 eq.) and using Intermediate 29 (1.15 eq.). Purification by pTLC on silica gel, using hexane:AcOEt:TEA (8:2:1), eluted twice, yielded SEM-protected Example 17 (55 mg, Y = 71%) as a single regioisomer. The product was dissolved in DCM (9.2 mL) and 2.7 mL of TFA was added. After 7 h, volatiles were removed and the residue was partitioned between 7 mL DCM and 5 mL sat. aq. NaHCO₃. The aqueous layer was extracted with DCM (3 × 7 mL). Combined organics were dried over Na₂SO₄, filtered and volatiles removed under reduced pressure. Trituration with ACN yielded Example 17 (20 mg, Y= 49%) as a brown powder. ¹H NMR (400 MHz, DMSO-*d*₆) δ 12.92 (s, 1H), 8.38 (s, 1H), 8.20 (s, 1H), 8.09 (d, *J* = 8.5 Hz, 1H), 7.92 (dd, *J* = 6.8, 2.8 Hz, 1H), 7.77 (s, 1H), 7.44 (ddd, *J* = 8.8, 4.1, 2.8 Hz, 1H), 7.29 (dt, *J* = 8.8, 5.7 Hz, 2H), 4.42 (dd, *J* = 5.1, 3.6 Hz, 2H), 4.06 - 3.99 (m, 2H). LCMS (Method E): 220 nm: 98.22%, 254 nm: 98.38%, 205 nm: 98.57%; r.t.= 2.937 min; m/z(+)= 381.89.

### Example 18: 3-[Bis(2-hydroxyethyl)amino]-N-{4-[7-(5-chloro-2-fluorophenyl)-1H,2H,3H-pyrido[3,4-b][1,4]oxazin-1-yl]pyridin-2-yl}propanamide

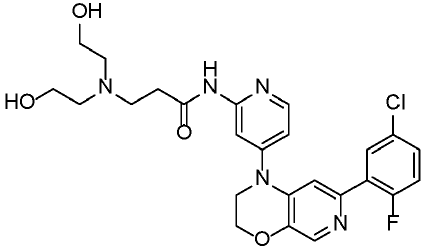

The title compound was prepared following the procedure used for the synthesis of Example 8, starting from freshly-prepared Intermediate 4 (500 mg, 1.0 eq.) and using diethanolamine (2.1 eq.). Purification by silica gel column chromatography eluting with gradient from 2% to 5% of MeOH in DCM yielded Example 18 (225 mg, Y = 46%) as a grey powder. ¹H NMR (400 MHz, DMSO-*d*₆) δ 10.82 (s, 1H), 8.25 (t, *J* = 2.8 Hz, 2H), 8.12 (d, *J* = 2.2 Hz, 1H), 7.93 (dd, *J* = 6.8, 2.8 Hz, 1H), 7.62 (s, 1H), 7.46 (ddd, *J* = 8.8, 4.2, 2.8 Hz, 1H), 7.31 (dd, *J* = 11.0, 8.8 Hz, 1H), 7.07 (dd, *J* = 5.7, 2.2 Hz, 1H), 4.40 (q, *J* = 4.3, 3.2 Hz, 4H), 3.88 (t, *J* = 4.3 Hz, 2H), 3.45 (q, *J* = 6.0 Hz, 4H), 2.82 (t, *J* = 6.8 Hz, 2H), 2.57 (t, *J* = 6.3 Hz, 4H). LCMS (Method A): 220 nm: 94.57%, 240 nm: 96.21%, 254 nm: 97.43%; r.t.= 1.640; m/z(+)= 516.12.

### Example 19: N-{4-[7-(5-chloro-2-fluorophenyl)-1H,2H,3H-pyrido[3,4-b][1,4]oxazin-1-yl]pyridin-2-yl}-3-[methyl(oxetan-3-yl)amino]propanamide

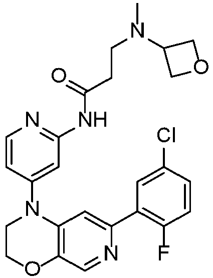

The title compound was prepared following the procedure used for the synthesis of Example 8, starting from freshly-prepared Intermediate 4 (123 mg, 1.0 eq.) and using *N*-methyl oxetane amine (3.4 eq.). Purification by preparative HPLC using ACN:water gradient with 0.1% of formic acid yielded Example 19 (27 mg, Y = 18%) as a white powder. ¹H NMR (400 MHz, DMSO-*d*₆) δ 10.61 (s, 1H), 8.30 - 8.21 (m, 2H), 8.14 (d, *J* = 2.2 Hz, 1H), 7.92 (dd, *J* = 6.8, 2.8 Hz, 1H), 7.62 (s, 1H), 7.45 (ddd, *J* = 8.7, 4.1, 2.8 Hz, 1H), 7.29 (dd, *J* = 11.0, 8.8 Hz, 1H), 7.06 (dd, *J* = 5.7, 2.2 Hz, 1H), 4.49 (t, *J* = 6.5 Hz, 2H), 4.38 (q, *J* = 6.2, 5.6 Hz, 4H), 3.88 (t, *J* = 4.4 Hz, 2H), 3.50 (q, *J* = 6.4 Hz, 1H), 2.06 (s, 3H). LCMS (Method G): 220 nm: 96.68%, 240 nm: 97.02%, 254 nm: 96.48%, r.t.= 2.410 min; m/z(+) = 497.79.

### Example 20: N-{4-[7-(5-chloro-2-fluorophenyl)-1H,2H,3H-pyrido[3,4-b][1,4]oxazin-1-yl]pyridin-2-yl}-3-[(2-hydroxyethyl)(methyl)amino]propanamide

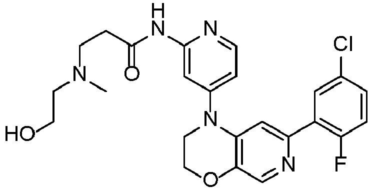

The title compound was prepared following the procedure used for the synthesis of Example 8, starting from freshly-prepared Intermediate 4 (123 mg, 1.0 eq.) and using *N*-methylethanolamine (3.4 eq.). Purification by preparative HPLC using ACN:water gradient with 0.1% of formic acid yielded Example 20 (37 mg, Y = 40%) as a white powder. ¹H NMR (400 MHz, DMSO-*d*₆) δ 10.80 (s, 1H), 8.27 - 8.21 (m, 2H), 8.12 (d, *J* = 2.2 Hz, 1H), 7.92 (dd, *J* = 6.8, 2.8 Hz, 1H), 7.61 (s, 1H), 7.45 (ddd, *J* = 8.8, 4.1, 2.8 Hz, 1H), 7.30 (dd, *J* = 11.0, 8.8 Hz, 1H), 7.06 (dd, *J* = 5.7, 2.2 Hz, 1H), 4.42 - 4.36 (m, 3H), 3.87 (t, *J* = 4.3 Hz, 2H), 3.48 (br. s, 2H), 2.67 (d, *J* = 13.5 Hz, 2H), 2.53 - 2.50 (m, 2H), 2.44 (t, *J* = 6.3 Hz, 2H), 2.21 (s, 3H). LCMS (Method D): 205 nm: 95.53%, 220 nm: 96.27%, 254 nm: 96.53%; r.t.= 3.030 min; m/z(+) = 486.00.

### Example 21: 3-[Bis(oxetan-3-yl)amino]-N-{4-[7-(5-chloro-2-fluorophenyl)-1H,2H,3H-pyrido [3,4-b] [1,4] oxazin-1-yl] pyridin-2-yl}propanamide

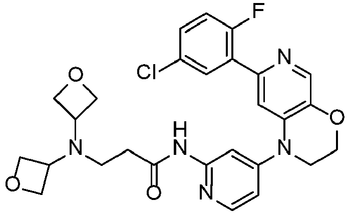

The title compound was prepared following the procedure used for the synthesis of Example 8, starting from freshly-prepared Intermediate 4 (123 mg, 1.0 eq.) and *N*-(oxetan-3-yl)oxetan-3-amine (3.4 eq.) and *p*-toluenesulfonic acid monohydrate (0.25 eq.). Purification by preparative HPLC using ACN:water gradient with 0.1% of formic acid yielded Example 21 (16 mg, Y = 10%) as a white powder. ¹H NMR (400 MHz, DMSO-*d*₆) δ 10.59 (s, 1H), 8.30 - 8.21 (m, 2H), 8.12 (d, *J* = 2.1 Hz, 1H), 7.93 (dd, *J* = 6.8, 2.8 Hz, 1H), 7.62 (s, 1H), 7.45 (ddd, *J* = 8.8, 4.2, 2.8 Hz, 1H), 7.30 (dd, *J* = 11.0, 8.8 Hz, 1H), 7.07 (dd, *J* = 5.7, 2.2 Hz, 1H), 4.46 (dd, *J* = 7.1, 1.7 Hz, 8H), 4.39 (t, *J* = 4.3 Hz, 2H), 4.00 (p, *J* = 7.2 Hz, 2H), 3.87 (t, *J* = 4.4 Hz, 2H), 2.83 (t, *J* = 7.0 Hz, 2H), 2.44 (t, *J* = 6.9 Hz, 2H). LCMS (Method B): 220 nm: 95.22%, 240 nm: 96.08%, 254 nm: 96.07%; r.t.= 1.727 min; m/z(+)= 513.72.

### Example 22: N-{4-[7-(5-chloro-2-fluorophenyl)-1H,2H,3H-pyrido[3,4-b][1,4]oxazin-1-yl]pyridin-2-yl}-3-(4-methyl-3-oxopiperazin-1-yl)propanamide

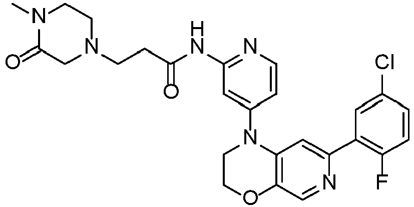

The title compound was prepared following the procedure used for the synthesis of Example 8, starting from freshly-prepared Intermediate 4 (135 mg, 1.0 eq.) and using 1-methylpiperazin-2-one (1.7 eq). Purification by pTLC on silica gel, using 8% MeOH in DCM as eluent, followed by trituration with MeOH/diethyl ether yielded Example 22 (50 mg, Y = 13%) as a white powder. ¹H NMR (400 MHz, DMSO-*d*₆) δ 10.61 (s, 1H), 8.31 - 8.21 (m, 2H), 8.14 (d, *J* = 2.1 Hz, 1H), 7.93 (dd, *J* = 6.8, 2.8 Hz, 1H), 7.61 (s, 1H), 7.50 - 7.42 (m, 1H), 7.31 (dd, *J* = 10.9, 8.8 Hz, 1H), 7.07 (dd, *J* = 5.7, 2.3 Hz, 1H), 4.45 - 4.32 (m, 2H), 3.87 (t, *J* = 4.3 Hz, 2H), 3.23 (t, *J* = 5.5 Hz, 2H), 3.01 (s, 2H), 2.79 (s, 3H), 2.66 (d, *J* = 2.7 Hz, 4H), 2.57 (d, *J* = 6.5 Hz, 2H). LCMS (Method E): 220 nm: 96.46%, 235 nm: 97.00%, 254 nm: 96.51%; r.t.= 2.910 min; m/z(+) = 524.96.

### Example 23: Methyl 2-{[2-({4-[7-(5-chloro-2-fluorophenyl)-1H,2H,3H-pyrido[3,4-b][1,4]oxazin-1-yl]pyridin-2-yl}carbamoyl)ethyl](methyl)amino}acetate

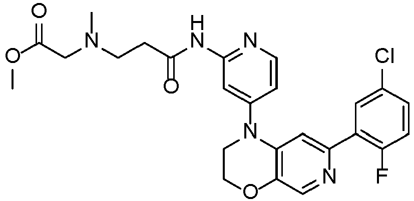

The title compound was prepared following the procedure used for the synthesis of Example 8, starting from freshly-prepared Intermediate 4 (135 mg, 1.0 eq.) and using sarcosine methyl ester hydrochloride (1.7 eq.). Purification by pTLC on silica gel, using 5% MeOH in DCM as eluent yielded Example 23 (55 mg, Y= 36%) as a green powder. ¹H NMR (400 MHz, DMSO-*d*₆) δ 10.60 (s, 1H), 8.28 - 8.21 (m, 2H), 8.13 (d, *J* = 2.1 Hz, 1H), 7.92 (dd, *J* = 6.8, 2.8 Hz, 1H), 7.61 (s, 1H), 7.45 (dt, *J* = 8.8, 3.6 Hz, 1H), 7.30 (dd, *J* = 11.0, 8.8 Hz, 1H), 7.06 (dd, *J* = 5.7, 2.2 Hz, 1H), 4.39 (t, *J* = 4.3 Hz, 2H), 3.87 (t, *J* = 4.2 Hz, 2H), 3.59 (s, 3H), 3.31 (s, 2H), 2.79 (t, *J* = 6.9 Hz, 2H), 2.56 - 2.53 (m, 2H), 2.29 (s, 3H). LCMS (Method B): 220 nm: 95.22%, 240 nm: 96.08%, 254 nm: 96.07%; r.t.= 1.727 min; m/z(+)= 513.72.

### Example 24: N-{4-[7-(5-chloro-2-fluorophenyl)-1H,2H,3H-pyrido[3,4-b][1,4]oxazin-1-yl]pyridin-2-yl}-3-methanesulfonamidopropanamide

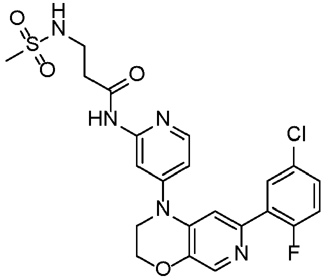

Intermediate 30 (20 mg, 1.0 eq.) was dissolved in dry DCM (2 mL) and TEA (5.0 eq.) was added. RM was cooled to 0 °C and mesyl chloride (1.2 eq.) was added. RM was warmed to RT and stirred for 1 h. Volatiles were removed. Purification by pTLC on silica gel using 8% MeOH in DCM as eluent yielded Example 24 (8.5 mg, Y = 43%) as a beige powder. ¹H NMR (400 MHz, DMSO-*d*₆) δ 8.29 - 8.21 (m, 2H), 8.13 (d, *J* = 2.1 Hz, 1H), 7.93 (dd, *J* = 6.9, 2.8 Hz, 1H), 7.61 (s, 1H), 7.46 (ddd, *J* = 8.8, 4.2, 2.8 Hz, 1H), 7.31 (dd, *J* = 11.0, 8.8 Hz, 1H), 7.07 (dd, *J* = 5.7, 2.2 Hz, 1H), 4.43 - 4.36 (m, 2H), 3.87 (t, *J* = 4.3 Hz, 2H), 3.22 (t, *J* = 6.9 Hz, 2H), 2.85 (s, 3H), 2.61 (t, *J* = 6.9 Hz, 2H). LCMS (Method B): 220 nm: 96.98%, 235 nm: 97.57%, 254 nm: 97.95%; r.t.= 2.190 min; m/z(+)= 505.97.

### Example 25: N-{4-[7-(5-chloro-2-fluorophenyl)-1H,2H,3H-pyrido[3,4-b][1,4]oxazin-1-yl]pyridin-2-yl}-3-(4-methyl-2-oxopiperazin-1-yl)propanamide

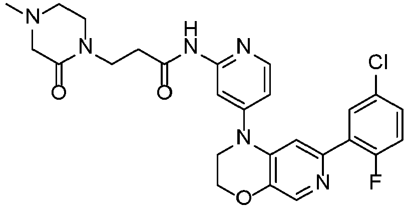

The title compound was prepared following the procedure used for the synthesis of Example 8, starting from freshly-prepared Intermediate 4 (100 mg, 1.0 eq.) and using 1-methylpiperazin-3-one (1.1 eq.) and NaOH (0.3 eq.). Purification by pTLC on silica gel using 8% MeOH in DCM as eluent yielded Example 25 (30 mg, Y = 29%) as a beige powder. ¹H NMR (400 MHz, DMSO-*d*₆) δ 10.61 (s, 1H), 8.31 - 8.20 (m, 2H), 8.10 (s, 1H), 7.93 (dd, *J* = 6.8, 2.8 Hz, 1H), 7.62 (s, 1H), 7.46 (ddd, *J* = 8.8, 4.1, 2.8 Hz, 1H), 7.30 (dd, *J* = 11.0, 8.8 Hz, 1H), 7.07 (dd, *J* = 5.7, 2.2 Hz, 1H), 4.39 (t, *J* = 4.3 Hz, 2H), 3.88 (t, *J* = 4.4 Hz, 2H), 3.52 (t, *J* = 7.1 Hz, 2H), 3.29 (t, *J* = 5.5 Hz, 2H), 2.87 (s, 2H), 2.63 (q, *J* = 6.8 Hz, 2H), 2.53 - 2.50 (m, 2H), 2.18 (s, 3H). LCMS (Method A): 205 nm: 96.90%, 220 nm: 96.92%, 254 nm: 97.47%; r.t =1.717 min; m/z(+) = 525.15.

### Example 26: N-{4-[7-(5-chloro-2-fluorophenyl)-1H,2H,3H-pyrido[3,4-b][1,4]oxazin-1-yl]pyridin-2-yl}-3-[(2-methanesulfonylethyl)amino]propanamide

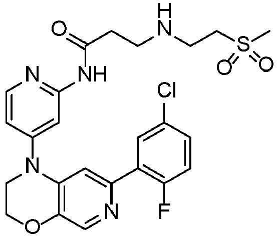

The title compound was prepared following the procedure used for the synthesis of Example 24, starting from Intermediate 30 (80 mg, 1.0 eq.) and using methyl vinyl sulfone (1.1 eq.). Purification by pTLC on silica gel using 10% MeOH in DCM as eluent (eluted twice) yielded Example 26 (13 mg, Y= 15%) as a beige powder. ¹H NMR (400 MHz, DMSO-*d*₆) δ 10.64 (s, 1H), 8.25 (d, *J* = 5.5 Hz, 2H), 8.13 (d, *J* = 2.1 Hz, 1H), 7.93 (dd, *J* = 6.8, 2.8 Hz, 1H), 7.61 (s, 1H), 7.46 (ddd, *J* = 8.7, 4.1, 2.8 Hz, 1H), 7.32 (dd, *J* = 11.0, 8.8 Hz, 1H), 7.08 (dd, *J* = 5.7, 2.2 Hz, 1H), 4.40 (t, *J* = 4.3 Hz, 2H), 3.88 (t, *J* = 4.3 Hz, 2H), 3.20 (q, *J* = 6.0, 5.4 Hz, 2H), 3.00 - 2.98 (m, 4H), 2.92 (d, *J* = 5.4 Hz, 2H), 2.81 (d, *J* = 6.5 Hz, 2H), 2.54 - 2.52 (m, 2H), 2.08 - 1.97 (br. s, 1H). LCMS (Method E): 205 nm: 99.67%, 220 nm: 99.23%, 254 nm: 98.66%; r.t.= 2.850 min; m/z(+) = 534.06.

### Example 27: N-{4-[7-(5-chloro-2-fluorophenyl)-1H,2H,3H-pyrido[3,4-b][1,4]oxazin-1-yl]pyridin-2-yl}-3-[(2-methanesulfonylethyl)(methyl)amino]propanamide

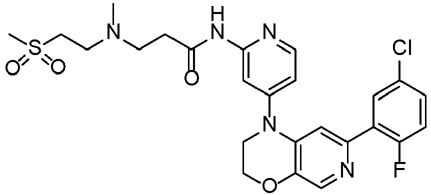

The title compound was prepared following the procedure used for the synthesis of Example 8, starting from freshly-prepared Intermediate 4 (146 mg, 1.0 eq.) and using 2-(methylamino)-1-(methylsulfonyl)ethane (2.2. eq.). Purification by pTLC on silica gel using 5% MeOH in DCM yielded Example 27 (50 mg, Y = 41%) as a grey powder. ¹H NMR (400 MHz, DMSO-*d*₆) δ 10.61 (s, 1H), 8.34 - 8.23 (m, 2H), 8.12 (d, *J* = 2.2 Hz, 1H), 7.94 (dd, *J* = 6.8, 2.8 Hz, 1H), 7.62 (s, 1H), 7.51 - 7.43 (m, 1H), 7.31 (dd, *J* = 10.9, 8.8 Hz, 1H), 7.08 (dd, *J* = 5.7, 2.2 Hz, 1H), 4.40 (t, *J* = 4.3 Hz, 2H), 3.88 (t, *J* = 4.2 Hz, 2H), 3.25 (t, *J* = 6.9 Hz, 2H), 2.95 (s, 3H), 2.77 (t, *J* = 7.0 Hz, 2H), 2.69 (m, 2H), 2.54 (m, 2H), 2.22 (s, 3H). LCMS (Method E): 254 nm: 98.81%, 220 nm: 99.18%; 240 nm (max. abs.): 99.07%, r.t.= 2.880 min; m/z(+)= 547.15.

### Example 28: N-{4-[7-(5-chloro-2-fluorophenyl)-1H,2H,3H-pyrido[3,4-b][1,4]oxazin-1-yl]pyridin-2-yl}-3-[4-(2-hydroxyethyl)piperazin-1-yl]propanamide

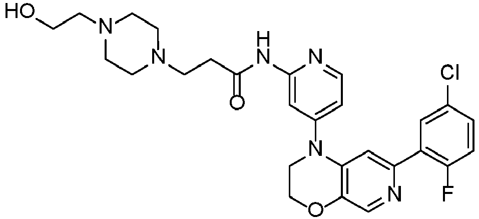

The title compound was prepared following the procedure used for the synthesis of Example 8, starting from freshly-prepared Intermediate 4 (146 mg, 1.0 eq.) and using 1-(2-hydroxyethyl)piperazine (2.2 eq.). Purification by pTLC on silica gel using 5% MeOH in DCM yielded the product which was dissolved in ACN and precipitated from water. Obtained Example 28 (20 mg, Y = 16%) as a beige powder. ¹H NMR (400 MHz, DMSO-*d*₆) δ 10.75 (s, 1H), 8.31 - 8.22 (m, 2H), 8.14 (d, *J* = 2.1 Hz, 1H), 7.93 (dd, *J* = 6.8, 2.8 Hz, 1H), 7.61 (s, 1H), 7.46 (ddd, *J* = 8.7, 4.1, 2.8 Hz, 1H), 7.31 (dd, *J* = 11.0, 8.8 Hz, 1H), 7.07 (dd, *J* = 5.7, 2.2 Hz, 1H), 4.44 - 4.31 (m, 2H), 3.88 (t, *J* = 4.3 Hz, 2H), 3.48 (t, *J* = 6.5 Hz, 2H), 2.59 (d, *J* = 6.1 Hz, 2H), 2.45 - 2.32 (m, 10H). LCMS (Method E): 254 nm: 97.03%, 220 nm: 97.68%; 205 nm (max. abs.): 97.15%, r.t.= 2.770 min; m/z(+)= 541.20.

### Example 29: N-{4-[7-(5-chloro-2-fluorophenyl)-1H,2H,3H-pyrido[3,4-b][1,4]oxazin-1-yl]pyridin-2-yl}-3-[(2-methanesulfonamidoethyl)(methyl)amino]propanamide

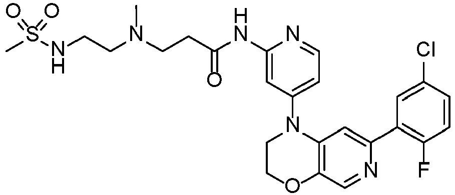

The title compound was prepared following the procedure used for the synthesis of Example 8, starting from freshly-prepared Intermediate 4 (146 mg, 1.0 eq.) and using 2-(methylamino)-1-(methylsulfonylamide)ethane hydrochloride (2.2 eq.). Purification by pTLC on silica gel using 5% MeOH in DCM yielded the product which was dissolved in ACN and precipitated from water. Obtained Example 29 (36 mg, Y = 18%) as a grey powder. ¹H NMR (400 MHz, DMSO-*d*₆) δ 10.78 (s, 1H), 8.29 - 8.22 (m, 2H), 8.13 (d, *J* = 2.2 Hz, 1H), 7.93 (dd, *J* = 6.8, 2.8 Hz, 1H), 7.62 (s, 1H), 7.46 (ddd, *J* = 8.8, 4.2, 2.8 Hz, 1H), 7.31 (dd, *J* = 11.0, 8.8 Hz, 1H), 7.08 (dd, *J* = 5.7, 2.2 Hz, 1H), 6.79 (s, 1H), 4.40 (dd, *J* = 5.1, 3.5 Hz, 2H), 3.88 (t, *J* = 4.4 Hz, 2H), 3.05 (d, *J* = 5.4 Hz, 2H), 2.89 (s, 3H), 2.68 (m, 2H), signals from ethylene protons overlayed by DMSO, 2.22 (s, 3H). LCMS (Method E): 254 nm: 98.13%, 220 nm: 98.44%; 205 nm (max. abs.): 98.51%, r.t.= 2.867 min; m/z(+)= 563.13.

### Example 30: N-{4-[7-(5-chloro-2-fluorophenyl)-1H,2H,3H-pyrido[3,4-b][1,4]oxazin-1-yl]pyridin-2-yl}-3-[4-(2-methanesulfonamidoethyl)piperazin-1-yl]propanamide

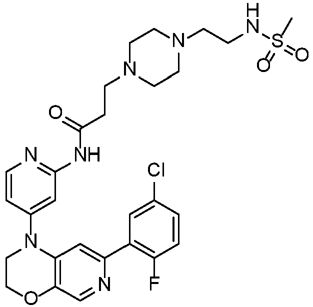

The title compound was prepared following the procedure used for the synthesis of Example 8, starting from freshly-prepared Intermediate 4 (72 mg, 1.0 eq.), using Intermediate 32 (3.6 eq.) and a 1:1 mixture of THF/DCM (6 mL) as solvent. Purification by pTLC on silica gel eluting with 15% MeOH in DCM, followed by an additional purification by pTLC using hexane/THF/MeOH 4:4:3 (v:v:v) yielded Example 30 (32 mg, Y = 29%) as a white powder. ¹H NMR (400 MHz, DMSO-*d*₆) δ 10.73 (s, 1H), 8.32 - 8.19 (m, 2H), 8.13 (d, *J* = 2.1 Hz, 1H), 7.93 (dd, *J* = 6.9, 2.8 Hz, 1H), 7.61 (s, 1H), 7.46 (ddd, *J* = 8.7, 4.1, 2.8 Hz, 1H), 7.30 (dd, *J* = 11.0, 8.8 Hz, 1H), 7.06 (dd, *J* = 5.7, 2.2 Hz, 1H), 6.84 (s, 1H), 4.39 (dd, *J* = 5.1, 3.4 Hz, 2H), 3.87 (t, *J* = 4.3 Hz, 2H), 3.03 (t, *J* = 6.8 Hz, 2H), 2.91 (s, 3H), 2.60 (t, *J* = 6.6 Hz, 2H), 2.39 (m, partially overlayed by DMSO, 12H). LCMS (Method K): 254 nm: 97.34%, 220 nm: 97.35%; 240 nm (max. abs.): 97.80%, r.t.= 1.753 min; m/z(+)= 618.01.

### Example 31: 3-{4-[2-({4-[7-(5-Chloro-2-fluorophenyl)-1H,2H,3H-pyrido[3,4-b][1,4]oxazin-1-yl]pyridin-2-yllcarbamoyl)ethyl]piperazin-1-yl}-N-methylpropanamide

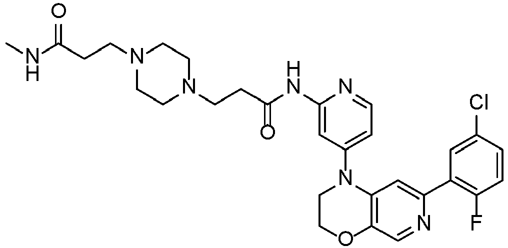

The title compound was prepared following the procedure used for the synthesis of Example 8, starting from freshly-prepared Intermediate 4 (65 mg, 1.0 eq.) and using Intermediate 33 (2.2 eq.) Purification by preparative HPLC using ACN:water gradient with 0.1% of formic acid, followed by neutralization using sat. aq. NaHCO₃ yielded Example 31 (17 mg, Y = 31%) as white powder. ¹H NMR (400 MHz, DMSO-*d*₆) δ 10.74 (s, 1H), 8.33 - 8.19 (m, 2H), 8.14 (d, J = 2.2 Hz, 1H), 7.93 (dd, J = 6.8, 2.8 Hz, 1H), 7.79 (d, J = 5.0 Hz, 1H), 7.61 (s, 1H), 7.46 (dt, J = 8.9, 3.6 Hz, 1H), 7.31 (dd, J = 10.9, 8.8 Hz, 1H), 7.07 (dd, J = 5.7, 2.2 Hz, 1H), 4.40 (t, J = 4.3 Hz, 2H), 3.88 (t, J = 4.4 Hz, 2H), 2.59 (d, J = 6.1 Hz, 2H), 2.56 (m, J = 4.7 Hz, 7H), 2.38 (br s, 8H), 2.20 (t, J = 7.3 Hz, 2H). LCMS (Method B): 254 nm: 98.54%, 220 nm: 98.50%; 235 nm (max. abs.): 98.77%, r.t.= 1.600 min; m/z(+)= 582.26.

### Example 32: N-{4-[7-(5-chloro-2-fluorophenyl)-1H,2H,3H-pyrido[3,4-b][1,4]oxazin-1-yl]pyridin-2-yl}-3-(1-methylpiperidin-4-yl)propanamide

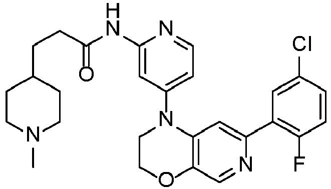

3-(1-Methylpiperidin-4-yl)propanoic acid hydrochloride (30 mg, 1.0 eq.) was dissolved in DMF (0.5 mL) and HATU (1.2 eq.) followed by DIPEA (4.0 eq.) were added. The RM was stirred for 30 min at RT and Example 3 (0.9 eq.), dissolved in DMF (0.2 mL) was added dropwise. The RM was stirred at 100 °C for 2 h. Volatiles were removed under reduced pressure and the residue was dissolved in DCM and washed with sat. aq. NaHCO₃ and water. Organics were dried over Na₂SO₄, filtered and solvent removed under reduced pressure. Purification by preparative HPLC using ACN:water gradient with 0.1% of formic acid, followed by neutralization using sat. aq. NaHCO₃ yielded Example 32 (5 mg, Y = 8%) as a beige powder. ¹H NMR (400 MHz, DMSO-*d*₆) δ 10.50 (s, 1H), 8.24 (d, *J* = 5.7 Hz, 2H), 8.14 (d, *J* = 2.1 Hz, 1H), 7.93 (dd, *J* = 6.8, 2.8 Hz, 1H), 7.61 (s, 1H), 7.46 (ddd, *J* = 8.8, 4.1, 2.8 Hz, 1H), 7.29 (dd, *J* = 11.0, 8.8 Hz, 1H), 7.05 (dd, *J* = 5.7, 2.2 Hz, 1H), 4.39 (t, *J* = 4.4 Hz, 2H), 3.87 (t, *J* = 4.4 Hz, 2H), 2.71 (d, *J* = 11.2 Hz, 2H), 2.39 (t, *J* = 7.5 Hz, 2H), 2.11 (s, 3H), 1.76 (t, *J* = 10.6 Hz, 2H), 1.60 (d, *J* = 9.9 Hz, 2H), 1.50 (q, *J* = 7.0 Hz, 2H), 1.33 - 0.99 (m, 4H). LCMS (Method B): 254 nm: 93.96%, 220 nm: 92.61%; 235 nm (max. abs.): 93.95%, r.t.= 1.763 min; m/z(-)= 507.98.

### Example 33: N-{4-[7-(5-cyclopropyl-2-fluorophenyl)-1H,2H,3H-pyrido[3,4-b][1,4]oxazin-1-yl]pyridin-2-yl}-3-(4-methylpiperazin-1-yl)propanamide

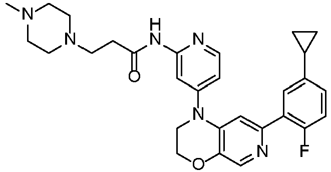

Following the procedure used for the synthesis of Intermediate 4, starting from Example 12 (50 mg, 1.0 eq.), the corresponding acrylamide was prepared and reacted with N-methylpiperazine (2.0 eq.) according to the procedure described for the synthesis of Example 8. Purification by pTLC on silica gel eluting with DCM:MeOH (9:1) followed by trituration in ACN yielded Example 33 (20 mg, Y = 28%) as white powder. ¹H NMR (400 MHz, DMSO-*d*₆) δ 10.73 (s, 1H), 8.27 - 8.19 (m, 2H), 8.13 (d, *J* = 2.2 Hz, 1H), 7.62 - 7.55 (m, 1H), 7.53 (s, 1H), 7.14 - 7.00 (m, 3H), 4.38 (t, *J* = 4.3 Hz, 2H), 3.86 (t, *J* = 4.4 Hz, 2H), 2.58 (d, *J* = 6.0 Hz, 2H), 2.54 - 2.21 (br s, 10H), 2.13 (s, 3H), 1.98 (tt, *J* = 8.5, 5.1 Hz, 1H), 0.98 - 0.90 (m, 2H), 0.70 - 0.62 (m, 2H). LCMS (Method B): 254 nm: 95.75%, 220 nm: 96.94%; 235 nm (max. abs.): 96.85%, r.t.= 1.577 min; m/z(-)= 517.26.

### Example 34: N-(4-{7-[2-fluoro-5-(propan-2-yl)phenyl]-1H,2H,3H-pyrido[3,4-b][1,4]oxazin-1-yl}pyridin-2-yl)-3-(4-methylpiperazin-1-yl)propanamide

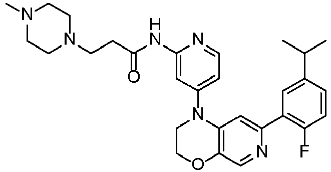

Following the procedure used for the synthesis of Intermediate 4, starting from Example 13 (65 mg, 1.0 eq.), the corresponding acrylamide was prepared and reacted with *N*-methylpiperazine (2.5 eq.) according to the procedure described for the synthesis of Example 8. Purification by pTLC on silica gel eluting with DCM:MeOH (9:1) followed by trituration in ACN and MeOH yielded Example 34 (20 mg, Y = 28%) as white powder. ¹H NMR (400 MHz, DMSO-*d*₆) δ 10.72 (s, 1H), 8.28 - 8.21 (m, 2H), 8.14 (d, *J* = 2.2 Hz, 1H), 7.74 (dd, *J* = 7.8, 2.4 Hz, 1H), 7.54 (s, 1H), 7.25 (ddd, *J* = 7.7, 4.7, 2.4 Hz, 1H), 7.12 (dd, *J* = 11.3, 8.4 Hz, 1H), 7.05 (dd, *J* = 5.7, 2.2 Hz, 1H), 4.38 (t, *J* = 4.3 Hz, 2H), 3.87 (t, *J* = 4.3 Hz, 2H), 2.94 (p, *J* = 7.1 Hz, 1H), 2.58 (d, *J* = 6.0 Hz, 2H), 2.54 - 2.21 (br s, 10H), 2.14 (s, 3H), 1.21 (d, *J* = 6.9 Hz, 6H). LCMS (Method B): 254 nm: 97.58%, 220 nm: 96.60%; 240 nm (max. abs.): 96.88%, r.t.= 1.677 min; m/z(-)= 519.28.

### Example 35: 3-{4-[2-({4-[7-(5-Chloro-2-fluorophenyl)-1H,2H,3H-pyrido[3,4-b][1,4]oxazin-1-yl]pyridin-2-yllcarbamoyl)ethyl]piperazin-1-yl)-N-(2-hydroxyethyl)propanamide

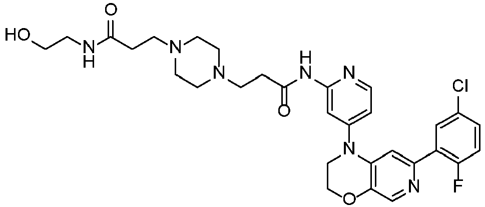

The title compound was prepared following the procedure used for the synthesis of Example 8, starting from freshly-prepared Intermediate 4 (100 mg, 1.0 eq.) and using Intermediate 34 (2.2 eq.). Purification by preparative HPLC using ACN:water gradient with 0.1% of formic acid, followed by neutralization using sat. aq. NaHCO₃ yielded Example 35 (13 mg, Y = 15%) as a white powder. ¹H NMR (400 MHz, Chloroform-*d*) δ 8.31 (s, 1H), 8.20 (d, *J* = 5.6 Hz, 1H), 8.08 (d, *J* = 2.1 Hz, 1H), 7.96 (dd, *J* = 6.8, 2.8 Hz, 1H), 7.64 (d, *J* = 1.3 Hz, 1H), 7.29 (s, 1H), 7.09 - 7.00 (m, 2H), 4.42 (t, *J* = 4.4 Hz, 2H), 3.93 - 3.87 (m, 2H), 3.80 - 3.73 (m, 2H), 3.45 (q, *J* = 5.2 Hz, 2H), 3.00 (br s, 6H), 2.88 (br s, 6H), 2.67 (br s, 2H), 2.62 (br s, 2H). LCMS (Method K): 254 nm: 92.60%, 220 nm: 93.23%; 205 nm (max. abs.): 92.81%, r.t.= 1.670 min; m/z(-)= 612.11.

### Example 36: 3-(4-{2-[Bis(2-hydroxyethyl)amino]ethyl}piperazin-1-yl)-N-{4-[7-(5-chloro-2-fluorophenyl)-1H,2H,3H-pyrido[3,4-b][1,4]oxazin-1-yl]pyridin-2-yl}propanamide

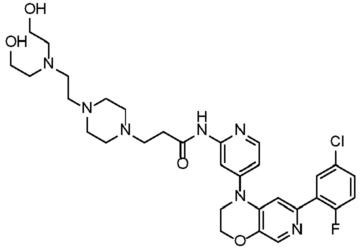

The title compound was prepared following the procedure used for the synthesis of Example 8, starting from freshly-prepared Intermediate 4 (100 mg, 1.0 eq.) and using Intermediate 35 (2.2 eq.). Purification by preparative HPLC using ACN:water gradient with 0.1% of formic acid, followed by neutralization using sat. aq. NaHCO₃ yielded Example 36 (9 mg, Y = 10%) as a beige powder. ¹H NMR (400 MHz, CDCl₃) δ 8.28 (s, 1H), 8.17 (d, J = 5.7 Hz, 1H), 8.05 (d, J = 2.2 Hz, 1H), 7.93 (dd, J = 6.8, 2.8 Hz, 1H), 7.61 (d, J = 1.3 Hz, 1H), 7.25 - 7.20 (m, 1H), 7.06 - 6.97 (m, 2H), 4.39 (dd, J = 5.1, 3.7 Hz, 2H), 3.91-3.83 (m, 2H), 3.65 (s, 4H), 2.68 (broad signal, 16H). LCMS (Method M): 254 nm: 92.34%, 220 nm: 93.53%, 240 nm (max abs.): 93.77%; r.t.= 2.570 min; m/z(+)= 628.35.

### Example 37: 3-[4-(2-Carbamoylethyl)piperazin-1-yl]-N-{4-[7-(5-chloro-2-fluorophenyl)-1H,2H,3H-pyrido[3,4-b][1,4]oxazin-1-yl]pyridin-2-yl}propanamide

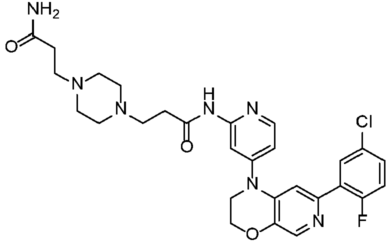

The title compound was prepared following the procedure used for the synthesis of Example 8, starting from freshly-prepared Intermediate 4 (100 mg, 1.0 eq.) and using Intermediate 36 (2.1 eq.). Purification by pTLC on silica gel eluting with DCM:MeOH (85:15), followed by trituration with ACN and MeOH yielded Example 37 (38 mg, Y = 34%) as a beige powder. ¹H NMR (400 MHz, DMSO-*d*₆) δ 10.74 (s, 1H), 8.29 - 8.22 (m, 2H), 8.14 (d, *J* = 2.1 Hz, 1H), 7.93 (dd, *J* = 6.8, 2.8 Hz, 1H), 7.62 (s, 1H), 7.46 (ddd, *J* = 8.7, 4.1, 2.8 Hz, 1H), 7.34 (s, 1H), 7.31 (dd, *J* = 10.9, 8.8 Hz, 1H), 7.07 (dd, *J* = 5.7, 2.2 Hz, 1H), 6.75 (s, 1H), 4.40 (t, *J* = 4.4 Hz, 2H), 3.88 (t, *J* = 4.2 Hz, 2H), 2.59 (m, 2H), 2.55 - 2.46 (signal overlayed by DMSO, 6H) 2.39 (br s, 6H), 2.19 (t, *J* = 7.2 Hz, 2H). LCMS (Method M): 254 nm: 98.39%, 220 nm: 97.10%, 240 nm (max. abs.): 98.54%; r.t.= 2.773 min; m/z(+)= 568.08.

### Example 38: N-{4-[7-(6-chloro-3-fluoropyridin-2-yl)-1H,2H,3H-pyrido[3,4-b][1,4]oxazin-1-yl]pyridin-2-yl}-3-(4-methylpiperazin-1-yl)propanamide

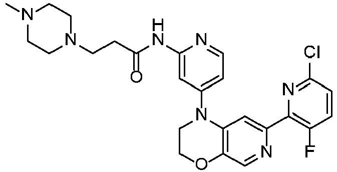

Following the procedure used for the synthesis of Intermediate 4, starting from Example 7 (90 mg, 1.0 eq.), the corresponding acrylamide was prepared and reacted with N-methylpiperazine (2.5 eq.) according to the procedure described for the synthesis of Example 8. Purification by pTLC on silica gel eluting with DCM:MeOH (9:1) + 0.1% TEA followed by trituration in ACN yielded Example 38 (45 mg, Y = 35%) as a beige powder. ¹H NMR (400 MHz, DMSO-*d*₆) δ 10.75 (s, 1H), 8.29 - 8.21 (m, 2H), 8.10 (d, *J* = 2.2 Hz, 1H), 7.90 (dd, *J* = 10.2, 8.6 Hz, 1H), 7.75 (s, 1H), 7.57 (dd, *J* = 8.6, 2.9 Hz, 1H), 7.09 (dd, *J* = 5.7, 2.2 Hz, 1H), 4.42 (dd, *J* = 5.1, 3.5 Hz, 2H), 3.88 (t, *J* = 4.3 Hz, 2H), 2.58 (m, 2H), 2.52 (m, 2H, overlayed by DMSO), 2.40 (br s, 4H), 2.30 (br s, 4H), 2.14 (s, 3H).LCMS (Method E): 254 nm: 99.59%, 220 nm: 100.00%, 240 nm (max. abs.): 99.64%; r.t.= 2.493 min; m/z(+)= 512.16.

### Example 39: N-{4-[7-(6-chloro-3-fluoropyridin-2-yl)-1H,2H,3H-pyrido[3,4-b][1,4]oxazin-1-yl]pyridin-2-yl}-3-(4-methylpiperazin-1-yl)propanamide

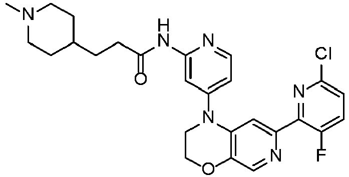

The title compound was prepared following the procedure used for the synthesis of Intermediate 33, starting from Example 7 (30 mg, 1.0 eq.) and using 3-(1-methylpiperidin-4-yl)propanamide hydrochloride (35 mg, 2.0 eq.). Purification by pTLC on silica gel eluting with EtOAc: MeOH (10:1) +2.5%TEA yielded Example 39 (13 mg, Y = 13%) as a beige powder. ¹H NMR (400 MHz, DMSO-*d*₆) δ 10.52 (s, 1H), 8.28 - 8.21 (m, 2H), 8.10 (d, *J* = 2.1 Hz, 1H), 7.94 - 7.85 (m, 1H), 7.75 (s, 1H), 7.57 (dd, *J* = 8.6, 2.9 Hz, 1H), 7.08 (dd, *J* = 5.7, 2.2 Hz, 1H), 4.44 - 4.38 (m, 2H), 3.88 (t, *J* = 4.4 Hz, 2H), 2.87 (m, 1H), 2.52 (peak overlayed by DMSO), 2.39 (t, *J* = 7.6 Hz, 2H), 2.26 (s, 2H), 1.65 (d, *J* = 11.6 Hz, 2H), 1.49 (q, *J* = 7.1 Hz, 2H), 1.18 (dd, *J* = 27.2, 15.6 Hz, 4H). LCMS (Method M): 254 nm: 96.91%, 220 nm: 96.39%, 240 nm (max. abs.): 97.29%; r.t.= 2.673 min; m/z(+)= 510.90.

### Example 40: N-{4-[7-(5-chloro-2-fluorophenyl)-1H,2H,3H-pyrido[3,4-b][1,4]oxazin-1-yl]pyridin-2-yl}-3-methoxypropanamide

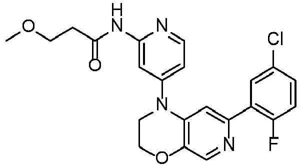

The title compound was prepared following the procedure used for the synthesis of Example 8, starting from freshly-prepared Intermediate 4 (220 mg, 1.0 eq.) and using MeOH (11.3 mL). Purification by silica gel column chromatography eluting with gradient from 0 to 50% of MeOH in DCM followed by p-TLC on silica gel eluting DCM:MeOH (9.5:0.5) + 0.1%TEA yielded Example 40 (18 mg, Y = 11%) as a beige powder. ¹H NMR (400 MHz, DMSO-*d*₆) δ 8.19 (s, 1H), 7.96 (d, *J* = 5.7 Hz, 1H), 7.92 (dd, *J* = 6.8, 2.8 Hz, 1H), 7.52 (d, *J* = 1.0 Hz, 1H), 7.46 (ddd, *J* = 8.7, 4.1, 2.8 Hz, 1H), 7.32 (dd, *J* = 11.1, 8.8 Hz, 1H), 6.67 (t, *J* = 5.8 Hz, 1H), 6.53 (dd, *J* = 5.7, 2.0 Hz, 1H), 6.37 (d, *J* = 2.0 Hz, 1H), 4.36 (dd, *J* = 5.1, 3.5 Hz, 2H), 3.80 (t, *J* = 4.3 Hz, 2H), 3.59 (s, 3H), 3.49 (q, *J* = 6.5 Hz, 2H), 2.58 (t, *J* = 6.8 Hz, 2H). LCMS (Method C): 254 nm: 96.37%, 220 nm: 93.58%, 240 nm (max. abs.): 93.94%; r.t.= 1.700 min; m/z(+)= 443.06.

### Example 41: N-{4-[7-(2-chloro-5-fluoropyridin-4-yl)-1H,2H,3H-pyrido[3,4-b][1,4]oxazin-1-yl]pyridin-2-yl}-3-(1-methylpiperidin-4-yl)propanamide

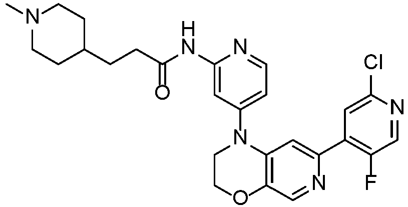

The title compound was prepared following the procedure used for the synthesis of Intermediate 33, starting from Intermediate 41 (27 mg, 1.0 eq.) and using 3-(1-methylpiperidin-4-yl)propanamide hydrochloride (2.0 eq.). Purification by pTLC on silica gel eluting with EtOAc:MeOH (9:1) + 5%TEA yielded Example 41 (11 mg, Y = 29%) as a white powder. ¹H NMR (400 MHz, DMSO-*d*₆) δ 10.53 (s, 1H), 8.48 (d, *J* = 2.7 Hz, 1H), 8.31 - 8.21 (m, 2H), 8.15 (d, *J* = 2.2 Hz, 1H), 7.99 (d, *J* = 5.7 Hz, 1H), 7.76 (s, 1H), 7.07 (dd, *J* = 5.7, 2.2 Hz, 1H), 4.42 (t, *J* = 4.3 Hz, 2H), 3.89 (t, *J* = 4.5 Hz, 2H), 2.72 (d, *J* = 11.2 Hz, 2H), 2.39 (d, *J* = 7.5 Hz, 2H), 2.12 (s, 3H), 1.78 (br s, 2H), 1.60 (d, *J* = 10.4 Hz, 2H), 1.50 (q, *J* = 7.0 Hz, 2H), 1.18 - 1.07 (m, 3H). LCMS (Method L): 254 nm: 96.92%, 220 nm: 95.60%, 235 nm (max. abs.): 96.19%; r.t.= 1.843 min; m/z(+)= 511.02.

### Example 42: N-{4-[7-(2-chloro-5-fluoropyridin-4-yl)-1H,2H,3H-pyrido[3,4-b][1,4]oxazin-1-yl]pyridin-2-yl}-3-(4-methylpiperazin-1-yl)propanamide

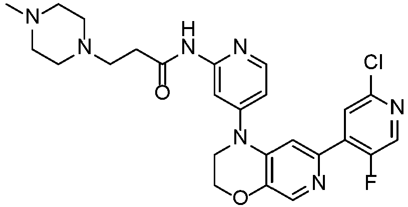

The title compound was prepared following the procedure used for the synthesis of Example 8, starting from freshly-prepared Intermediate 4 (220 mg, 1.0 eq.) and using *N*-methylpiperazine (3.5 eq.). Purification by pTLC on silica gel eluting with EtOAc:MeOH (10:1) + 2.5%TEA yielded Example 42 (9 mg, Y = 21%) as a beige powder. ¹H NMR (400 MHz, DMSO-*d*₆) δ 10.75 (s, 1H), 8.48 (d, *J* = 2.7 Hz, 1H), 8.32 - 8.24 (m, 2H), 8.14 (d, *J* = 2.1 Hz, 1H), 7.99 (d, *J* = 5.7 Hz, 1H), 7.75 (s, 1H), 7.07 (dd, *J* = 5.7, 2.2 Hz, 1H), 4.42 (t, *J* = 4.3 Hz, 2H), 3.92 - 3.85 (m, 2H), 2.60 - 2.50 (m, overlayed by DMSO, 4H), 2.41 (br s, 4H), 2.30 (br s, 4H), 2.14 (s, 3H). LCMS (Method L): 254 nm: 88.46%, 220 nm: 87.31%, 205 nm (max. abs.): 89.43%; r.t.= 1.720 min; m/z(+)= 511.91.

### Example 43: N-{4-[7-(5-chloro-2-fluorophenyl)-1H,2H,3H-pyrido[3,4-b][1,4]oxazin-1-yl]pyridin-2-yl}-2-(4-methylpiperazin-1-yl)acetamide

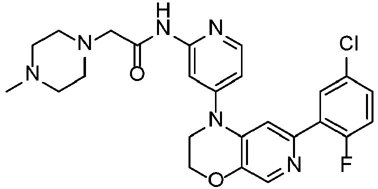

The title compound was prepared following the procedure used for the synthesis of Intermediate 3, starting from Intermediate 2 (200 mg, 1.0 eq.), using Intermediate 42 (2.0 eq.), XantphosPdG3 (0.2 eq.) and Cs₂CO₃ (2.0 eq.). Purification by silica gel column chromatography eluting with gradient from 0 to 100% of EtOAc+2.5%TEA in hexane followed by trituration in MeOH yielded Example 43 (88 mg, Y = 24%) as a white powder. ¹H NMR (400 MHz, DMSO-*d*₆) δ 9.93 (s, 1H), 8.30 - 8.22 (m, 2H), 8.12 (d, *J* = 2.1 Hz, 1H), 7.92 (dd, *J* = 6.8, 2.8 Hz, 1H), 7.63 (s, 1H), 7.45 (ddd, *J* = 8.8, 4.1, 2.8 Hz, 1H), 7.30 (dd, *J* = 11.0, 8.8 Hz, 1H), 7.10 (dd, *J* = 5.7, 2.2 Hz, 1H), 4.42 - 4.37 (m, 2H), 3.88 (t, *J* = 4.4 Hz, 2H), 3.16 (s, 2H), 2.53 (br s, 4H), 2.36 (br s, 4H), 2.17 (s, 3H). LCMS (Method K): 254 nm: 97.43%, 220 nm: 97.52%, 240 nm (max. abs.): 97.46%; r.t.= 1.737 min; m/z(+)= 497.18

### Example 44: 7-(5-Chloro-2-fluorophenyl)-1-{2-[3-(4-methylpiperazin-1-yl)propanamido]pyridin-4-yl}-1H,2H,3H-pyrido[3,4-b][1,4]oxazine-5-carboxylic acid

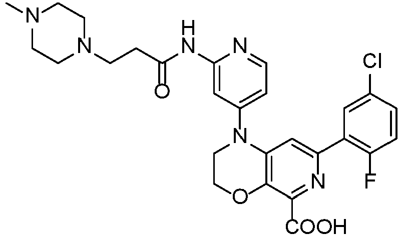

The title compound was prepared following the procedure used for the synthesis of Example 43, using 28 mg of Intermediate 46 (28 mg, 1.0 eq.) and Intermediate (2.0 eq.). Purification by preparative HPLC using ACN:water gradient with 0.1% of formic acid yielded Example 44 (4 mg, Y = 10%) as a double formic acid salt in form of a white powder. ¹H NMR (400 MHz, DMSO-*d*₆) δ 10.69 (s, 1H), 8.27 (s, 2H, 2 x formyl), 8.22 (d, *J* = 5.7 Hz, 1H), 8.09 (d, *J* = 2.2 Hz, 1H), 7.94 (dd, *J* = 6.8, 2.8 Hz, 1H), 7.48 (s, 1H), 7.43 (dt, *J* = 7.1, 3.4 Hz, 1H), 7.27 (dd, *J* = 11.0, 8.7 Hz, 1H), 7.03 (dd, *J* = 5.7, 2.2 Hz, 1H), 4.32 (d, *J* = 4.8 Hz, 2H), 3.86 - 3.80 (m, 2H), 2.63 - 2.57 (m, 2H), 2.56 - 2.26 (broad signal overlayed by DMSO, 10H), 2.30 (s, 2H), 2.14 (s, 3H). LCMS (Method B): 254 nm: 98.12%, 205 nm (max. abs.): 98.18%; r.t.= 1.477 min; m/z(+)= 555.18

### Example 45: Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazine-2-carboxylate

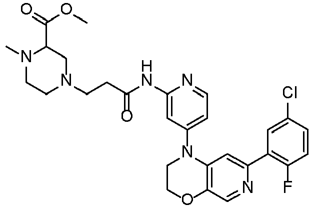

The title compound was prepared following the procedure used for the synthesis of Intermediate 55, starting from Intermediate 2 (25 mg, 1.0 eq.) and using Intermediate 53 (1.1 eq.). Purification by NH flash chromatography using gradient from 0 to 60% of EtOAc in c-Hex yielded Example 45 (32 mg, Y = 60%) as an off-white solid. ¹H NMR (400 MHz, DMSO-*d₆*) δ ppm 10.60 (s, 1 H), 8.24 - 8.26 (m, 1 H), 8.24 (s, 1 H), 8.12 (d, *J*=1.8 Hz, 1 H), 7.92 (dd, *J*=6.7, 2.7 Hz, 1 H), 7.60 (s, 1 H), 7.41 - 7.51 (m, 1 H), 7.30 (dd, *J*=11.0, 8.8 Hz, 1 H), 7.06 (dd, *J*=5.7, 2.2 Hz, 1 H), 4.31 - 4.48 (m, 2 H), 3.78 - 3.94 (m, 2 H), 3.59 (s, 3 H), 2.91 - 2.99 (m, 1 H), 2.65 - 2.75 (m, 1 H), 2.47 - 2.64 (m, 4 H), 2.25 - 2.44 (m, 1 H), 2.20 (s, 3 H), 2.12 - 2.89 (m, 4 H). LC-MS (ESI): *m*/*z* (M+1): 569.4 (Method 2)

### Example 46: Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]piperazine-2-carboxylate

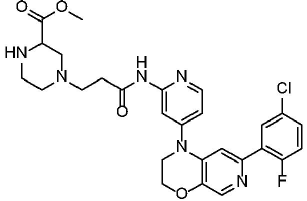

The title compound was prepared following the procedure used for the synthesis of Intermediate 52, starting from Intermediate 55 (80 mg, 1.0 eq.). Purification by NH flash chromatography using gradient from 0 to 2% of MeOH in DCM yielded Example 46 (49 mg, Y = 72%) as white glassy solid. ¹H NMR (500 MHz, DMSO-*d₆*) δ ppm 10.67 (s, 1 H), 8.25 (d, *J*=5.7 Hz, 1 H), 8.24 (s, 1 H), 8.13 (d, *J*=1.5 Hz, 1 H), 7.92 (dd, *J*=6.8, 2.8 Hz, 1 H), 7.60 (s, 1 H), 7.40 - 7.51 (m, 1 H), 7.30 (dd, *J*=10.9, 8.8 Hz, 1 H), 7.06 (dd, *J*=5.7, 2.1 Hz, 1 H), 4.30 - 4.50 (m, 2 H), 3.81 - 3.96 (m, 2 H), 3.60 (s, 3 H), 3.43 (dd, *J*=7.7, 2.9 Hz, 1 H), 2.82 - 2.93 (m, 1 H), 2.74 (br d, *J*=9.3 Hz, 1 H), 2.56 - 2.66 (m, 3 H), 2.44 - 2.55 (m, 3 H), 2.40 (br s, 1 H), 2.24 - 2.35 (m, 1 H), 2.16 (br t, *J*=8.3 Hz, 1 H). LC-MS (ESI): *m*/*z* (M+1): 555.4 (Method 2)

### Example 47: Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazin-2-yl}acetate

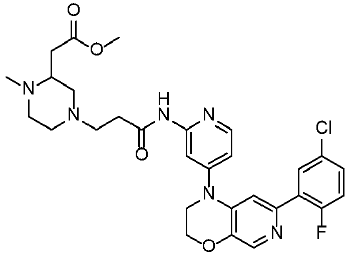

The title compound was prepared following the procedure used for the synthesis of Intermediate 55, starting from Intermediate 2 (45 mg, 1.0 eq.) and using Intermediate 62 (1.1 eq.). Purification by NH flash chromatography using gradient from 0 to 60% of EtOAc in c-Hex, followed by silica gel flash chromatography uing gradient from 0 to 8% of MeOH in DCM yielded Example 47 (24 mg, Y = 24%) as white solid. ¹H NMR (400 MHz, DMSO-*d₆*) δ ppm 10.91 (br s, 1 H), 8.29 (s, 1 H), 8.21 (d, *J*=5.5 Hz, 1 H), 8.07 (d, *J*=2.0 Hz, 1 H), 7.94 (dd, *J*=6.8, 2.6 Hz, 1 H), 7.62 (s, 1 H), 7.21 - 7.26 (m, 1 H), 6.95 - 7.07 (m, 2 H), 4.34 - 4.45 (m, 2 H), 3.84 - 3.93 (m, 2 H), 3.68 (s, 3 H), 2.75 (br t, *J*=4.7 Hz, 2 H), 2.52 - 2.57 (m, 2 H), 2.31 - 2.42 (m, 3 H), 2.27 - 3.10 (m, 9 H). LC-MS (ESI): m/z (M+1): 583.4 (Method 2)

### Example 48: Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-2-yl}acetate

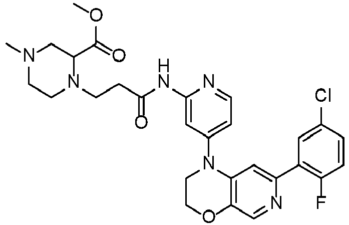

The title compound was prepared following the procedure used for the synthesis of Intermediate 52, starting from Intermediate 63 (105 mg, 1.0 eq.). Purification by NH flash chromatography using gradient from 0 to 2% of MeOH in DCM yielded Example 48 (60 mg, Y = 67%) as white solid. ¹H NMR (500 MHz, DMSO-*d*₆) δ ppm 11.14 (s, 1 H), 8.29 (s, 1 H), 8.21 (d, *J*=5.6 Hz, 1 H), 8.07 (d, *J*=2.1 Hz, 1 H), 7.94 (dd, *J*=6.9, 2.7 Hz, 1 H), 7.61 (d, *J*=0.8 Hz, 1 H), 7.23 - 7.26 (m, 1 H), 6.98 - 7.07 (m, 2 H), 4.35 - 4.46 (m, 2 H), 3.83 - 3.94 (m, 2 H), 3.71 (s, 3 H), 3.33 - 3.44 (m, 1 H), 3.02 - 3.19 (m, 2 H), 2.86 - 2.98 (m, 2 H), 2.69 - 2.79 (m, 2 H), 2.52 - 2.61 (m, 2 H), 2.48 (br d, *J*=6.3 Hz, 2 H), 2.23 - 2.31 (m, 1 H), 2.02 (br t, *J*=10.1 Hz, 1 H). LC-MS (ESI): m/z (M+1): 569.4 (Method 2)

### Example 49: Methyl 1-[2-({4-[7-(5-chloro-2-fluorophenyl)-1H,2H,3H-pyrido[3,4-b][1,4]oxazin-1-yl]pyridin-2-yllcarbamoyl)ethyl]-4-methylpiperazine-2-carboxylate

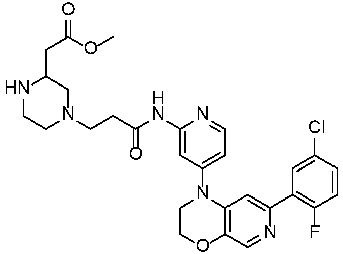

The title compound was prepared following the procedure used for the synthesis of Intermediate 55, starting from Intermediate 2 (25 mg, 1.0 eq.) and using Intermediate 53 (1.1 eq.). Purification by NH flash chromatography using gradient from 0 to 60% of EtOAc in c-Hex yielded Example 49 (46 mg, Y = 49%) as white solid. ¹H NMR (400 MHz, DMSO-*d*₆) δ ppm 10.47 (s, 1 H), 8.29 (s, 1 H), 8.23 (d, *J*=5.7 Hz, 1 H), 8.08 (d, *J*=2.1 Hz, 1 H), 7.94 (dd, *J*=6.8, 2.7 Hz, 1 H), 7.62 (d, *J*=0.8 Hz, 1 H), 7.22 - 7.26 (m, 1 H), 6.99 - 7.08 (m, 2 H), 4.33 - 4.49 (m, 2 H), 3.82 - 3.96 (m, 2 H), 3.75 (s, 3 H), 3.51 (br t, *J*=3.9 Hz, 1 H), 3.10 - 3.37 (m, 2 H), 2.81 - 3.04 (m, 2 H), 2.75 (br dd, *J*=11.2, 3.1 Hz, 1 H), 2.64 (br d, *J*=6.5 Hz, 2 H), 2.42 - 2.57 (m, 3 H), 2.33 (s, 3 H). LC-MS (ESI): m/z (M+1): 569.0 (Method 2).

### Example 50: Methyl 2-{1-[2-({4-[7-(5-chloro-2-fluorophenyl)-1H,2H,3H-pyrido[3,4-b][1,4]oxazin-1-yl]pyridin-2-yl}carbamoyl)ethyl]-4-methylpiperazin-2-yl}acetate

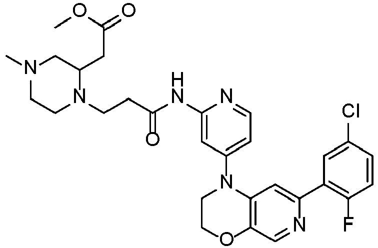

The title compound was prepared following the procedure used for the synthesis of Intermediate 55, starting from Intermediate 2 (50 mg, 1.0 eq.) and using Intermediate 69 (1.1 eq.). Purification by NH flash chromatography using gradient from 0 to 60% of EtOAc in c-Hex, followed by silica gel flash chromatography uing gradient from 0 to 8% of MeOH in DCM yielded Example 49 (56 mg, Y = 51%) as white solid. ¹H NMR (400 MHz, DMSO-*d*₆) δ ppm 10.74 (br s, 1 H), 8.29 (s, 1 H), 8.21 (d, *J*=5.6 Hz, 1 H), 8.06 (d, *J*=2.0 Hz, 1 H), 7.94 (dd, *J*=6.8, 2.6 Hz, 1 H), 7.62 (d, *J*=0.9 Hz, 1 H), 7.21 - 7.26 (m, 1 H), 6.98 - 7.08 (m, 2 H), 4.25 - 4.54 (m, 2 H), 3.80 - 3.96 (m, 2 H), 3.69 (s, 3 H), 3.29 (br s, 1 H), 2.40 - 3.03 (m, 12 H), 2.31 (s, 3 H). LC-MS (ESI): m/z (M+1): 583.4 (Method 2)

### Example 51: N-{4-[5-amino-7-(5-chloro-2-fluorophenyl)-1H,2H,3H-pyrido[3,4-b][1,4]oxazin-1-yl]pyridin-2-yl}-3-(4-methylpiperazin-1-yl)propanamide

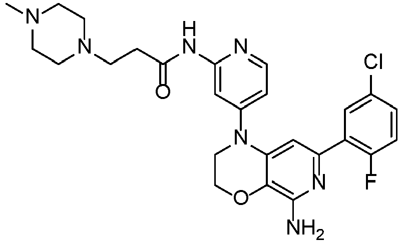

Example 44 (20 mg, 1.0 eq.) was suspended in dry THF (1.0 mL) and T3P (50% solution in EtOAc, 5.0 eq.), TMSN₃ (5.0 eq.), TEA (5.0 eq.) and *tert*-buthanol (10 eq.) were added. The RM was sealed and stirred at 78 °C for 15 minutes. The RM was cooled to RT and partitioned between DCM and water. The aqueous phase was alkalized using aq. 6M NaOH and extracted with DCM. Combined organics were dried over Na₂SO₄, filtered and dried under reduced pressure. Purification by preparative HPLC under basic conditions using gradient of water with 0.05% NH₃ and ACN yielded Example 51 (4 mg, Y = 21%) as a white powder. ¹H NMR (400 MHz, DMSO-*d*₆) δ 10.64 (s, 1H), 8.17 (d, *J* = 5.7 Hz, 1H), 8.05 (d, *J* = 2.1 Hz, 1H), 7.97 (dd, *J* = 6.9, 2.8 Hz, 1H), 7.39 (ddd, *J* = 8.6, 4.1, 2.8 Hz, 1H), 7.24 (dd, *J* = 11.1, 8.7 Hz, 1H), 7.00 - 6.93 (m, 2H), 5.90 (s, 2H), 4.34 (t, *J* = 4.3 Hz, 2H), 3.83 (t, *J* = 3.9 Hz, 2H), 2.58 (d, *J* = 6.1 Hz, 2H), 2.55 - 2.22 (m, 10H), 2.14 (s, 3H). LCMS (Method H): 254 nm: 98.77%, 220 nm (max. abs.): 96.58%; r.t.= 2.623 min; m/z(+)= 526.37.

### Example 52: N-{4-[7-(5-chloro-2-fluorophenyl)-1H,2H,3H-pyrido[3,4-b][1,4]oxazin-1-yl]pyridin-2-yll-2-15-methyl-2,5-diazabicyclo[2.2.1]heptan-2-yl}acetamide

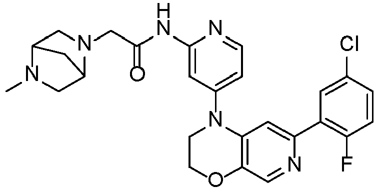

The title compound was prepared following the procedure used for the synthesis of Intermediate 3, starting from Intermediate 2 (9 mg, 1.0 eq.) and using Intermediate 70 (2.0 eq.), Cs₂CO₃ (2.0 eq.) and XantphosPdG3 (0.2 eq.). Purification by preparative HPLC under basic conditions using gradient of water with 0.05% NH₃ and ACN yielded Example 52 (10.6 mg, Y = 43%) as a beige powder. ¹H NMR (400 MHz, DMSO-*d*₆) δ 9.88 (s, 1H), 8.26 (d, *J* = 5.1 Hz, 2H), 8.11 (d, *J* = 2.1 Hz, 1H), 7.91 (dd, *J* = 6.8, 2.8 Hz, 1H), 7.62 (s, 1H), 7.46 (ddd, *J* = 8.8, 4.1, 2.8 Hz, 1H), 7.31 (dd, *J* = 11.0, 8.8 Hz, 1H), 7.11 (dd, *J* = 5.7, 2.2 Hz, 1H), 4.39 (dd, *J* = 5.1, 3.4 Hz, 2H), 3.88 (t, *J* = 4.2 Hz, 2H), 3.31 (s, 2H), 3.17 (s, 1H), 2.76 (d, *J* = 9.5 Hz, 1H), 2.69 (d, *J* = 2.5 Hz, 2H), 2.58 (d, *J* = 1.5 Hz, 2H), 2.26 (s, 3H), 1.63 (d, *J* = 2.1 Hz, 2H). LCMS (Method E): 254 nm: 95.16%, 240 nm (max. abs.): 94.56%, 220 nm: 93.91%, ; r.t.= 1.743 min; m/z(+)= 509.14.

### Example 53: N-{4-[7-(5-chloro-2-fluorophenyl)-1H,2H,3H-pyrido[3,4-b][1,4]oxazin-1-yl]pyridin-2-yl}-2-{6-methyl-2,6-diazaspiro[3.3]heptan-2-yl}acetamide

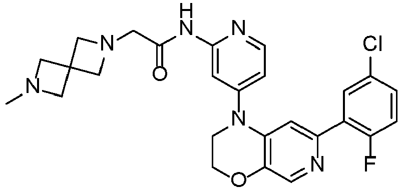

The title compound was prepared following the procedure used for the synthesis of Intermediate 3, starting from Intermediate 2 (20 mg, 1.0 eq.) and using Intermediate 71 (2.0 eq.), Cs₂CO₃ (2.0 eq.) and XantphosPdG3 (0.2 eq.). Purification by NH₂-modified silica preparative TLC eluting with DCM/MeOH 5% yielded Example 53 (6.0 mg, Y = 16%) as a white powder. ¹H NMR (400 MHz, DMSO-*d*₆) δ 9.95 (s, 1H), 8.25 (t, *J* = 2.8 Hz, 2H), 8.07 (d, *J* = 2.2 Hz, 1H), 7.91 (dd, *J* = 6.8, 2.8 Hz, 1H), 7.61 (s, 1H), 7.46 (ddd, *J* = 8.8, 4.2, 2.8 Hz, 1H), 7.31 (dd, *J* = 11.0, 8.8 Hz, 1H), 7.09 (dd, *J* = 5.7, 2.2 Hz, 1H), 4.43 - 4.34 (m, 2H), 3.87 (t, *J* = 4.3 Hz, 2H), 3.33 (s, 2H, overlayed by H₂O), 3.23 - 3.11 (m, 8H), 2.13 (s, 3H). LCMS (Method E): 254 nm: 95.16%, 240 nm (max. abs.): 94.56%, 220 nm: 93.91%, ; r.t.= 1.743 min; m/z(+)= 509.14.

### Example 54: N-{4-[7-(5-chloro-2-fluorophenyl)-1H,2H,3H-pyrido[3,4-b][1,4]oxazin-1-yl]pyridin-2-yll-2-12-methyl-5-oxa-2,8-diazaspiro[3.5]nonan-8-yl}acetamide

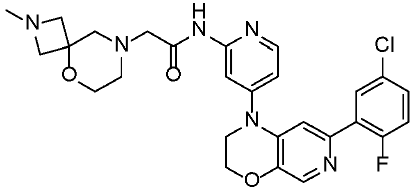

The title compound was prepared following the procedure used for the synthesis of Intermediate 3, starting from Intermediate 2 (31 mg, 1.0 eq.) and using Intermediate 72 (2.0 eq.), Cs₂CO₃ (2.0 eq.) and XantphosPdG3 (0.2 eq.). Purification by preparative HPLC under basic conditions using gradient of water with 0.05% NH₃ and ACN yielded Example 54 (12 mg, Y = 19%) as a white powder. ¹H NMR (400 MHz, DMSO-*d*₆) δ 10.04 (s, 1H), 8.29 - 8.23 (m, 2H), 8.14 (d, *J* = 2.2 Hz, 1H), 7.92 (dd, *J* = 6.8, 2.8 Hz, 1H), 7.64 (s, 1H), 7.45 (ddd, *J* = 8.8, 4.1, 2.8 Hz, 1H), 7.31 (dd, *J* = 11.0, 8.8 Hz, 1H), 7.11 (dd, *J* = 5.7, 2.2 Hz, 1H), 4.40 (t, *J* = 4.3 Hz, 2H), 3.89 (t, *J* = 4.4 Hz, 2H), 3.55 (t, *J* = 4.7 Hz, 2H), 3.21 (s, 2H), 2.78 (d, *J* = 7.7 Hz, 2H), 2.62 (s, 2H), 2.53 - 2.51 (m, 4H), 2.24 (s, 3H). LCMS (Method F): 254 nm: 99.49%, 240 nm (max. abs.): 99.71%, 220 nm: 99.86%, ; r.t.= 1.860 min; m/z(+)= 539.19.

### Example 55: 1-(4-(7-(5-Chloro-2-fluorophenyl)-2,3-dihydro-1H-pyrido [3,4-b] [1,4] oxazin-1-yl)pyridin-2-yl)-3-(2-(4-methylpiperazin-1-yl)ethyl)urea

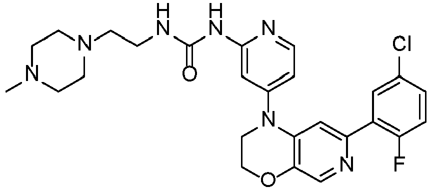

The title compound was prepared following the procedure used for the synthesis of Intermediate 3, starting from Intermediate 2 (100 mg, 1.0 eq.) and using Intermediate 73 (1.5 eq.), Cs₂CO₃ (2.0 eq.) and XantphosPdG3 (0.2 eq.). Purification by reverse phase flash chromatography using gradient from 0 to 50% of B in A (A:water/ACN 95:5 + 0.1% formic acid, B: ACN/water 95:5 + 0.1% formic acid) yielded Example 55 (8 mg, Y = 4%) as yellowish solid. LC-MS (ESI): m/z (M+1): 526.3 (Method 1). ¹H NMR (400 MHz, DMSO-*d6*) δ 9.24 (s, 1 H), 8.27 (br s, 1 H), 8.23 (s, 1 H), 8.12 (d, J=5.70 Hz, 1 H), 7.93 (dd, J=6.69, 1.86 Hz, 1 H), 7.59 (s, 1 H), 7.41 - 7.50 (m, 1 H), 7.28 - 7.36 (m, 2 H), 6.91 (br d, J=5.70 Hz, 1 H), 4.34 - 4.42 (m, 2 H), 3.78 - 3.90 (m, 2 H), 3.23 - 3.27 (m, 2 H), 2.40 (br t, J=6.03 Hz, 2 H), 2.31 - 2.43 (m, 8 H), 2.16 (s, 3 H).

### Example 56: N-(3-(7-(5-chloro-2-fluorophenyl)-5-(trifluoromethyl)-2,3-dihydro-1H-pyrido[3,4-b][1,4]oxazin-1-yl)phenyl)-3-(4-methylpiperazin-1-yl)propanamide

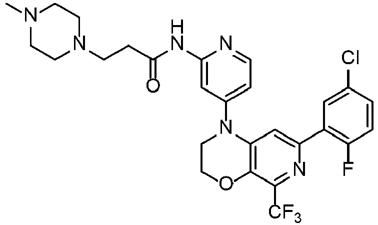

The title compound was prepared following the procedure used for the synthesis of Intermediate 3, starting from Intermediate 75 (40 mg, 1.0 eq.) and using Intermediate 47 (1.0 eq.). Purification by reverse phase flash chromatography using gradient from 0 to 50% of B in A (A:water/ACN 95:5 + 0.1% formic acid , B: ACN/water 95:5 + 0.1% formic acid) yielded Example 56 (7 mg, Y = 11%) as yellowish solid. LC-MS (ESI): m/z (M+1): 579.1 (Method 1). ¹H NMR (600 MHz, DMSO-*d6*) δ 10.80 (s, 1 H) 8.31 (d, J=5.64 Hz, 1 H) 8.15 (s, 1 H) 7.87 (dd, J=6.67, 2.82 Hz, 1 H) 7.72 (s, 1 H) 7.47 - 7.56 (m, 1 H) 7.33 (dd, J=10.77, 8.85 Hz, 1 H) 7.13 (dd, J=5.51, 2.18 Hz, 1 H) 4.48 - 4.56 (m, 2 H) 3.90 - 3.98 (m, 2 H) 2.58 - 2.62 (m, 2 H) 2.52 - 2.55 (m, 2 H) 2.15 - 2.49 (m, 8 H) 2.14 (s, 3 H).

### Comparative newly synthesised compounds having a 5-membered heterocyclic ring linked to the oxazine of the pyrido oxazine ring, whereas in that position, the compounds of the present invention show a pyridine or pyridine fused to a 5-membered heterocycloalkyl linked to the oxazine of the pyrido oxazine ring:

### Example C1: 5-[7-(5-chloro-2-fluorophenyl)-1H,2H,3H-pyrido[3,4-b][1,4]oxazin-1-yl]-1H-pyrazole

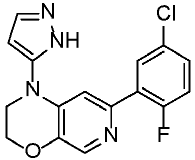

The title compound was prepared following the procedure used for the synthesis of Intermediate 3, starting from 60 mg of Intermediate 2 (60 mg, 1.0 eq.) and using Intermediate 31 (1.15 eq.). Purification by pTLC on silica gel eluting twice with hexane:EtOAc (8:2) + 1% TEA yielded SEM-protected compound (70 mg) as a mixture of regioisomers. The product was dissolved in DCM (9.6 mL) and TFA (3.0 mL) was added. After 17 h, the RM was dried under reduced pressure and the residue was suspended in a 1:1 mixture of dioxane (12 mL) and 25% aq. NH_{3(aq.)} (12 mL). After stirring for 1h volatiles were removed and the residue was washed with water and MeOH yielding CHD-065479 (40 mg, Y= 53%) as a beige powder. ¹H NMR (400 MHz, DMSO-*d*₆) δ 12.65 (s, 1H), 8.10 (s, 1H), 8.04 (d, *J* = 1.4 Hz, 1H), 7.89 (dd, *J* = 6.8, 2.8 Hz, 1H), 7.79 (t, *J* = 1.9 Hz, 1H), 7.45 (ddd, *J* = 8.8, 4.2, 2.8 Hz, 1H), 7.33 (dd, *J* = 11.0, 8.8 Hz, 1H), 6.24 (t, *J* = 2.1 Hz, 1H), 4.37 (dd, *J* = 5.2, 3.7 Hz, 2H), 3.85 (t, *J* = 4.5 Hz, 2H). LCMS (Method A): 210 nm: 94.02%, 254 nm: 94.27%; r.t.= 1.997 min; m/z(+)= 330.88.

### Example C2: 5-[7-(5-Chloro-2-fluorophenyl)-1H,2H,3H-pyrido[3,4-b][1,4]oxazin-1-yl]-1,3-thiazole

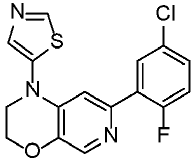

The title compound was prepared following the procedure used for the synthesis of Intermediate 3, starting from Intermediate 2 (40 mg, 1.0 eq.) and using 5-bromo-1,3-thiazole (0.9 eq.). Purification by preparative HPLC using ACN:water gradient with 0.1% of formic acid yielded CHD-064783 (19 mg, Y= 36%) as a beige powder. ¹H NMR (400 MHz, DMSO-*d*₆) δ 9.07 (d, *J* = 0.8 Hz, 1H), 8.16 (s, 1H), 7.92 (d, *J* = 0.8 Hz, 1H), 7.89 (dd, *J* = 6.8, 2.8 Hz, 1H), 7.46 (ddd, *J* = 8.8, 4.2, 2.8 Hz, 1H), 7.30 (dd, *J* = 11.2, 8.8 Hz, 1H), 7.11 (d, *J* = 1.2 Hz, 1H), 4.43 (dd, *J* = 5.2, 3.7 Hz, 2H), 3.87 - 3.80 (m, 2H). LCMS (Method I): 254 nm: 99.45%, 220 nm: 99.56%; r.t.= 1.950 min; m/z(+)= 347.98.

### PHARMACOLOGICAL ACTIVITY OF THE COMPOUNDS OF THE INVENTION

### In vitro Assay

The enzymatic activity of compounds of the present invention was monitored measuring the formation of ADP using the ADP-GLO Kinases assay. Following the incubation of the purified enzyme, a substrate and ATP, the produced ADP was converted into ATP, which in turn was converted into light by Ultra-Glo Luciferase. The luminescent signal positively correlated with ADP amount and kinase activity. Briefly, the kinase reaction was performed by incubating 2.6nM of the purified, commercially available human ALK5 (recombinant TGF β1 N-term GST-tagged, 80-end), a final concentration of TGFβ1 peptide 94.5µM (Promega, T36-58) and ultra-pure ATP (Promega V915B). The ATP concentration was set at the Km value (concentration of substrate which permits the enzyme to achieve half maximal velocity (Vmax)) of ALK5 (5µM). All reactions/incubations were performed at 25°C. Compound and ALK5 kinase were mixed and incubated for 15 mins. Reactions were initiated by addition of ATP at a final concentration in the assay of 0.83µM. After an incubation of 150 min, the reaction was stopped, and ADP production detected with ADP-Glo kit according to manufacturer's indications. The assay was performed in 384-well format and was validated using a selection of reference compounds that was tested in 11 point concentration-response curve.

The results for individual compounds are provided below in Table 4 wherein the compounds are classified in term of potency (nM) with respect to their inhibitory activity on ALK5 receptor

**Table 4**

| **Example No** | **ALK5 IC50 (nM)** |
|---|---|
| 6, 8, 9, 10, 14, 16, 18, 19, 20, 21, 22, 23, 24, 25, 26, 28, 29, 30, 31, 32, 33, 34, 35, 36, 37, 38, 39, 41, 42, 43, 45, 46, 47, 48, 49, 50, 51, 52, 53, 54, 55 | < 1 |
| 3, 12, 13, 15, 27, 44 | 1 - 5 |
| 1, 2, 4, 5, 7, 11, 17, 40, 56 | 5 - 10 |

As it can be appreciated, all the compounds of Table 4 show a good activity as inhibitors of ALK5 receptor.

### Comparative Examples

Compounds of the examples C1 and C2 were tested in the same *in vitro* assay described above.

**Table 5**

| **Example No** | **ALK5 IC50 (nM)** |
|---|---|
| C1 | 9205 |
| C2 | 10200 |

The compounds of the present invention, as shown in Table 4, have a potency even lower than 1 nM, whereas comparative examples C1 and C2 have a potency higher than 9000 nM and even largely higher.

These data, not even comparable, demonstrate that, conversely to the compounds C1 and C2 characterized by a pyrido oxazine ring linked to a 5-membered heterocyclic ring, in the compounds of the present invention, characterized by an oxazine ring linked to a pyridine or pyridine fused to a 5-membered heteocyclic ring, the presence of such pyridine or pyridine fused to a 5-membered heterocyclic ring unexpectedly and remarkably determines a relevant increase in the inhibitory activity on the ALK5 receptor.

## Claims

1. A compound of general formula (I) 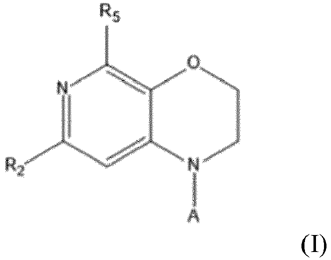 wherein
**A** is selected from the groups consisting of **A1** and **A2** 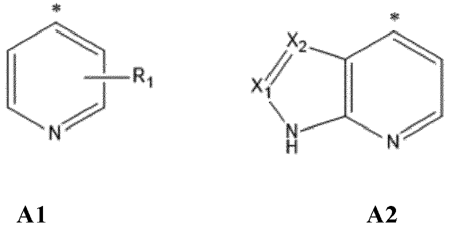
**R₁** is H or is selected from the group consisting of -NR₃C(O)R₄ and -NR₃R₃;
**X₁** and **X₂** are independently N or CH;
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄** is selected from the group consisting of -(C₁-C₆)alkyl, -NR_{A}R_{B}, -(C₁-C₆)alkylene-NR_{A}R_{B}, -(C₁-C₆)alkylene-O-(C₁₋C₆)alkyl, -NH(C₁-C₆)alkylene-heterocycloalkyl and -(C₁-C₆)alkylene-heterocycloalkyl, wherein said heterocycloalkyl is optionally substituted by one or more groups selected from oxo, alkoxy, -(C₁₋C₆)alkylene-OH, -C(O)O-(C₁-C₆)alkyl, -(C₁₋C₆)alkylene-C(O)O-(C₁-C₆)alkyl, -(C₁₋C₆)alkylene-C(O)NH-(C₁₋C₆)alkyl, -(C₁₋C₆)alkylene-C(O)NH-(C₁₋C₆)alkyl-OH, -(C₁₋C₆)alkylene-NHSO₂-(C₁₋C₆)alkyl, -(C₁₋C₆)alkylene-CONH₂, -(C₁-C₆)alkylene-NR_{A}R_{B}, and -(C₁-C₆)alkyl;
**R_{A}** is H or is selected from the group consisting of -(C₁-C₆)alkyl, heterocycloalkyl and -(C₁-C₆)hydroxyalkyl;
**R_{B}** is selected from the group consisting of -(C₁-C₆)alkyl, -(C₁-C₆)alkylene-C(O)O-(C₁-C₆)alkyl, heterocycloalkyl, -(C₁-C₆)alkylene-heterocycloalkyl, -SOz-(C₁-C₆)alkyl, -(C₁-C₆)alkylene-NH-SO₂-(C₁-C₆)alkyl, -(C₁-C₆)alkylene-SO₂-(C₁₋C₆)alkyl and -(C₁-C₆)hydroxyalkyl, wherein said heterocycloalkyl of the -(C₁-C₆)alkylene-heterocycloalkyl may be optionally substituted by one or more -(C₁-C₆)alkyl;
**R₅** is H or selected from the group consisting of -NH₂, -C(O)OH, -C(O)O-(C₁₋C₆)alkyl, -(C₁-C₆)haloalkyl and -OH;
and pharmaceutically acceptable salts thereof.

2. The compound of formula (I) according to claim 1, wherein A is group A1 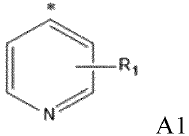 represented by the formula (Ia) 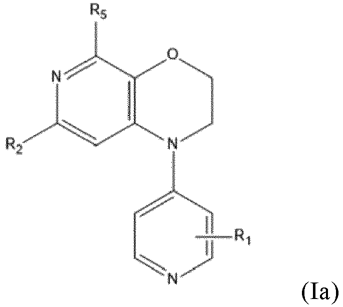
**R₁** is H or is selected from the group consisting of -NR₃C(O)R₄ and -NR₃R_{3'};
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄** is selected from the group consisting of -(C₁-C₆)alkyl, -NR_{A}R_{B}, -(C₁-C₆)alkylene-NR_{A}R_{B}, -(C₁-C₆)alkylene-O-(C₁₋C₆)alkyl, -NH(C₁-C₆)alkylene-heterocycloalkyl and -(C₁-C₆)alkylene-heterocycloalkyl, wherein said heterocycloalkyl is optionally substituted by one or more groups selected from oxo, alkoxy, -(C₁₋C₆)alkylene-OH, -C(O)O-(C₁-C₆)alkyl, -(C₁₋C₆)alkylene-C(O)O-(C₁-C₆)alkyl, -(C₁₋C₆)alkylene-C(O)NH-(C₁₋C₆)alkyl, -(C₁₋C₆)alkylene-C(O)NH-(C₁₋C₆)alkyl-OH, -(C₁₋C₆)alkylene-NHSO₂-(C₁₋C₆)alkyl, -(C₁₋C₆)alkylene-CONH₂, -(C₁-C₆)alkylene-NR_{A}R_{B}, and -(C₁-C₆)alkyl;
**R_{A}** is H or is selected from the group consisting of -(C₁-C₆)alkyl, heterocycloalkyl and -(C₁-C₆)hydroxyalkyl;
**R_{B}** is selected from the group consisting of -(C₁-C₆)alkyl, -(C₁-C₆)alkylene-C(O)O-(C₁-C₆)alkyl, heterocycloalkyl, -(C₁-C₆)alkylene-heterocycloalkyl, -SOz-(C₁-C₆)alkyl, -(C₁-C₆)alkylene-NH-SO₂-(C₁-C₆)alkyl, -(C₁-C₆)alkylene-SO₂-(C₁₋C₆)alkyl and -(C₁-C₆)hydroxyalkyl, wherein said heterocycloalkyl of the -(C₁-C₆)alkylene-heterocycloalkyl may be optionally substituted by one or more -(C₁-C₆)alkyl;
**R₅** is H or selected from the group consisting of -NH₂, -C(O)OH, -C(O)O-(C₁₋C₆)alkyl, -(C₁-C₆)haloalkyl and -OH;
and pharmaceutically acceptable salts thereof.

3. The compound of formula (Ia) according to claim 2 selected from at least one of:
4-[7-(5-chloro-2-fluorophenyl)-1H,2H,3H-pyrido[3,4-b][1,4]oxazin-1-yl]pyridine;
7-(5-chloro-2-fluorophenyl)-1-(pyridin-4-yl)-1H,2H,3H-pyrido[3,4-b][1,4]oxazin-5-amine;
4-[7-(5-chloro-2-fluorophenyl)-1H,2H,3H-pyrido[3,4-b][1,4]oxazin-1-yl]pyridin-2-amine;
4-[7-(2,5-difluorophenyl)-1H,2H,3H-pyrido[3,4-b][1,4]oxazin-1-yl]pyridin-2-amine;
4-[7-(3-chlorophenyl)-1H,2H,3H-pyrido[3,4-b][1,4]oxazin-1-yl]pyridin-2-amine;
N-{4-[7-(5-chloro-2-fluorophenyl)-1H,2H,3H-pyrido[3,4-b][1,4]oxazin-1-yl]pyridin-2-yl} acetamide;
[7-(6-chloro-3-fluoropyridin-2-yl)-1H,2H,3H-pyrido[3,4-b][1,4]oxazin-1-yl]pyridin-2-amine;
N-{4-[7-(5-chloro-2-fluorophenyl)-1H,2H,3H-pyrido[3,4-b][1,4]oxazin-1-yl]pyridin-2-yl}-3-(morpholin-4-yl)propanamide;
N-{4-[7-(5-chloro-2-fluorophenyl)-1H,2H,3H-pyrido[3,4-b][1,4]oxazin-1-yl]pyridin-2-yl}-3-(dimethylamino)propanamide;
N-{4-[7-(5-chloro-2-fluorophenyl)-1H,2H,3H-pyrido[3,4-b][1,4]oxazin-1-yl]pyridin-2-yl}-3-(piperazin-1-yl)propanamide;
4-[7-(2-fluoro-5-methylphenyl)-1H,2H,3H-pyrido[3,4-b][1,4]oxazin-1-yl]pyridin-2-amine;
4-[7-(5-cyclopropyl-2-fluorophenyl)-1H,2H,3H-pyrido[3,4-b][1,4]oxazin-1-yl]pyridin-2-amine;
4-{7-[2-fluoro-5-(propan-2-yl)phenyl]-1H,2H,3H-pyrido[3,4-b][1,4]oxazin-1-yl}pyridin-2-amine;
N-{4-[7-(5-chloro-2-fluorophenyl)-1H,2H,3H-pyrido[3,4-b][1,4]oxazin-1-yl]pyridin-2-yl}-3-(4-methylpiperazin-1-yl)propanamide;
3-[bis(2-hydroxyethyl)amino]-N-{4-[7-(5-chloro-2-fluorophenyl)-1H,2H,3H-pyrido[3,4-b][1,4]oxazin-1-yl]pyridin-2-yl}propanamide;
N-{4-[7-(5-chloro-2-fluorophenyl)-1H,2H,3H-pyrido[3,4-b][1,4]oxazin-1-yl]pyridin-2-yl}-3-[methyl(oxetan-3-yl)amino]propanamide;
N-{4-[7-(5-chloro-2-fluorophenyl)-1H,2H,3H-pyrido[3,4-b][1,4]oxazin-1-yl]pyridin-2-yl}-3-[(2-hydroxyethyl)(methyl)amino]propanamide;
3-[bis(oxetan-3-yl)amino]-N-{4-[7-(5-chloro-2-fluorophenyl)-1H,2H,3H-pyrido[3,4-b][1,4]oxazin-1-yl]pyridin-2-yl}propanamide;
N-{4-[7-(5-chloro-2-fluorophenyl)-1H,2H,3H-pyrido[3,4-b][1,4]oxazin-1-yl]pyridin-2-yl}-3-(4-methyl-3-oxopiperazin-1-yl)propanamide;
methyl 2-{[2-({4-[7-(5-chloro-2-fluorophenyl)-1H,2H,3H-pyrido[3,4-b][1,4]oxazin-1-yl]pyridin-2-yl}carbamoyl)ethyl](methyl)amino}acetate;
N-{4-[7-(5-chloro-2-fluorophenyl)-1H,2H,3H-pyrido[3,4-b][1,4]oxazin-1-yl]pyridin-2-yl}-3-methanesulfonamidopropanamide;
N-{4-[7-(5-chloro-2-fluorophenyl)-1H,2H,3H-pyrido[3,4-b][1,4]oxazin-1-yl]pyridin-2-yl}-3-(4-methyl-2-oxopiperazin-1-yl)propanamide;
N-{4-[7-(5-chloro-2-fluorophenyl)-1H,2H,3H-pyrido[3,4-b][1,4]oxazin-1-yl]pyridin-2-yl}-3-[(2-methanesulfonylethyl)amino]propanamide;
N-{4-[7-(5-chloro-2-fluorophenyl)-1H,2H,3H-pyrido[3,4-b][1,4]oxazin-1-yl]pyridin-2-yl}-3-[(2-methanesulfonylethyl)(methyl)amino]propanamide;
N-{4-[7-(5-chloro-2-fluorophenyl)-1H,2H,3H-pyrido[3,4-b][1,4]oxazin-1-yl]pyridin-2-yl}-3-[4-(2-hydroxyethyl)piperazin-1-yl]propanamide;
N-{4-[7-(5-chloro-2-fluorophenyl)-1H,2H,3H-pyrido[3,4-b][1,4]oxazin-1-yl]pyridin-2-yl}-3-[(2-methanesulfonamidoethyl)(methyl)amino]propanamide;
N-{4-[7-(5-chloro-2-fluorophenyl)-1H,2H,3H-pyrido[3,4-b][1,4]oxazin-1-yl]pyridin-2-yl}-3-[4-(2-methanesulfonamidoethyl)piperazin-1-yl]propanamide;
3-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-1-yl}-N-methylpropanamide;
N-{4-[7-(5-chloro-2-fluorophenyl)-1H,2H,3H-pyrido[3,4-b][1,4]oxazin-1-yl]pyridin-2-yl}-3-(1-methylpiperidin-4-yl)propanamide;
N-{4-[7-(5-cyclopropyl-2-fluorophenyl)-1H,2H,3H-pyrido[3,4-b][1,4]oxazin-1-yl]pyridin-2-yl}-3-(4-methylpiperazin-1-yl)propanamide;
N-(4-{7-[2-fluoro-5-(propan-2-yl)phenyl]-1H,2H,3H-pyrido[3,4-b][1,4]oxazin-1-yl}pyridin-2-yl)-3-(4-methylpiperazin-1-yl)propanamide;
3-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-1-yl}-N-(2-hydroxyethyl)propanamide;
3-(4-{2-[bis(2-hydroxyethyl)amino]ethyl}piperazin-1-yl)-N-{4-[7-(5-chloro-2-fluorophenyl)-1H,2H,3H-pyrido[3,4-b][1,4]oxazin-1-yl]pyridin-2-yl}propanamide;
3-[4-(2-carbamoylethyl)piperazin-1-yl]-N-{4-[7-(5-chloro-2-fluorophenyl)-1H,2H,3H-pyrido[3,4-b][1,4]oxazin-1-yl]pyridin-2-yl}propanamide;
N-{4-[7-(6-chloro-3-fluoropyridin-2-yl)-1H,2H,3H-pyrido[3,4-b][1,4]oxazin-1-yl]pyridin-2-yl}-3-(4-methylpiperazin-1-yl)propanamide;
N-{4-[7-(6-chloro-3-fluoropyridin-2-yl)-1H,2H,3H-pyrido[3,4-b][1,4]oxazin-1-yl]pyridin-2-yl}-3-(1-methylpiperidin-4-yl)propanamide;
N-{4-[7-(5-chloro-2-fluorophenyl)-1H,2H,3H-pyrido[3,4-b][1,4]oxazin-1-yl]pyridin-2-yl}-3-methoxypropanamide;
N-{4-[7-(2-chloro-5-fluoropyridin-4-yl)-1H,2H,3H-pyrido[3,4-b][1,4]oxazin-1-yl]pyridin-2-yl}-3-(1-methylpiperidin-4-yl)propanamide;
N-4-[7-(2-chloro-5-fluoropyridin-4-yl)-1H,2H,3H-pyrido[3,4-b][1,4]oxazin-1-yl]pyridin-2-yl}-3-(4-methylpiperazin-1-yl)propanamide;
N-{4-[7-(5-chloro-2-fluorophenyl)-1H,2H,3H-pyrido[3,4-b][1,4]oxazin-1-yl]pyridin-2-yl}-2-(4-methylpiperazin-1-yl)acetamide;
7-(5-chloro-2-fluorophenyl)-1-{2-[3-(4-methylpiperazin-1-yl)propanamido]pyridin-4-yl}-1H,2H,3H-pyrido[3,4-b][1,4]oxazine-5-carboxylic acid;
Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazine-2-carboxylate;
Methyl 4-[2-({4-[7-(5-chloro-2-fluorophenyl)-1H,2H,3H-pyrido[3,4-b][1,4]oxazin-1-yl]pyridin-2-yl}carbamoyl)ethyl]piperazine-2-carboxylate;
Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]-1-methylpiperazin-2-yl}acetate;
Methyl 2-{4-[2-({4-[7-(5-chloro-2-fluorophenyl)-1H,2H,3H-pyrido[3,4-b][1,4]oxazin-1-yl]pyridin-2-yl}carbamoyl)ethyl]piperazin-2-yl}acetate;
Methyl 1-[2-({4-[7-(5-chloro-2-fluorophenyl)-1H,2H,3H-pyrido[3,4-b][1,4]oxazin-1-yl]pyridin-2-yl}carbamoyl)ethyl]-4-methylpiperazine-2-carboxylate;
Methyl 2-{ 1-[2-({4-[7-(5-chloro-2-fluorophenyl)-1H,2H,3H-pyrido[3,4-b][1,4]oxazin-1-yl]pyridin-2-yl}carbamoyl)ethyl]-4-methylpiperazin-2-yl}acetate;
N- f 4-[5-amino-7-(5-chloro-2-fluorophenyl)-1H,2H,3H-pyrido[3,4-b][1,4]oxazin-1-yl]pyridin-2-yl}-3-(4-methylpiperazin-1-yl)propanamide;
N-{4-[7-(5-chloro-2-fluorophenyl)-1H,2H,3H-pyrido[3,4-b][1,4]oxazin-1-yl]pyridin-2-yl}-2-{5-methyl-2,5-diazabicyclo[2.2.1]heptan-2-yl}acetamide;
N-{4-[7-(5-chloro-2-fluorophenyl)-1H,2H,3H-pyrido[3,4-b][1,4]oxazin-1-yl]pyridin-2-yl}-2-{6-methyl-2,6-diazaspiro[3.3]heptan-2-yl}acetamide;
N- f 4-[7-(5-chloro-2-fluorophenyl)-1H,2H,3H-pyrido[3,4-b][1,4]oxazin-1-yl]pyridin-2-yl}-2- f 2-methyl-5-oxa-2,8-diazaspiro[3.5]nonan-8-yl}acetamide;
1-(4-(7-(5-chloro-2-fluorophenyl)-2,3-dihydro-1H-pyrido[3,4-b][1,4]oxazin-1-yl)pyridin-2-yl)-3-(2-(4-methylpiperazin-1-yl)ethyl)urea;
N-(3-(7-(5-chloro-2-fluorophenyl)-5-(trifluoromethyl)-2,3-dihydro-1H-pyrido[3,4-b][1,4]oxazin-1-yl)phenyl)-3-(4-methylpiperazin-1-yl)propanamide.

4. The compound of formula (Ia) according to claim 2, wherein A is A1a 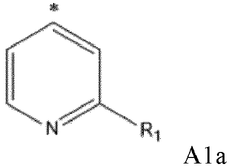 represented by the formula (Iaa) 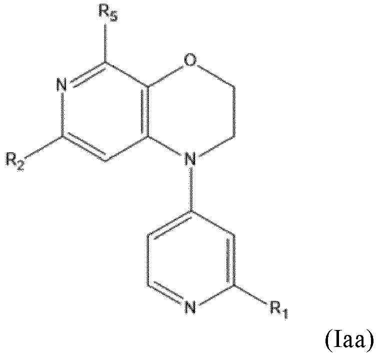
**R₁** is selected from the group consisting of -NR₃C(O)R₄ and -NR₃R_{3'};
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₃** is H or -(C₁-C₆)alkyl;
**R_{3'}** is H or -(C₁-C₆)alkyl;
**R₄** is selected from the group consisting of -(C₁-C₆)alkyl, -NR_{A}R_{B}, -(C₁₋C₆)alkylene-NR_{A}R_{B}, -(C₁-C₆)alkylene-O-(C₁₋C₆)alkyl, -NH(C₁₋C₆)alkylene-heterocycloalkyl and -(C₁-C₆)alkylene-heterocycloalkyl, wherein said heterocycloalkyl is optionally substituted by one or more groups selected from oxo, alkoxy, -(C₁₋C₆)alkylene-OH, -C(O)O-(C₁-C₆)alkyl, -(C₁₋C₆)alkylene-C(O)O-(C₁-C₆)alkyl, -(C₁₋C₆)alkylene-C(O)NH-(C₁₋C₆)alkyl, -(C₁₋C₆)alkylene-C(O)NH-(C₁₋C₆)alkyl-OH, -(C₁₋C₆)alkylene-NHSO₂-(C₁₋C₆)alkyl, -(C₁₋C₆)alkylene-CONH₂, -(C₁-C₆)alkylene-NR_{A}R_{B}, and -(C₁-C₆)alkyl;
**R_{A}** is H or is selected from the group consisting of -(C₁₋C₆)alkyl, heterocycloalkyl and -(C₁-C₆)hydroxyalkyl;
**R_{B}** is selected from the group consisting of -(C₁-C₆)alkyl, -(C₁-C₆)alkylene-C(O)O-(C₁-C₆)alkyl, heterocycloalkyl, -(C₁-C₆)alkylene-heterocycloalkyl, -SO₂-(C₁-C₆)alkyl, -(C₁-C₆)alkylene-NH-SO₂-(C₁-C₆)alkyl, -(C₁-C₆)alkylene-SO₂-(C₁₋C₆)alkyl and -(C₁-C₆)hydroxyalkyl, wherein said heterocycloalkyl of the -(C₁-C₆)alkylene-heterocycloalkyl may be optionally substituted by one or more -(C₁-C₆)alkyl;
**R₅** is H or selected from the group consisting of -NH₂, -C(O)OH, -C(O)O-(C₁₋C₆)alkyl, -(C₁-C₆)haloalkyl and -OH;
and pharmaceutically acceptable salts thereof.

5. The compound of formula (Iaa) according to claim 4, wherein
**R₁** is -NHC(O)R₄;
**R₄** is selected from the group consisting of methyl, 4-ethylmorpholine, (dimethylamino)ethyl, 4-ethylpiperazine, 1-ethyl-4-methylpiperazine, 2-[bis(2-hydroxyethyl)amino]ethyl, -[(2-hydroxyethyl)(methyl)amino]ethyl, 3-[bis(oxetan-3-yl)amino]ethyl, -3-(4-methyl-3-oxopiperazin-1-yl)ethyl, methyl-2-(ethyl(methyl)amino)acetate, -ethylmethanesulfonamide, -3-(4-methyl-2-oxopiperazin-1-yl)ethyl, -3-[(2-methanesulfonylethyl)amino]ethyl, -3-[(2-methanesulfonylethyl)(methyl)amino]ethyl, -3-[4-(2-hydroxyethyl)piperazin-1-yl]ethyl, -3-[(2-methanesulfonamidoethyl)(methyl)amino]ethyl, -3-[4-(2-methanesulfonamidoethyl)piperazin-1-yl]ethyl, -(4-ethyl piperazin-1-yl)-N-methylpropanamide, -3-(1-methylpiperidin-4-yl)ethyl, -(4-ethyl)piperazin-1-yl-N-(2-hydroxyethyl)propanamide, 3-(4-{2-[bis(2-hydroxyethyl)amino]ethyl}piperazin-1-yl)ethyl, 3-[4-(2-carbamoylethyl)piperazin-1-yl]ethyl, -3-methoxyethyl, -2-(4-methylpiperazin-1-yl)methyl, methyl 4-(ethyl)-1-methylpiperazine-2-carboxylate, methyl 4-(ethyl)-piperazine-2-carboxylate, methyl 2-[4-(ethyl)-1-methylpiperazin-2-yl]acetate, methyl 2-[4-(ethyl)-piperazin-2-yl]acetate, methyl 1- (ethyl)-4-methylpiperazine-2-carboxylate, methyl 2-[1-(ethyl)-4-methylpiperazin-2-yl]acetate, 2-{5-methyl-2,5-diazabicyclo[2.2.1]heptan-2-yl}methyl, 2-{ 6-methyl-2,6-diazaspiro[3.3]heptan-2-yl}methyl, 2-{2-methyl-5-oxa-2,8-diazaspiro[3.5]nonan-8-yl}methyl and -3-(2-(4-methylpiperazin-1-yl)ethyl)urea.

6. The compound of formula (I) according to claim 1, wherein A is A2 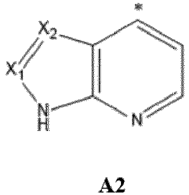 represented by the formula (Ib) 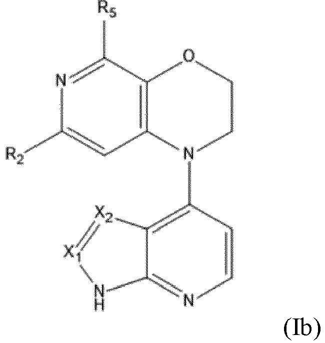
**X₁** and **X₂** are independently N or CH;
**R₂** is aryl optionally substituted by one or more groups selected from halogen atoms, -(C₁-C₆)alkyl, cycloalkyl and -(C₁-C₄)haloalkyl or **R₂** is heteroaryl optionally substituted by one or more halogen atoms;
**R₅** is H;
and pharmaceutically acceptable salts thereof.

7. The compound of formula (Ib) according to claim 6 selected from at least one of:
7-(5-chloro-2-fluorophenyl)-1-{1H-pyrazolo[3,4-b]pyridin-4-yl}-1H,2H,3H-pyrido[3,4-b][1,4]oxazine;
7-(5-chloro-2-fluorophenyl)-1-{1H-pyrrolo[2,3-b]pyridin-4-yl}-1H,2H,3H-pyrido[3,4-b][1,4]oxazine;
7-(5-chloro-2-fluorophenyl)-1-{3H-imidazo[4,5-b]pyridin-7-yl}-1H,2H,3H-pyrido[3,4-b][1,4]oxazine.

8. A pharmaceutical composition comprising a compound of formula (I) according to any one of claims 1 to 7, in a mixture with one or more pharmaceutically acceptable carrier or excipient.

9. The pharmaceutical composition according to claim 8 for administration by inhalation.

10. A compound of formula (I) according to any one of claims 1 to 7 or a pharmaceutical composition according to claims 8 and 9 for use as a medicament.

11. A compound of formula (I) or a pharmaceutical composition for use according to claim 10 in the prevention and/or treatment of a disease, disorder or condition mediated by ALK5 signaling pathway in a mammal.

12. A compound of formula (I) or a pharmaceutical composition for use according to claims 10 and 11 in the prevention and/or treatment of fibrosis and/or diseases, disorders or conditions that involve fibrosis.

13. A compound of formula (I) or a pharmaceutical composition for use according to claim 12 in the prevention and/or treatment of fibrosis including pulmonary fibrosis, idiopathic pulmonary fibrosis (IPF), hepatic fibrosis, renal fibrosis, ocular fibrosis, cardiac fibrosis, arterial fibrosis and systemic sclerosis.

14. A compound of formula (I) or a pharmaceutical composition for use according to claim 13 in the prevention and/or treatment idiopathic pulmonary fibrosis (IPF).

## Patentansprüche

1. Verbindung der allgemeinen Formel (I) 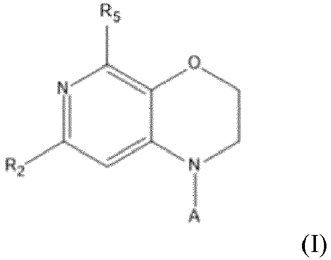 wobei
**A** ausgewählt ist aus den Gruppen bestehend aus **A1** und **A2** 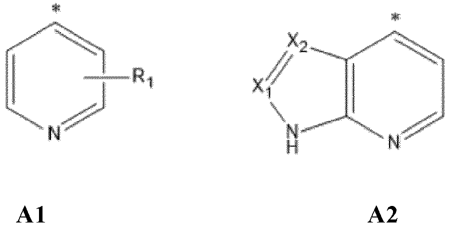
**R₁** H ist oder ausgewählt ist aus der Gruppe bestehend aus -NR₃C(O)R₄ und -NR₃R_{3'};
**X₁** und **X₂** unabhängig voneinander N oder CH sind;
**R₂** optional durch eine oder mehrere Gruppen substituiert ist, die ausgewählt sind aus Halogenatomen, -(C₁-C₆)Alkyl, Cycloalkyl und -(C₁-C₄)Haloalkyl, oder **R₂** optional durch ein oder mehrere Halogenatome substituiert ist;
**R₃** H oder -(C₁-C₆)Alkyl ist;
**R_{3'}** H oder -(C₁-C₆)-Alkyl ist;
**R₄** ausgewählt ist aus der Gruppe bestehend aus -(C₁-C₆)Alkyl, -NR_{A}R_{B}, -(C₁-C₆)Alkylen-NR_{A}R_{B}, -(C₁-C₆)Alkylen-O-(C₁-C₆)Alkyl, -NH(C₁-C₆)Alkylen-Heterocycloalkyl und -(C₁-C₆)Alkylen-Heterocycloalkyl, wobei das Heterocycloalkyl optional durch eine oder mehrere Gruppen substituiert ist, die ausgewählt sind aus Oxo, Alkoxy, -(C₁-C₆)Alkylen-OH, -C(O)O-(C₁-C₆)Alkyl, -(C₁₋C₆)Alkylen-C(O)O-(C₁-C₆)Alkyl, -(C-C₆)Alkylen-C(O)NH-(C₁-C₆)Alkyl, -(C₁-C₆)Alkylen-C(O)NH-(C₁-C₆)Alkyl-OH, -(C₁-C₆)Alkylen-NHSO₂-(C₁-C₆)Alkyl, -(C₁-C₆)Alkylen-CONH₂, -(C₁-C₆)Alkylen-NR_{A}R_{B} und -(C₁-C₆)Alkyl;
**R_{A}** H ist oder ausgewählt ist aus der Gruppe bestehend aus -(C₁-C₆)Alkyl, Heterocycloalkyl und -(C₁-C₆)Hydroxyalkyl,
**R_{B}** ausgewählt ist aus der Gruppe bestehend aus -(C₁-C₆)Alkyl, -(C₁-C₆)Alkylen-C(O)O-(C₁-C₆)Alkyl, Heteroalkyl, -(C₁-C₆)Alkylen-Heterocycloalkyl, -SO₂-(C₁-C₆)Alkyl, -(C₁-C₆)Alkylen-NH-SO₂-(C₁-C₆)Alkyl, -(C₁-C₆)Alkylen-SO₂-(C₁-C₆)Alkyl und -(C₁-C₆)Hydroxyalkyl, wobei das Heterocycloalkyl des -(C₁-C₆)Alkylen-Heterocycloalkyls optional durch ein oder mehrere -(C₁-C₆)Alkyle substituiert werden kann;
**R₅** H ist oder ausgewählt ist aus der Gruppe bestehend aus -NH₂, -C(O)OH, - C(O)O-(C₁-C₆)Alkyl, -(C₁-C₆)Haloalkyl und -OH;
und pharmazeutisch akzeptable Salze davon.

2. Die Verbindung der Formel (I) nach Anspruch 1, wobei A die Gruppe A1 ist 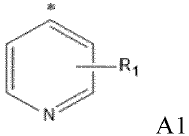 durch die Formel (Ia) dargestellt 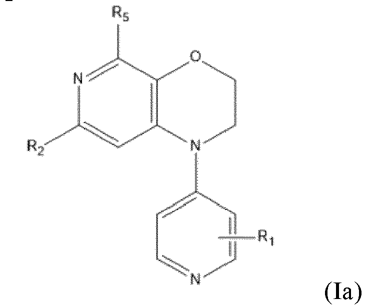
**R₁** H ist oder ausgewählt ist aus der Gruppe bestehend aus -NR₃C(O)R₄ und - NR₃R_{3'};
**R₂** optional durch eine oder mehrere Gruppen substituiert ist, die ausgewählt sind aus Halogenatomen, -(C₁-C₆)Alkyl, Cycloalkyl und -(C₁-C₄)Haloalkyl, oder **R₂** optional durch ein oder mehrere Halogenatome substituiert ist;
**R₃** H oder -(C₁-C₆)Alkyl ist;
**R_{3'}** H oder -(C₁-C₆)-Alkyl ist;
**R₄** ausgewählt ist aus der Gruppe bestehend aus -(C₁-C₆)Alkyl, -NR_{A}R_{B}, -(C₁-C₆)Alkylen-NR_{A}R_{B}, -(C₁-C₆)Alkylen-O-(C₁-C₆)Alkyl, -NH(C₁-C₆)Alkylen-Heterocycloalkyl und -(C₁-C₆)Alkylen-Heterocycloalkyl, wobei das Heterocycloalkyl optional durch eine oder mehrere Gruppen substituiert ist, die ausgewählt sind aus Oxo, Alkoxy, -(C₁-C₆)Alkylen-OH, -C(O)O-(C₁-C₆)Alkyl, -(C ₁₋C₆)Alkylen-C(O)O-(C₁-C₆)Alkyl, -(C-C₆)Alkylen-C(O)NH-(C₁-C₆)Alkyl, -(C₁-C₆)Alkylen-C(O)NH-(C₁-C₆)Alkyl-OH, -(C₁-C₆)Alkylen-NHSO₂-(C₁-C₆)Alkyl, -(C₁-C₆)Alkylen-CONH₂, -(C₁-C₆)Alkylen-NR_{A}R_{B} und -(C₁-C₆)Alkyl;
**R_{A}** H ist oder ausgewählt ist aus der Gruppe bestehend aus -(C₁-C₆)Alkyl, Heterocycloalkyl und -(C₁-C₆)Hydroxyalkyl,
**R_{B}** ausgewählt ist aus der Gruppe bestehend aus -(C₁-C₆)Alkyl, -(C₁-C₆)Alkylen-C(O)O-(C₁-C₆)Alkyl, Heteroalkyl, -(C₁-C₆)Alkylen-Heterocycloalkyl, -SO₂-(C₁-C₆)Alkyl, -(C₁-C₆)Alkylen-NH-SO₂-(C₁-C₆)Alkyl, -(C₁-C₆)Alkylen-SO₂-(C₁-C₆)Alkyl und -(C₁-C₆)Hydroxyalkyl, wobei das Heterocycloalkyl des -(C₁-C₆)Alkylen-Heterocycloalkyls optional durch ein oder mehrere -(C₁-C₆)Alkyle substituiert werden kann;
**R₅** H ist oder ausgewählt ist aus der Gruppe bestehend aus -NH₂, -C(O)OH,-C(O)O-(C₁-C₆)Alkyl, -(C₁-C₆)Haloalkyl und -OH;
und pharmazeutisch akzeptable Salze davon.

3. Die Verbindung der Formel (Ia) nach Anspruch 2, ausgewählt aus mindestens einer der folgenden Verbindungen:
4-[7-(5-Chlor-2-fluorphenyl)-1H,2H,3H-pyrido[3,4-b][1,4]oxazin-1-yl]pyridin;
7-(5-Chlor-2-fluorphenyl)-1-(pyridin-4-yl)-1H,2H,3H-pyrido[3,4-b][1,4]oxalzin-5-amin;
4-[7-(5-Chlor-2-fluorphenyl)-1H,2H,3H-pyrido[3,4-b][1,4]oxazin-1-yl]pyridin-2-amin;
4-[7-(2,5-Difluorphenyl)-1H,2H,3H-pyrido[3,4-b][1,4]oxazin-1-yl]pyridin-2-amin;
4-[7-(3-Chlorphenyl)-1H,2H,3H-pyrido[3,4-b][1,4] oxazin-1-yl]pyridin-2-amin;
N-{4-[7-(5-Chlor-2-fluorphenyl)-1H,2H,3H-pyrido[3,4-b][1,4]oxazin-1-yl]pyridin-2-yl}acetamid;
[7-(6-Chlor-3-fluorpyridin-2-yl)-1H,2H,3H-pyrido[3,4-b][1,4]oxazin-1-yl]pyridin-2-amin;
N-{4-[7-(5-Chlor-2-fluorphenyl)-1H,2H,3H-pyrido[3,4-b][1,4]oxazin-1-yl]pyridin-2-yl}-3-(morpholin-4-yl)propanamid;
N-{4-[7-(5-Chlor-2-fluorphenyl)-1H,2H,3H-pyrido[3,4-b][1,4]oxazin-1-yl]pyridin-2-yl}-3-(dimethylamino)propanamid;
N-{4-[7-(5-Chlor-2-fluorphenyl)-1H,2H,3H-pyrido[3,4-b][1,4]oxazin-1-yl]pyridin-2-yl}-3-(piperazin-1-yl)propanamid;
4-[7-(2-Fluoro-5-methylphenyl)-1H,2H,3H-pyrido[3,4-b][1,4]oxazin-1-yl]pyridin-2-amin;
4-[7-(5-Cyclopropyl-2-fluorphenyl)-1H,2H,3H-pyrido[3,4-b][1,4]oxazin-1-yl]pyridin-2-amin;
4-{7-[2-Fluoro-5-(propan-2-yl)phenyl]-1H,2H,3H-pyrido[3,4-b][1,4]oxazin-1-yl}pyridin-2-amin;
N-{4-[7-(5-Chlor-2-fluorphenyl)-1H,2H,3H-pyrido[3,4-b][1,4]oxazin-1-yl]pyridin-2-yl}-3-(4-methylpiperazin-1-yl)propanamid;
3-[Bis(2-hydroxyethyl)amino]-N-{4-[7-(5-chlor-2-fluorphenyl)-1H,2H,3H-pyrido[3,4-b][1,4]oxazin-1-yl]pyridin-2-yl}propanamid;
N-{4-[7-(5-Chlor-2-fluorphenyl)-1H,2H,3H-pyrido[3,4-b][1,4]oxazin-1-yl]pyridin-2-yl}-3-[methyl(oxetan-3-yl)amino]propanamid;
N-{4-[7-(5-Chlor-2-fluorphenyl)-1H,2H,3H-pyrido[3,4-b][1,4]oxazin-1-yl]pyridin-2-yl}-3-[(2-hydroxyethyl)(methyl)amino]propanamid;
3-[Bis(oxetan-3-yl)amino]-N-{4-[7-(5-chlor-2-fluorphenyl)-1H,2H,3H-pyrido[3,4-b][1,4]oxazin-1-yl]pyridin-2-yl}propanamid;
N-{4-[7-(5-Chlor-2-fluorphenyl)-1H,2H,3H-pyrido[3,4-b][1,4]oxazin-1-yl]pyridin-2-yl}-3-(4-methyl-3-oxopiperazin-1-yl)propanamid;
Methyl-2-{[2-({4-[7-(5-Chlor-2-fluorphenyl)-1H,2H,3H-pyrido[3,4-b][1,4]oxazin-1-yl]pyridin-2-yl}carbamoyl)ethyl](methyl)amino}acetat;
N-{4-[7-(5-Chlor-2-fluorphenyl)-1H,2H,3H-pyrido[3,4-b][1,4]oxazin-1-yl]pyridin-2-yl}-3-methansulfonamidopropanamid;
N-{4-[7-(5-Chlor-2-fluorphenyl)-1H,2H,3H-pyrido[3,4-b][1,4]oxazin-1-yl]pyridin-2-yl}-3-(4-methyl-2-oxopiperazin-1-yl)propanamid;
N-{4-[7-(5-Chlor-2-fluorphenyl)-1H,2H,3H-pyrido[3,4-b][1,4]oxazin-1-yl]pyridin-2-yl}-3-[(2-methansulfonylethyl)amino]propanamid;
N-{4-[7-(5-Chlor-2-fluorphenyl)-1H,2H,3H-pyrido[3,4-b][1,4]oxazin-1-yl]pyridin-2-yl}-3-[(2-methansulfonylethyl)(methyl)amino]propanamid;
N-{4-[7-(5-Chlor-2-fluorphenyl)-1H,2H,3H-pyrido[3,4-b][1,4]oxazin-1-yl]pyridin-2-yl}-3-[4-(2-hydroxyethyl)piperazin-1-yl]propanamid;
N-{4-[7-(5-Chlor-2-fluorphenyl)-1H,2H,3H-pyrido[3,4-b][1,4]oxazin-1-yl]pyridin-2-yl}-3-[(2-methansulfonamidoethyl)(methyl)amino]propanamid;
N-{4-[7-(5-Chlor-2-fluorphenyl)-1H,2H,3H-pyrido[3,4-b][1,4]oxazin-1-yl]pyridin-2-yl}-3-[4-(2-methansulfonamidoethyl)piperazin-1-yl]propanamid;
3-{4-[2-({4-[7-(5-Chlor-2-fluorphenyl)-1H,2H,3H-pyrido[3,4-b][1,4]oxazin-1-yl]pyridin-2-yl}carbamoyl)ethyl]piperazin-1-yl}-N-methylpropanamid;
N-{4-[7-(5-Chlor-2-fluorphenyl)-1H,2H,3H-pyrido[3,4-b][1,4]oxazin-1-yl]pyridin-2-yl}-3-(1-methylpiperidin-4-yl)propanamid;
N-{4-[7-(5-Cyclopropyl-2-fluorphenyl)-1H,2H,3H-pyrido[3,4-b][1,4]oxazin-1-yl]pyridin-2-yl}-3-(4-methylpiperazin-1-yl)propanamid;
N-(4-{7-[2-Fluor-5-(propan-2-yl)phenyl]-1H,2H,3H-pyrido[3,4-b][1,4]oxazin-1-yl}pyridin-2-yl)-3-(4-methylpiperazin-1-yl)propanamid;
3-{4-[2-({4-[7-(5-Chlor-2-fluorphenyl)-1H,2H,3H-pyrido[3,4-b][1,4]oxazin-1-yl]pyridin-2-yl}carbamoyl)ethyl]piperazin-1-yl}-N-(2-hydroxyethyl)propanamid;
3-(4-{2-[Bis(2-hydroxyethyl)amino]ethyl}piperazin-1-yl)-N-{4-[7-(5-chlor-2-fluorphenyl)-1H,2H,3H-pyrido[3,4-b][1,4]oxazin-1-yl]pyridin-2-yl}propanamid;
3-[4-(2-Cardamylethyl)piperazin-1-yl]-N- f 4-[7-(5-chlor-2-fluorphenyl)-1H,2H,3H-pyrido[3,4-b][1,4]oxazin-1-yl]pyridin-2-yl}propanamid;
N-{4-[7-(6-Chlor-3-fluorpyridin-2-yl)-1H,2H,3H-pyrido[3,4-b][1,4]oxalzin-1-yl]pyridin-2-yl}-3-(4-methylpiperazin-1-yl)propanamid;
N-{4-[7-(6-Chlor-3-fluorpyridin-2-yl)-1H,2H,3H-pyrido[3,4-b][1,4]oxazin-1-yl]pyridin-2-yl}-3-(1-methylpiperidin-4-yl)propanamid;
N-{4-[7-(5-Chlor-2-fluorphenyl)-1H,2H,3H-pyrido[3,4-b][1,4]oxazin-1-yl]pyridin-2-yl}-3-methoxypropanamid;
N-{4-[7-(2-Chlor-5-fluorpyridin-4-yl)-1H,2H,3H-pyrido[3,4-b][1,4]oxazin-1-yl]pyridin-2-yl}-3-(1-methylpiperidin-4-yl)propanamid;
N-{4-[7-(2-Chlor-5-fluorpyridin-4-yl)-1H,2H,3H-pyrido[3,4-b][1,4]oxazin-1-yl]pyridin-2-yl}-3-(4-methylpiperazin-1-yl)propanamid;
N-{4-[7-(5-Chlor-2-fluorphenyl)-1H,2H,3H-pyrido[3,4-b][1,4]oxazin-1-yl]pyridin-2-yl}-2-(4-methylpiperazin-1-yl)acetamid;
7-(5-Chlor-2-fluorphenyl)-1-{2-[3-(4-methylpiperazin-1-yl)propanamido]pyridin-4-yl}-1H,2H,3H-pyrido[3,4-b][1,4]oxazin-5-carbonsäure;
Methyl-4-[2-({4-[7-(5-Chlor-2-fluorphenyl)-1H,2H,3H-pyrido[3,4-b][1,4]oxazin-1-yl]pyridin-2-yl}carbamoyl)ethyl]-1-methylpiperazin-2-carboxylat;
Methyl-4-[2-({4-[7-(5-Chlor-2-fluorphenyl)-1H,2H,3H-pyrido[3,4-b][1,4]oxazin-1-yl]pyridin-2-yl}carbamoyl)ethyl]piperazin-2-carboxylat;
Methyl-2-{4-[2-({4-[7-(5-Chlor-2-fluorphenyl)-1H,2H,3H-pyrido[3,4-b][1,4]oxazin-1-yl]pyridin-2-yl}carbamoyl)ethyl]-1-methylpiperazin-2-yl}acetat;
Methyl-2-{4-[2-({4-[7-(5-Chlor-2-fluorphenyl)-1H,2H,3H-pyrido[3,4-b][1,4]oxazin-1-yl]pyridin-2-yl}carbamoyl)ethyl]piperazin-2-yl}acetat;
Methyl-1-[2-({4-[7-(5-Chlor-2-fluorphenyl)-1H,2H,3H-pyrido[3,4-b][1,4]oxazin-1-yl]pyridin-2-yl}carbamoyl)ethyl]-4-methylpiperazin-2-carboxylat;
Methyl-2-{1-[2-({4-[7-(5-Chlor-2-fluorphenyl)-1H,2H,3H-pyrido[3,4-b][1,4]oxazin-1-yl]pyridin-2-yl}carbamoyl)ethyl]-4-methylpiperazin-2-yl}acetat;
N-{4-[5-Amino-7-(5-chlor-2-fluorphenyl)-1H,2H,3H-pyrido[3,4-b][1,4]oxazin-1-yl]pyridin-2-yl}-3-(4-methylpiperazin-1-yl)propanamid;
N-{4-[7-(5-Chlor-2-fluorphenyl)-1H,2H,3H-pyrido[3,4-b][1,4]oxazin-1-yl]pyridin-2-yl}-2-{5-methyl-2,5-diazabicyclo[2.2.1]heptan-2-yl}acetamid;
N-{4-[7-(5-Chlor-2-fluorphenyl)-1H,2H,3H-pyrido[3,4-b][1,4]oxazin-1-yl]pyridin-2-yl}-2-{6-methyl-2,6-diazaspiro[3.3]heptan-2-yl}acetamid;
N-{4-[7-(5-Chlor-2-fluorphenyl)-1H,2H,3H-pyrido[3,4-b][1,4]oxazin-1-yl]pyridin-2-yl}-2-{2-methyl-5-oxa-2,8-diazaspiro[3.5]nonan-8-yl}acetamid;
1-(4-(7-(5-Chlor-2-fluorphenyl)-2,3-dihydro-1H-pyrido[3,4-b][1,4]oxazin-1-yl)pyridin-2-yl)-3-(2-(4-methylpiperazin-1-yl)ethyl)harnstoff;
N-(3-(7-(5-Chlor-2-fluorphenyl)-5-(trifluormethyl)-2,3-dihydro-1H-pyrido[3,4-b][1,4]oxazin-1-yl)phenyl)-3-(4-methylpiperazin-1-yl)propanamid;

4. Die Verbindung der Formel (Ia) nach Anspruch 2, wobei A gleich A1a 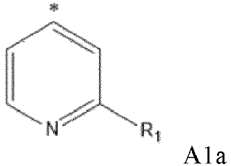 durch die Formel (Iaa) dargestellt 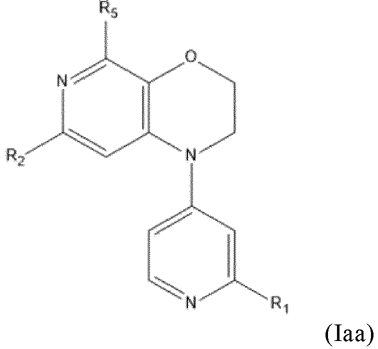
**Ri** ausgewählt ist aus der Gruppe bestehend aus -NR₃C(O)R₄ und -NR₃R_{3'};
**R₂** optional durch eine oder mehrere Gruppen substituiert ist, die ausgewählt sind aus Halogenatomen, -(C₁-C₆)Alkyl, Cycloalkyl und -(C₁-C₄)Haloalkyl, oder **R₂** optional durch ein oder mehrere Halogenatome substituiert ist;
**R₃** H oder -(C₁-C₆)Alkyl ist;
**R_{3'}** H oder -(C₁-C₆)-Alkyl ist;
**R₄** ausgewählt ist aus der Gruppe bestehend aus -(C₁-C₆)Alkyl, -NR_{A}R_{B}, -(C₁-C₆)Alkylen-NR_{A}R_{B}, -(C₁-C₆)Alkylen-O-(C₁-C₆)Alkyl, -NH(C₁-C₆)Alkylen-Heterocycloalkyl und -(C₁-C₆)Alkylen-Heterocycloalkyl, wobei das Heterocycloalkyl optional durch eine oder mehrere Gruppen substituiert ist, die ausgewählt sind aus Oxo, Alkoxy, -(C₁-C₆)Alkylen-OH, -C(O)O-(C₁-C₆)Alkyl, -(C ₁₋C₆)Alkylen-C(O)O-(C₁-C₆)Alkyl, -(C-C₆)Alkylen-C(O)NH-(C₁-C₆)Alkyl, -(C₁-C₆)Alkylen-C(O)NH-(C₁-C₆)Alkyl-OH, -(C₁-C₆)Alkylen-NHSO₂-(C₁-C₆)Alkyl, -(C₁-C₆)Alkylen-CONH₂, -(C₁-C₆)Alkylen-NR_{A}R_{B} und -(C₁-C₆)Alkyl;
**R_{A}** H ist oder ausgewählt ist aus der Gruppe bestehend aus -(C₁-C₆)Alkyl, Heterocycloalkyl und -(C₁-C₆)Hydroxyalkyl,
**R_{B}** ausgewählt ist aus der Gruppe bestehend aus -(C₁-C₆)Alkyl, -(C₁-C₆)Alkylen-C(O)O-(C₁-C₆)Alkyl, Heteroalkyl, -(C₁-C₆)Alkylen-Heterocycloalkyl, -SO₂-(C₁-C₆)Alkyl, -(C₁-C₆)Alkylen-NH-SO₂-(C₁-C₆)Alkyl, -(C₁-C₆)Alkylen-SO₂-(C₁-C₆)Alkyl und -(C₁-C₆)Hydroxyalkyl, wobei das Heterocycloalkyl des -(C₁-C₆)Alkylen-Heterocycloalkyls optional durch ein oder mehrere -(C₁-C₆)Alkyle substituiert werden kann;
**R₅** H ist oder ausgewählt ist aus der Gruppe bestehend aus -NH₂, -C(O)OH, - C(O)O-(C₁-C₆)Alkyl, -(C₁-C₆)Haloalkyl und -OH;
und pharmazeutisch akzeptable Salze davon.

5. Die Verbindung der Formel (Iaa) nach Anspruch 4, wobei
**R₁** -NHC(O)R₄ ist;
**R₄** ausgewählt ist aus der Gruppe bestehend aus Methyl, 4-Ethylmorpholin, (Dimethylamino)ethyl, 4-Ethylpiperazin, 1-Ethyl-4-methylpiperazin, 2-[Bis(2-hydroxyethyl)amino]ethyl, -[(2-Hydroxyethyl)(methyl)amino]ethyl, 3-[Bis(oxetan-3-yl)amino]ethyl, -3-(4-Methyl-3-oxopiperazin-1-yl)ethyl, Methyl-2-(ethyl(methyl)amino)acetat, -Ethylmethansulfonamid, -3-(4-Methyl-2-oxopiperazin-1-yl)ethyl, -3-[(2-Methansulfonylethyl)amino]ethyl, -3-[(2-Methansulfonylethyl)(methyl)amino]ethyl, -3-[4-(2-Hydroxyethyl)piperazin-1-yl]ethyl, -3-[(2-Methansulfonamidoethyl)(methyl)amino]ethyl, -3-[4-(2-Methansulfonamidoethyl)piperazin-1-yl]ethyl, -(4-Ethylpiperazin-1-yl)-N-methylpropanamid, -3-(1-Methylpiperidin-4-yl)ethyl, -(4-Ethyl)piperazin-1-yl-N-(2-hydroxyethyl)propanamid, 3-(4-{2-[Bis(2-hydroxyethyl)amino]ethyl}piperazin-1-yl)ethyl, 3-[4-(2-Carbamoylethyl)piperazin-1-yl]ethyl, -3-Methoxyethyl, -2-(4-Methylpiperazin-1-yl)methyl, Methyl-4-(ethyl)-1-methylpiperazin-2-carboxylat, Methyl-4-(ethyl)-piperazin-2-carboxylat, Methyl-2-[4-(ethyl)-1-methylpiperazin-2-yl]acetat, Methyl-2-[4-(ethyl)-piperazin-2-yl]acetat, Methyl-1-(ethyl)-4-methylpiperazin-2-carboxylat, Methyl-2-[1-(ethyl)-4-methylpiperazin-2-yl]acetat, 2-{5-Methyl-2,5-diazabicyclo[2.2.1]heptan-2-yl}methyl, 2-{6-Methyl-2,6-diazaspiro[3.3]heptan-2-yl}methyl, 2-{2-Methyl-5-oxa-2,8-diazaspiro[3.5]nonan-8-yl}methyl und -3-(2-(4-Methylpiperazin-1-yl)ethyl)harnstoff.

6. Die Verbindung der Formel (I) nach Anspruch 1, wobei A gleich A2 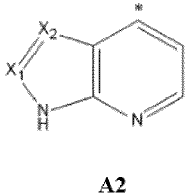 durch die Formel (Ib) dargestellt 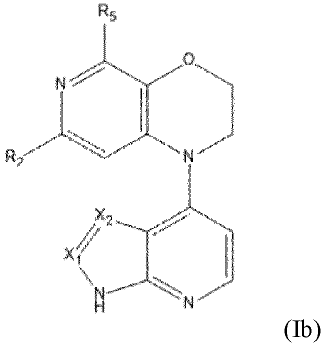
**X₁** und **X₂** unabhängig voneinander N oder CH sind;
**R₂** optional durch eine oder mehrere Gruppen substituiert ist, die ausgewählt sind aus Halogenatomen, -(C₁-C₆)Alkyl, Cycloalkyl und -(C₁-C₄)Haloalkyl, oder **R₂** optional durch ein oder mehrere Halogenatome substituiert ist;
**R₅** H ist;
und pharmazeutisch akzeptable Salze davon.

7. Die Verbindung der Formel (Ib) nach Anspruch 6, ausgewählt aus mindestens einer der folgenden Verbindungen:
7-(5-Chlor-2-fluorphenyl)-1-{1H-pyrazolo[3,4-b]pyridin-4-yl}-1H,2H,3H-pyrido[3,4-b][1,4]oxazin;
7-(5-Chlor-2-fluorphenyl)-1-{1H-pyrrolo[2,3-b]pyridin-4-yl}-1H,2H,3H-pyrido[3,4-b][1,4]oxazin;
7-(5-Chlor-2-fluorphenyl)-1-{3H-imidazo[4,5-b]pyridin-7-yl}-1H,2H,3H-pyrido[3,4-b][1,4]oxazin.

8. Pharmazeutische Zusammensetzung, die eine Verbindung der Formel (I) umfasst, nach einem der Ansprüche 1 bis 7, unter Beigabe eines oder mehrerer pharmazeutisch annehmbarer Träger oder Hilfsstoffe.

9. Pharmazeutische Zusammensetzung nach Anspruch 8 zur Verabreichung durch Inhalation.

10. Verbindung der Formel (I) gemäß einem der Ansprüche 1 bis 7 oder pharmazeutische Zusammensetzung gemäß den Ansprüchen 8 und 9 zur Verwendung als Arzneimittel.

11. Verbindung der Formel (I) oder pharmazeutische Zusammensetzung zur Verwendung gemäß Anspruch 10 bei der Prävention und/oder Behandlung von Krankheiten, Störungen oder Zuständen, die durch den ALKS-Signalweg in einem Säugetier vermittelt werden.

12. Verbindung der Formel (I) oder pharmazeutische Zusammensetzung zur Verwendung gemäß den Ansprüchen 10 und 11 bei der Prävention und/oder Behandlung von Fibrose und/oder Krankheiten, Störungen oder Zuständen, die Fibrose beinhalten.

13. Verbindung der Formel (I) oder pharmazeutische Zusammensetzung zur Verwendung gemäß Anspruch 12 bei der Prävention und/oder Behandlung von Fibrose, einschließlich Lungenfibrose, idiopathischer pulmonaler Fibrose (IPF), Leberfibrose, Nierenfibrose, Augenfibrose, Herzfibrose, arterieller Fibrose und systemischer Sklerose.

14. Verbindung der Formel (I) oder pharmazeutische Zusammensetzung zur Verwendung gemäß Anspruch 13 bei der Prävention und/oder Behandlung von idiopathischer pulmonaler Fibrose (IPF).

## Revendications

1. Composé de formule générale (I) 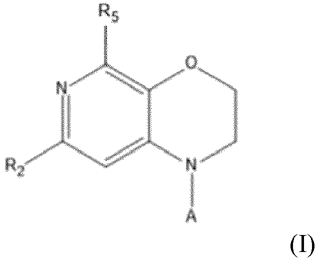 dans lequel
**A** est sélectionné dans les groupe constitués de **A1** et **A2** 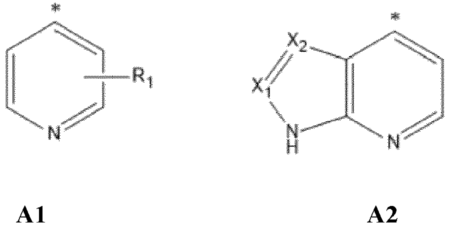
**R₁** est H ou est sélectionné dans le groupe constitué de -NR₃C(O)R₄ et -NR₃R_{3'} ;
**X₁** et **X₂** sont indépendamment N ou CH ;
**R₂** est un aryle optionnellement substitué par un ou plusieurs groupes choisis parmi les atomes halogènes, alkyle -(C₁-C₆), cycloalkyle et haloalkyle -(C₁-C₄) ou **R₂** est un hétéroaryle éventuellement substitué par un ou plusieurs atomes halogènes ;
**R₃** est H ou alkyle -(C₁-C₆) ;
**R_{3'}** est H ou alkyle -(C₁-C₆) ;
**R₄** est choisi dans le groupe constitué d'alkyle -(C₁-C₆), -NR_{A}R_{B} , alkylène -(C₁-C₆)-NR_{A}R_{B}, alkylène -(C₁-C₆)-O alkyle -(C₁₋C₆), alkylène-hétérocycloalkyle-NH(C₁-C₆) et alkylène-hétérocycloalkyle -(C₁-C₆), dans lequel cet hétérocycloalkyle est éventuellement substitué par un ou plusieurs groupes choisis parmi oxo, alcoxyle, alkylène -(C₁₋C₆)-OH, -C(O)O alkyle -(C₁-C₆), alkylène -(C₁₋C₆)-C(O)O alkyle -(C₁-C₆), alkylène -(C₁₋C₆)-C(O)NH, alkyle-(C₁₋C₆), alkylène -(C₁₋C₆)-C(O)NH, alkyle -(C₁₋C₆)-OH, alkylène -(C₁₋C₆)-NHSO₂, alkyle- (C₁₋C₆), alkylène -(C₁₋C₆)-CONH₂, alkylène -(C₁₋C₆)-NR_{A}R_{B}, et alkyle -(C₁-C₆) ;
**R_{A}** est H ou est sélectionné dans le groupe constitué d'alkyle -(C₁-C₆), hétérocycloalkyle et hydroxyalkyle -(C₁-C₆) ;
**R_{B}** est sélectionné dans le groupe constitué d'alkyle -(C₁-C₆), alkylène -(C₁-C₆)-C(O)O, alkyle -(C₁-C₆), hétérocycloalkyle, alkylène-hétérocycloalkyle -(C₁-C₆), alkyle -SO₂-(C₁-C₆), alkylène -(C₁-C₆)-NH-SO₂, alkyle -(C₁-C₆), alkylène -(C₁-C₆)-SO₂, alkyle -(C₁-C₆) et hydroxyalkyle -(C₁-C₆), dans lequel cet hétérocycloalkyle de l'alkylène-hétérocycloalkyle -(C₁-C₆) peut éventuellement être substitué par un ou plusieurs alkyles -(C₁-C₆) ;
**R₅** est H ou sélectionné dans le groupe constitué d'alkyle -NH₂, -C(O)OH,-C(O)O-(C₁₋C₆), haloalkyle -(C₁-C₆) et -OH ;
et ses sels acceptables du point de vue pharmaceutique.

2. Composé de formule (I) selon la revendication 1, dans lequel A est le groupe A1 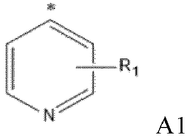 représentée par la formule (Ia) 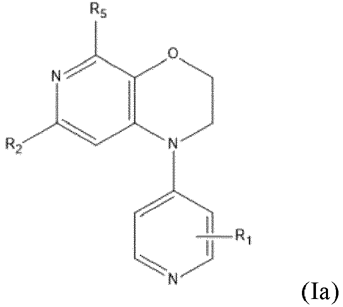
**R₁** est H ou est sélectionné dans le groupe constitué de -NR₃C(O)R₄ et -NR₃R_{3'} ;
**R₂** est optionnellement substitué par un ou plusieurs groupes choisis parmi les atomes halogènes, alkyle -(C₁-C₆), cycloalkyle et haloalkyle -(C₁-C₄) ou **R₂** est éventuellement substitué par un ou plusieurs atomes halogènes ;
**R₃** est H ou alkyle -(C₁-C₆) ;
**R_{3'}** est H ou alkyle -(C₁-C6) ;
**R₄** est choisi dans le groupe constitué d'alkyle -(C₁-C₆), -NR_{A}R_{B} , alkylène -(C₁-C₆)-NR_{A}R_{B}, alkylène -(C₁-C₆)-O alkyle -(C₁₋C₆), alkylène-hétérocycloalkyle - NH(C₁-C₆) et alkylène-hétérocycloalkyle -(C₁-C₆), dans lequel cet hétérocycloalkyle est éventuellement substitué par un ou plusieurs groupes choisis parmi oxo, alcoxyle, alkylène -(C₁₋C₆)-OH, -C(O)O alkyle -(C₁-C₆), alkylène -(C₁₋C₆)-C(O)O alkyle -(C₁-C₆), alkylène -(C₁₋C₆)-C(O)NH, alkyle - (C₁₋C₆), alkylène -(C₁₋C₆)-C(O)NH, alkyle -(C₁₋C₆)-OH, alkylène -(C₁₋C₆)-NHSO₂, alkyle- (C₁₋C₆), alkylène -(C₁₋C₆)-CONH₂, alkylène -(C₁₋C₆)-NR_{A}R_{B}, et alkyle -(C₁-C₆) ;
**R_{A}** est H ou est sélectionné dans le groupe constitué d'alkyle -(C₁-C₆), hétérocycloalkyle et hydroxyalkyle -(C₁-C₆) ;
**R_{B}** est sélectionné dans le groupe constitué d'alkyle -(C₁-C₆), alkylène -(C₁-C₆)-C(O)O, alkyle -(C₁-C₆), hétérocycloalkyle, alkylène-hétérocycloalkyle -(C₁-C₆), alkyle -SO₂-(C₁-C₆), alkylène -(C₁-C₆)-NH-SO₂, alkyle -(C₁-C₆), alkylène -(C₁-C₆)-SO₂, alkyle -(C₁-C₆) et hydroxyalkyle -(C₁-C₆), dans lequel cet hétérocycloalkyle de l'alkylène-hétérocycloalkyle -(C₁-C₆) peut éventuellement être substitué par un ou plusieurs alkyles -(C₁-C₆) ;
**R₅** est H ou sélectionné dans le groupe constitué d'alkyle -NH₂, -C(O)OH,-C(O)O-(C₁₋C₆), haloalkyle -(C₁-C₆) et -OH ;
et ses sels acceptables du point de vue pharmaceutique.

3. Composé de formule (Ia) selon la revendication 2 choisi parmi au moins l'un des :
4-[7-(5-chloro-2-fluorophényl)-1H,2H,3H-pyrido[3,4-b][1,4]oxazine-1-yl]pyridine ;
7-(5-chloro-2-fluorophényl)-1-(pyridine-4-yl)-1H,2H,3H-pyrido[3,4-b][1,4]oxazine-5-amine ;
4-[7-(5-chloro-2-fluorophényl)-1H,2H,3H-pyrido[3,4-b][1,4]oxazine-1-yl]pyridine-2-amine ;
4-[7-(2,5-difluorophényl)-1H,2H,3H-pyrido[3,4-b][1,4]oxazine-1-yl]pyridine-2-amine ;
4-[7-(3-chlorophényl)-1H,2H,3H-pyrido[3,4-b][1,4]oxazine-1-yl]pyridine-2-amine;
N-{4-[7-(5-chloro-2-fluorophényl)-1H,2H,3H-pyrido[3,4-b][1,4]oxazine-1-yl]pyridine-2-yl}acétamide ;
[7-(6-chloro-3-fluoropyridine-2-yl)-1H,2H,3H-pyrido[3,4-b][1,4]oxazine-1-yl]pyridine-2-amine;
N-{4-[7-(5-chloro-2-fluorophényl)-1H,2H,3H-pyrido[3,4-b][1,4]oxazine-1-yl]pyridine-2-yl}-3-(morpholine-4-yl)propanamide ;
N-{4-[7-(5-chloro-2-fluorophényl)-1H,2H,3H-pyrido[3,4-b][1,4]oxazine-1-yl]pyridine-2-yl}-3-(diméthylamino)propanamide ;
N-{4-[7-(5-chloro-2-fluorophényl)-1H,2H,3H-pyrido[3,4-b][1,4]oxazine-1-yl]pyridine-2-yl}-3-(pipérazine-1-yl)propanamide ;
4-[7-(2-fluoro-5-méthylphényl)-1H,2H,3H-pyrido[3,4-b][1,4]oxazine-1-yl]pyridine-2-amine ;
4-[7-(5-cyclopropyle-2-fluorophényl)-1H,2H,3H-pyrido[3,4-b][1,4]oxazine-1-yl]pyridine-2-amine;
4-{7-[2-fluoro-5-(propan-2-yl)phényl]-1H,2H,3H-pyrido[3,4-b][1,4]oxazine-1-yl}pyridine-2-amine ;
N-{4-[7-(5-chloro-2-fluorophényl)-1H,2H,3H-pyrido[3,4-b][1,4]oxazine-1-yl]pyridine-2-yl}-3-(4-méthylpipérazine-1-yl)propanamide ;
3-[bis(2-hydroxyéthyle)amino]-N-{4-[7-(5-chloro-2-fluorophényl)-1H,2H,3H-pyrido[3,4-b][1,4]oxazine-1-yl]pyridine-2-yl}propanamide ;
N-{4-[7-(5-chloro-2-fluorophényl)-1H,2H,3H-pyrido[3,4-b][1,4]oxazine-1-yl]pyridine-2-yl}-3-[méthyle(oxétane-3-yl)amino]propanamide ;
N-{4-[7-(5-chloro-2-fluorophényl)-1H,2H,3H-pyrido[3,4-b][1,4]oxazine-1-yl]pyridine-2-yl}-3-[(2-hydroxyéthyle)(méthyle)amino]propanamide ;
3-[bis(oxétane-3-yl)amino]-N-{4-[7-(5-chloro-2-fluorophényl)-1H,2H,3H-pyrido[3,4-b][1,4]oxazine-1-yl]pyridine-2-yl}propanamide ;
N-{4-[7-(5-chloro-2-fluorophényl)-1H,2H,3H-pyrido[3,4-b][1,4]oxazine-1-yl]pyridine-2-yl}-3-(4-méthyle-3-oxopipérazine-1-yl)propanamide ;
méthyle 2-{[2-({4-[7-(5-chloro-2-fluorophényl)-1H,2H,3H-pyrido[3,4-b][1,4]oxazine-1-yl]pyridine-2-yl}carbamoyl)éthyle](méthyle)amino}acétate ;
N- f 4-[7-(5-chloro-2-fluorophényl)-1H,2H,3H-pyrido[3,4-b][1,4]oxazine-1-yl]pyridine-2-yl}-3-méthane propanamide-sulfonamide ;
N- f 4-[7-(5-chloro-2-fluorophényl)-1H,2H,3H-pyrido[3,4-b][1,4]oxazine-1-yl]pyridine-2-yl}-3-(4-méthyle-2-oxopipérazine-1-yl)propanamide ;
N- f 4-[7-(5-chloro-2-fluorophényl)-1H,2H,3H-pyrido[3,4-b][1,4]oxazine-1-yl]pyridine-2-yl}-3-[(2-méthanesulfonyléthyl)amino]propanamide ;
N- f 4-[7-(5-chloro-2-fluorophényl)-1H,2H,3H-pyrido[3,4-b][1,4]oxazine-1-yl]pyridine-2-yl}-3-[(2-méthanesulfonyléthyl)(méthyle)amino]propanamide ;
N-{4-[7-(5-chloro-2-fluorophényl)-1H,2H,3H-pyrido[3,4-b][1,4]oxazine-1-yl]pyridine-2-yl}-3-[4-(2-hydroxyéthyle)pipérazine-1-yl]propanamide ;
N-{4-[7-(5-chloro-2-fluorophényl)-1H,2H,3H-pyrido[3,4-b][1,4]oxazine-1-yl]pyridine-2-yl}-3-[(2-méthanesulfonate d'éthyle)(méthyle)amino]propanamide ;
N-{4-[7-(5-chloro-2-fluorophényl)-1H,2H,3H-pyrido[3,4-b][1,4]oxazine-1-yl]pyridine-2-yl}-3-[4-(2-méthanesulfonate d'éthyle)pipérazine-1-yl]propanamide ;
3-{4-[2-({4-[7-(5-chloro-2-fluorophényl)-1H,2H,3H-pyrido[3,4-b][1,4]oxazine-1-yl]pyridine-2-yl}carbamoyl)éthyl]pipérazine-1-yl}-N-méthylpropanamide ;
N-{4-[7-(5-chloro-2-fluorophényl)-1H,2H,3H-pyrido[3,4-b][1,4]oxazine-1-yl]pyridine-2-yl}-3-(1-méthylpipéridine-4-yl)propanamide ;
N-{4-[7-(5-cyclopropyle-2-fluorophényl)-1H,2H,3H-pyrido[3,4-b][1,4]oxazine-1-yl]pyridine-2-yl}-3-(4-méthylpipérazine-1-yl)propanamide ;
N-(4-{7-[2-fluoro-5-(propan-2-yl)phényl]-1H,2H,3H-pyrido[3,4-b][1,4]oxazine-1-yl}pyridine-2-yl)-3-(4-méthylpipérazine-1-yl)propanamide ;
3-{4-[2-({4-[7-(5-chloro-2-fluorophényl)-1H,2H,3H-pyrido[3,4-b][1,4]oxazine-1-yl]pyridine-2-yl}carbamoyl)éthyl]pipérazine-1-yl}-N-(2-hydroxyéthyle)propanamide ;
3-(4-{2-[bis(2-hydroxyéthyle)amino]éthyl}pipérazine-1-yl)-N-{4-[7-(5-chloro-2-fluorophényl)-1H,2H,3H-pyrido[3,4-b][1,4]oxazine-1-yl]pyridine-2-yl}propanamide ;
3-[4-(2-carbamoyléthyl)pipérazine-1-yl]-N-{4-[7-(5-chloro-2-fluorophényl)-1H,2H,3H-pyrido[3,4-b][1,4]oxazine-1-yl]pyridine-2-yl}propanamide ;
N-{4-[7-(6-chloro-3-fluoropyridine-2-yl)-1H,2H,3H-pyrido[3,4-b][1,4]oxazine-1-yl]pyridine-2-yl}-3-(4-méthylpipérazine-1-yl)propanamide ;
N-{4-[7-(6-chloro-3-fluoropyridine-2-yl)-1H,2H,3H-pyrido[3,4-b][1,4]oxazine-1-yl]pyridine-2-yl}-3-(1-méthylpipéridine-4-yl)propanamide ;
N-{4-[7-(5-chloro-2-fluorophényl)-1H,2H,3H-pyrido[3,4-b][1,4]oxazine-1-yl]pyridine-2-yl}-3-méthoxypropanamide ;
N-{4-[7-(2-chloro-5-fluoropyridine-4-yl)-1H,2H,3H-pyrido[3,4-b][1,4]oxazine-1-yl]pyridine-2-yl}-3-(1-méthylpipéridine-4-yl)propanamide ;
N-{4-[7-(2-chloro-5-fluoropyridine-4-yl)-1H,2H,3H-pyrido[3,4-b][1,4]oxazine-1-yl]pyridine-2-yl}-3-(4-méthylpipérazine-1-yl)propanamide ;
N-{4-[7-(5-chloro-2-fluorophényl)-1H,2H,3H-pyrido[3,4-b][1,4]oxazine-1-yl]pyridine-2-yl}-2-(4-méthylpipérazine-1-yl)acétamide ;
acide 7-(5-chloro-2-fluorophényl)-1-{2-[3-(4-méthylpipérazine-1-yl)propanamide]pyridine-4-yl}-1H,2H,3H-pyrido[3,4-b][1,4]oxazine-5-carboxylique ;
Méthyle 4-[2-({4-[7-(5-chloro-2-fluorophényl)-1H,2H,3H-pyrido[3,4-b][1,4]oxazine-1-yl]pyridine-2-yl}carbamoyl)éthyl]-1-méthylpipérazine-2-carboxylate ;
Méthyle 4-[2-({4-[7-(5-chloro-2-fluorophényl)-1H,2H,3H-pyrido[3,4-b][1,4]oxazine-1-yl]pyridine-2-yl}carbamoyl)éthyl]pipérazine-2-carboxylate ;
Acétate de méthyle 2-{4-[2-({4-[7-(5-chloro-2-fluorophényl)-1H,2H,3H-pyrido[3,4-b][1,4]oxazine-1-yl]pyridine-2-yl}carbamoyl)éthyl]-1-méthylpipérazine-2-yl} ;
Acétate de méthyle 2-{4-[2-({4-[7-(5-chloro-2-fluorophényl)-1H,2H,3H-pyrido[3,4-b][1,4]oxazine-1-yl]pyridine-2-yl}carbamoyl)éthyl]pipérazine-2-yl} ;
Méthyle 1-[2-({4-[7-(5-chloro-2-fluorophényl)-1H,2H,3H-pyrido[3,4-b][1,4]oxazine-1-yl]pyridine-2-yl}carbamoyl)éthyl]-4-méthylpipérazine-2-carboxylate ;
Acétate de méthyle 2-{1-[2-({4-[7-(5-chloro-2-fluorophényl)-1H,2H,3H-pyrido[3,4-b][1,4]oxazine-1-yl]pyridine-2-yl}carbamoyl)éthyl]-4-méthylpipérazine-2-yl} ;
N-{4-[5-amino-7-(5-chloro-2-fluorophényl)-1H,2H,3H-pyrido[3,4-b][1,4]oxazine-1-yl]pyridine-2-yl}-3-(4-méthylpipérazine-1-yl)propanamide ;
N- f 4-[7-(5-chloro-2-fluorophényl)-1H,2H,3H-pyrido[3,4-b][1,4]oxazine-1-yl]pyridine-2-yl}-2-{5-méthyle-2,5-diazabicyclo[2,2,1]heptane-2-yl}acétamide ;
N- f 4-[7-(5-chloro-2-fluorophényl)-1H,2H,3H-pyrido[3,4-b][1,4]oxazine-1-yl]pyridine-2-yl}-2-{6-méthyle-2,6-diazaspiro[3,3]heptane-2-yl}acétamide ;
N- f 4-[7-(5-chloro-2-fluorophényl)-1H,2H,3H-pyrido[3,4-b][1,4]oxazine-1-yl]pyridine-2-yl}-2-{2-méthyl-5-oxa-2,8-diazaspiro[3,5]nonan-8-yl}acétamide ;
1-(4-(7-(5-chloro-2-fluorophényl)-2,3-dihydro-1H-pyrido[3,4-b][1,4]oxazine-1-yl)pyridine-2-yl)-3-(2-(4-méthylpipérazine-1-yl)éthyl)urée ;
N-(3-(7-(5-chloro-2-fluorophényl)-5-(trifluorométhyle)-2,3-dihydro-1H-pyrido[3,4-b][1,4]oxazine-1-yl)phényl)-3-(4-méthylpipérazine-1-yl)propanamide.

4. Composé de formule (Ia) selon la revendication 2, dans lequel A est A1a 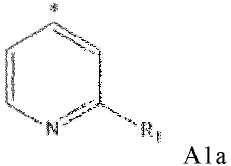 représenté par la formule (Iaa) 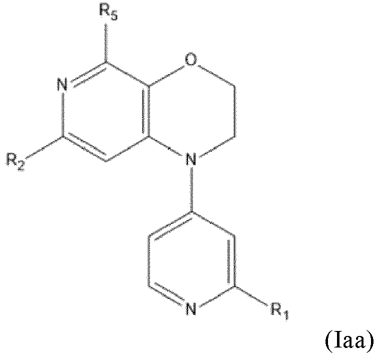
**R₁** est sélectionné dans le groupe constitué de -NR₃C(O)R₄ et -NR₃R_{3'} ;
**R₂** est optionnellement substitué par un ou plusieurs groupes choisis parmi les atomes halogènes, alkyle -(C₁-C₆), cycloalkyle et haloalkyle -(C₁-C₄) ou **R₂** est éventuellement substitué par un ou plusieurs atomes halogènes ;
**R₃** est H ou alkyle -(C₁-C₆) ;
**R_{3'}** est H ou alkyle -(C₁-C6) ;
**R₄** est choisi dans le groupe constitué d'alkyle -(C₁-C₆), -NR_{A}R_{B} , alkylène -(C₁-C₆)-NR_{A}R_{B}, alkylène -(C₁-C₆)-O alkyle -(C₁₋C₆), alkylène-hétérocycloalkyle-NH(C₁-C₆) et alkylène-hétérocycloalkyle -(C₁-C₆), dans lequel cet hétérocycloalkyle est éventuellement substitué par un ou plusieurs groupes choisis parmi oxo, alcoxyle, alkylène -(C₁₋C₆)-OH, -C(O)O alkyle -(C₁-C₆), alkylène -(C₁₋C₆)-C(O)O alkyle -(C₁-C₆), alkylène -(C₁₋C₆)-C(O)NH, alkyle - (C₁₋C₆), alkylène -(C₁₋C₆)-C(O)NH, alkyle -(C₁₋C₆)-OH, alkylène -(C₁₋C₆)-NHSO₂, alkyle- (C₁₋C₆), alkylène -(C₁₋C₆)-CONH₂, alkylène -(C₁₋C₆)-NR_{A}R_{B}, et alkyle -(C₁-C₆) ;
**R_{A}** est H ou est sélectionné dans le groupe constitué d'alkyle -(C₁-C₆), hétérocycloalkyle et hydroxyalkyle -(C₁-C₆) ;
**R_{B}** est sélectionné dans le groupe constitué d'alkyle -(C₁-C₆), alkylène -(C₁-C₆)-C(O)O, alkyle -(C₁-C₆), hétérocycloalkyle, alkylène-hétérocycloalkyle -(C₁-C₆), alkyle -SO₂-(C₁-C₆), alkylène -(C₁-C₆)-NH-SO₂, alkyle -(C₁-C₆), alkylène -(C₁-C₆)-SO₂, alkyle -(C₁-C₆) et hydroxyalkyle -(C₁-C₆), dans lequel cet hétérocycloalkyle de l'alkylène-hétérocycloalkyle -(C₁-C₆) peut éventuellement être substitué par un ou plusieurs alkyles -(C₁-C₆) ;
**R₅** est H ou sélectionné dans le groupe constitué d'alkyle -NH₂, -C(O)OH, - C(O)O-(C₁₋C₆), haloalkyle -(C₁-C₆) et -OH ;
et ses sels acceptables du point de vue pharmaceutique.

5. Composé de formule (Iaa) selon la revendication 4, dans lequel
**R₁** est -NHC(O)R₄ ;
**R₄** est sélectionné dans le groupe constitué de méthyle, 4-éthylmorpholine, (diméthylamino)éthyle, 4-éthylpipérazine, 1-éthyle-4-méthylpipérazine, 2-[bis(2-hydroxyéthyle)amino]éthyle, -[(2-hydroxyéthyle)(méthyl)amino]éthyle, 3-[bis(oxétan-3-yl)amino]éthyle, -3-(4-méthyl-3-oxopipérazine-1-yl)éthyle, méthyl-2-(éthyl(méthyl)amino)acétate, -méthanesulfonate d'éthyle, -3-(4-méthyl-2-oxopipérazine-1-yl)éthyle, -3-[(2-méthanesulfonate d'éthyle)amino]éthyl, -3-[(2-méthanesulfonate d'éthyle)(méthyl)amino]éthyl, -3-[4-(2-hydroxyéthyl)pipérazine-1-yl]éthyle, -3-[(2-méthanesulfonate d'éthyle)(méthyl)amino]éthyl, -3-[4-(2-méthanesulfonate d'éthyle)pipérazine-1-yl]éthyle, -(4-éthyl pipérazine-1-yl)-N-méthylpropanamide, -3-(1-méthylpipéridine-4-yl)éthyle, -(4-éthyl)pipérazine-1-yl-N-(2-hydroxyéthyl)propanamide, 3-(4-{2-[bis(2-hydroxyéthyle)amino]éthyl}pipérazine-1-yl)éthyl, 3-[4-(2-carbamoyléthyl)pipérazine-1-yl]éthyle, -3-méthoxyéthyle, -2-(4-méthylpipérazine-1-yl)méthyle, méthyle 4-(éthyle)-1-méthylpipérazine-2-carboxylate, méthyle 4-(éthyle)-pipérazine-2-carboxylate, méthyle 2-[4-(éthyle)-1-méthylpipérazine-2-yl]acétate, méthyle 2-[4-(éthyle)-pipérazine-2-yl]acétate, méthyle 1- (éthyle)-4-méthylpipérazine-2-carboxylate, méthyle 2-[1-(éthyle)-4-méthylpipérazine-2-yl]acétate, 2-{5-méthyle-2,5-diazabicyclo[2,2,1]heptane-2-yl}méthyle, 2-{6-méthyle-2,6-diazaspiro[3,3]heptane-2-yl }méthyle, 2-{2-méthyle-5-oxa-2,8-diazaspiro[3,5]nonane-8-yl}méthyle et -3-(2-(4-méthylpipérazine-1-yl)éthyle)urée.

6. Le composé de formule (I) selon la revendication 1, dans lequel A est A2 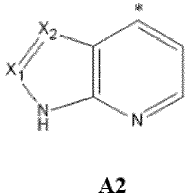 représentée par la formule (Ib) 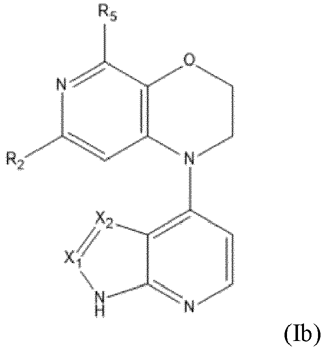
**X₁** et **X₂** sont indépendamment N ou CH ;
**R₂** est optionnellement substitué par un ou plusieurs groupes choisis parmi les atomes halogènes, alkyle -(C₁-C₆), cycloalkyle et haloalkyle -(C₁-C₄) ou **R₂** est éventuellement substitué par un ou plusieurs atomes halogènes ;
**R₅** est H ;
et ses sels acceptables du point de vue pharmaceutique.

7. Composé de formule (Ib) selon la revendication 6 sélectionné parmi au moins l'un des :
7-(5-chloro-2-fluorophényl)-1-{1H-pyrazolo[3,4-b]pyridine-4-yl}-1H,2H,3H-pyrido[3,4-b][1,4]oxazine ;
7-(5-chloro-2-fluorophényl)-1-{1H-pyrrolo[2,3-b]pyridine-4-yl}-1H,2H,3H-pyrido[3,4-b][1,4]oxazine ;
7-(5-chloro-2-fluorophényl)-1-{3H-imidazo[4,5-b]pyridine-7-yl}-1H,2H,3H-pyrido[3,4-b][1,4]oxazine.

8. Composition pharmaceutique comprenant un composé de formule (I) selon l'une des revendications 1 à 7, dans un mélange avec un ou plusieurs transporteurs ou excipients acceptables du point de vue pharmaceutique.

9. Composition pharmaceutique selon la revendication 8 pour administration par inhalation.

10. Composé de formule (I) selon l'une des revendications 1 à 7 ou composition pharmaceutique selon les revendications 8 et 9 pour une utilisation comme médicament.

11. Composé de formule (I) ou composition pharmaceutique pour une utilisation selon la revendication 10 dans la prévention et/ou le traitement d'une maladie, d'un trouble ou d'une affection médié par la voie de signalisation ALK5 chez un mammifère.

12. Composé de formule (I) ou composition pharmaceutique pour une utilisation selon les revendications 10 et 11 dans la prévention et/ou le traitement de la fibrose et/ou de maladies, troubles ou affections impliquant la fibrose.

13. Composé de formule (I) ou composition pharmaceutique pour une utilisation selon la revendication 12 dans la prévention et/ou le traitement de la fibrose incluant la fibrose pulmonaire, la fibrose pulmonaire idiopathique (FPI), la fibrose hépatique, la fibrose rénale, la fibrose oculaire, la fibrose cardiaque, la fibrose artérielle et la sclérose systémique.

14. Composé de formule (I) ou composition pharmaceutique pour une utilisation selon la revendication 13 dans la prévention et/ou le traitement de la fibrose pulmonaire idiopathique (FPI).
